# EUROPEAN PATENT APPLICATION

(11) **EP 3 804 707 A1**
(43) Date of publication of application: **14.04.2021**
(21) Application number: 19814348.9
(22) Date of filing: 06.06.2019
(51) Int. Cl.: A61K 31/165, A61K 31/175, A61P 1/16, A61P 7/02, A61P 7/04, A61P 7/10, A61P 9/10, A61P 17/02, A61P 19/00, A61P 21/00, A61P 25/00, A61P 29/00, A61P 37/00, A61P 43/00, C07C 251/80, C07C 281/14, C07C 281/18, C07C 323/62, C07C 337/08

(54) **KINASE INHIBITOR**

(30) Priority: 08.06.2018 JP 2018110748; 30.01.2019 JP 2019014898
(71) Applicant: Nissan Chemical Corporation, Tokyo 103-6119 (JP)
(72) Inventor: NISHINO, Taito, Shiraoka-shi, Saitama 349-0294 (JP); OTSUKA, Keiichiro, Shiraoka-shi, Saitama 349-0294 (JP); FUKASAWA, Natsuki, Shiraoka-shi, Saitama 349-0294 (JP); NAKAJIMA, Hiroyuki, Shiraoka-shi, Saitama 349-0294 (JP); AIHARA, Ayako, Shiraoka-shi, Saitama 349-0294 (JP)
(74) Representative: J A Kemp LLP
(86) International application number: PCT/JP2019/022536
(87) International publication number: WO 2019/235569

(57) **Abstract**

The present invention aims to provide a novel kinase inhibitor and the like, and a therapeutic agent for a disease, a drug discovery screening method and the like utilizing such inhibitor and the like. The compound represented by the following formula (I) and a salt thereof can inhibit plural kinases including LATS (particularly LATS2) which is the major kinase in the Hippo signal transduction pathway. In addition, diseases or tissue damage associated with failure of cellular proliferation can be treated. Therefore, the present invention is beneficial, for example, in the research field of cell functions and diseases, in which the Hippo signal transduction pathway is involved, and the like. Furthermore, it is beneficial in the medical field for the treatment of such diseases and the like. 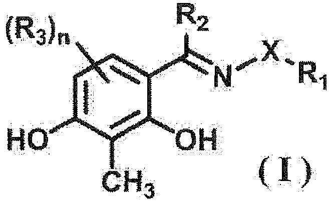 wherein each symbol is as defined in the DESCRIPTION.

## Description

### [Technical Field]

The present invention relates to a kinase inhibitor, a Hippo signal transduction pathway inhibitor, a therapeutic agent for a disease or tissue damage associated with failure of cellular proliferation, a proliferation promoter of a cell or tissue for a transplantation treatment, and a method for screening for a substance for treating and/or preventing a disease associated with promoted nuclear translocation of YAP and/or TAZ, and the like.

### [Background Art]

Kinases (protein phosphorylating enzymes, protein kinases) are enzymes that phosphorylate the hydroxyl groups of serine, threonine, or tyrosine in proteins, and are a group of enzymes responsible for signal transduction such as cell proliferation and differentiation. Not less than 500 kinds of genes encoding kinases have been cloned so far. Protein kinases are present throughout the cells and are deeply involved in the regulation and control of cell proliferation, differentiation, and functional expression (non-patent document 1) .

Abnormal protein kinase activity has been confirmed in many diseases, and diseases are being treated by inhibiting protein kinase activity (non-patent document 2). For example, it is known that many oncogenes involved in canceration of cells encode protein kinases. Gleevec, a protein kinase inhibitor, is marketed as a therapeutic agent for chronic myeloid leukemia (non-patent document 3), and Herceptin is marketed as a therapeutic agent for breast cancer (patent document 1).

LATS1 and LATS2 (Large tumor suppressors 1 and 2) are protein kinases that are major factors constituting the Hippo signal transduction pathway (patent document 2, non-patent documents 4, 5). It is known that this transduction pathway is activated when the cell density increases and contact inhibition is applied, or when cells are injured by active oxygen, DNA damage, or the like (non-patent documents 6, 7, 8). When this transduction pathway is activated, LATS phosphorylates the transcriptional co-factor YAP (Yes-associated protein) or TAZ (transcriptional co-activator with PDZ-binding motif). Phosphorylated YAP or TAZ transfers from the cell nucleus to the cytoplasm and is subjected to proteolysis, thus suppressing the expression of the target gene of YAP or TAZ. Known examples of the genes whose expression is regulated by YAP include CCDN1, CTGF, BIRC2, CYR61, AMOTL2, TGFB2 and the like (non-patent documents 9, 10, 11, 12). Activation of the Hippo signal transduction pathway leads to the suppression of the expression of these genes, and causes suppression of cell proliferation and induction of cell death.

Hippo signal transduction pathway is known to regulate self-renewal and differentiation of stem cells (non-patent document 13). Hippo signal transduction pathway is necessary for maintaining the skin, intestines and nerves, and repair during tissue damage is inhibited unless YAP functions normally (non-patent document 14). Furthermore, deletion of the Hippo signal transduction pathway has been reported to ameliorate systolic heart failure after myocardial infarction (non-patent document 15). In this way, Hippo signal transduction pathway controls the proliferation, maintenance and recovery of cells, tissues and organs. Thus, inhibitors of the Hippo signal transduction pathway and activators of YAP and TAZ are expected to be therapeutic drugs for diseases caused by failure of cellular proliferation. Examples of such diseases include burn, trauma, muscular atrophy, ischemic diseases, and neurodegenerative diseases (e.g., Alzheimer's, Parkinson's and Huntington's diseases). As an example of an inhibitor of the Hippo signal transduction pathway, XMU-MP-1 has been reported as an inhibitor of MST1 (non-patent document 16). XMU-MP-1 has been shown to promote recovery from hepatopathy by inhibiting the Hippo signal transduction pathway. Furthermore, it has been reported that compounds that inhibit LATS1 and LATS2 have an effect of promoting repair of eyeball, skin and liver (patent document 3). In addition, IBS008738 has been reported as an activator of TAZ, and the effect of promoting recovery of muscle damage by this activator has been shown (non-patent document 17). As described above, a regulator of the Hippo signal transduction pathway is expected to be a therapeutic drug for various diseases.

### [Document List]

### [Patent documents]

patent document 1: US Patent No. 5705151
patent document 2: US Patent No. 5994503
patent document 3: WO 2018/198077

### [non-patent document]

non-patent document 1: Robert Roskoski Jr., Pharmacological Research 2016,100: 1-23
non-patent document 2: Doriano F et al, Br J Pharmacol. 2015, 172(11): 2675-2700
non-patent document 3: Druker BJ et al, Nat Med. 1996, 2 (5) :561-566
non-patent document 4: Justice RW et al, Genes Dev. 1995, 9(5):534-546
non-patent document 5: Hergovich A, Cell Biosci. 2013, 3(1):32 non-patent document 6: Pefani DE et al, FEBS J. 2016, 283(8) :1392-1403
non-patent document 7: Meng Z et al, Genes Dev. 2016, 30(1):1-17
non-patent document 8: Zhao B et al, Curr Opin Cell Biol. 2008, 20(6):638-646
non-patent document 9: Imajo M et al, EMBO J. 2012, 31:1109-1122
non-patent document 10: Hong W et al, Semin Cell Dev Biol. 2012, 23(7):785-793
non-patent document 11: Gujral TS et al, Proc Natl Acad Sci U S A. 2017, 114(18):E3729-E3738
non-patent document 12: Lee DH et al, Nat Commun. 2016, 7:11961 non-patent document 13: Aylon Y et al, Cell Death Differ. 2014, 21(4):624-633
non-patent document 14: Johnson R et al, Nat Rev Drug Discov. 2014, 13 (1):63-79
non-patent document 15: Leach JP et al, Nature. 2017, 550(7675) :260-264
non-patent document 16: Fan F et al, Sci Transl Med. 2016, 8(352):352ra108
non-patent document 17: Yang Z et al, Mol Cell Biol. 2014, 34(9): 1607-1621

### [Summary of Invention]

### [Technical Problem]

The present invention aims to provide a novel kinase inhibitor. Particularly, the present invention aims to provide a novel kinase inhibitor that inhibits the Hippo signal transduction pathway. In addition, the present invention also aims to provide a therapeutic agent for a disease, a screening method for drug discovery, and the like that utilize the inhibitor.

### [Solution to Problem]

The present inventors have conducted intensive studies in an attempt to solve the above-mentioned problems and found that a specific compound inhibits the activity of plural protein kinases including LATS (particularly LATS2). In addition, the present inventors found that the specific compound promotes nuclear translocation of an intracellular YAP protein, promotes expression of the target gene group of YAP or TAZ protein, the activity varies greatly depending on the optical isomer in specific compounds having an asymmetric carbon, and the like. Based on such finding, they have conducted further studies and completed the present invention. Therefore, the present invention provides the following.
[1] A kinase inhibitor comprising a compound represented by the following formula (I), or a salt thereof: 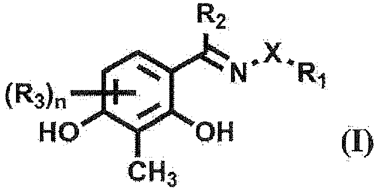 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.
[2] The inhibitor of [1], wherein the kinase is selected from the group consisting of CGK2, LATS2, MSK1, p70S6K, PKACα, PKACβ, SGK2, and SGK3.
[3] The inhibitor of [2], wherein the kinase is LATS2.
[4] A Hippo signal transduction pathway inhibitor comprising a compound represented by the following formula (I) or a salt thereof: 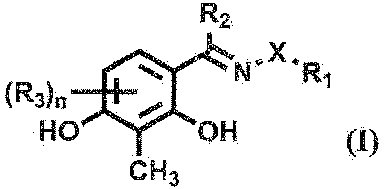 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.
[5] A therapeutic agent for a disease or tissue damage associated with failure of cellular proliferation, comprising a compound represented by the following formula (I) or a salt thereof: 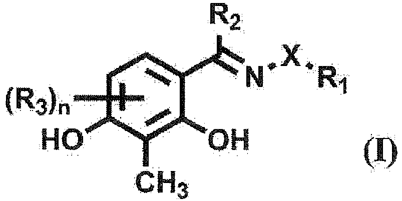 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.
[6] The therapeutic agent of [5], wherein the disease or tissue damage associated with failure of cellular proliferation is a disease associated with suppressed nuclear translocation of YAP and/or TAZ.
[7] The therapeutic agent of [5], wherein the disease or tissue damage associated with failure of cellular proliferation is selected from the group consisting of inflammatory disease, inflammatory bowel disease, neurodegenerative disease, immune-nerve disease, muscular dystrophy, myopathy, trauma, burn, chemical burn, skin ulcer, traumatic ulcer, lower leg ulcer, frostbite ulcer, immune-ulcer, postherpetic ulcer, radiation ulcer, pressure ulcer, diabetic skin ulcer, giant pigmented nevus, scar, disorder due to tattoo, vitiligo vulgaris, leukopathia, spinal cord damage, muscle damage, liver failure, drug-induced hepatopathy, alcohol-induced hepatopathy, ischemic hepatopathy, viral hepatopathy, autoimmune hepatopathy, acute hepatitis, chronic hepatitis, cirrhosis, skin damage, brain edema, myocardial infarction, cerebral hemorrhage, and cerebral infarction.
[8] A proliferation promoter of a cell or tissue for a transplantation treatment, comprising a compound represented by the following formula (I) or a salt thereof: 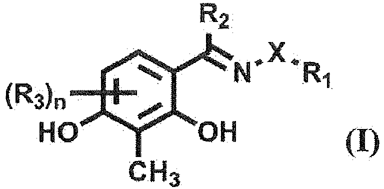 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.
[9] The inhibitor, therapeutic agent or proliferation promoter of any of [1] to [8], wherein X is -NHCO-.
[10] The inhibitor, therapeutic agent or proliferation promoter of any of [1] to [9], wherein R₂ is an alkyl group having 1 - 6 carbon atoms, and n is 0.
[11] The inhibitor, therapeutic agent or proliferation promoter of any of [1] to [10], wherein R₁ is -Y-W-Z-Ar, Y is a methylene group optionally having an alkyl group having 1 - 6 carbon atoms, W is N(R₄), Z is a single bond, and Ar is an aryl group optionally having a halogen atom, a hydroxyl group, an alkyl group having 1 - 6 carbon atoms or an alkoxy group having 1 - 6 carbon atoms.
[12] The inhibitor, therapeutic agent or proliferation promoter of any of [1] to [11], wherein R₂ is a methyl group, an ethyl group, or an isobutyl group,
   n is 0,
   Ar is a phenyl group optionally substituted by a hydroxyl group or a methyl group, and
   Y is a methylene group optionally substituted by a methyl group or an ethyl group.
[13] The inhibitor, therapeutic agent or proliferation promoter of any of [1] to [12], wherein the compound represented by the formula (I) is a compound selected from the group consisting of the following: 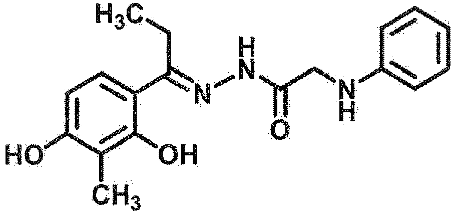 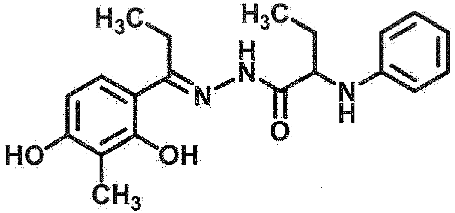 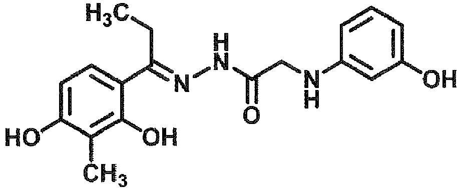 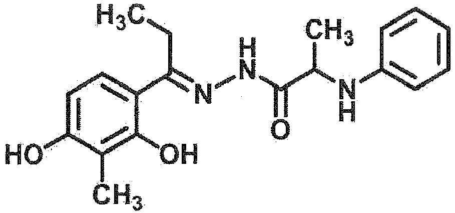 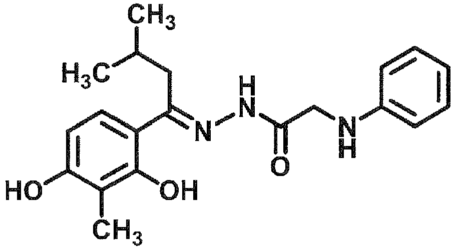 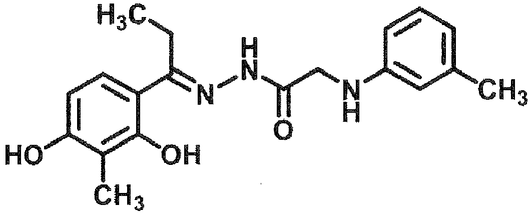 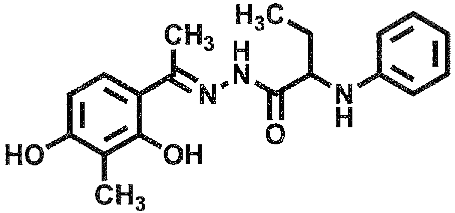 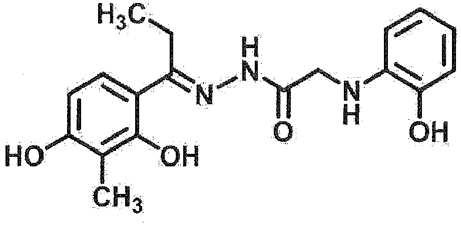 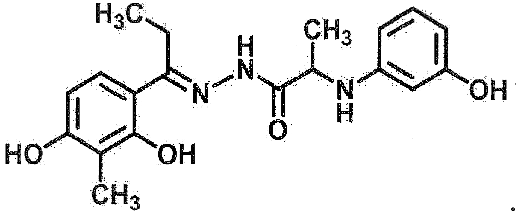
[14] The inhibitor, therapeutic agent or proliferation promoter of any of [1] to [12], wherein the compound represented by the formula (I) is an optical isomer of the R form of a compound selected from the group consisting of: 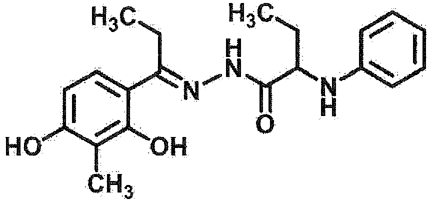 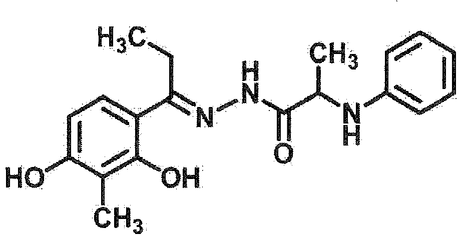 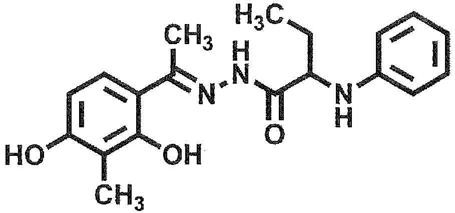 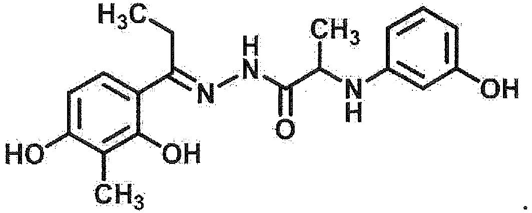
[15] The inhibitor, therapeutic agent or proliferation promoter of any of [1] to [12], wherein R₁ is -Y-W-Z-Ar, Y is a single bond, W is N(R₄), R₄ is a hydrogen atom, Z is a methylene group optionally having an alkyl group having 1 - 6 carbon atoms, and Ar is an aryl group optionally having a halogen atom, a hydroxyl group, an alkyl group having 1 - 6 carbon atoms or an alkoxy group having 1 - 6 carbon atoms.
[16] The inhibitor, therapeutic agent or proliferation promoter of any of [1] to [10] and [15], wherein R₂ is a methyl group, an ethyl group, or an isobutyl group,
   n is 0,
   Ar is a phenyl group optionally substituted by a hydroxyl group, and
   Z is a methylene group optionally substituted by a methyl group or an ethyl group.
[17] The inhibitor, therapeutic agent or proliferation promoter of any of [1] to [10], [15] and [16], wherein the compound represented by the formula (I) is a compound selected from the group consisting of the following: 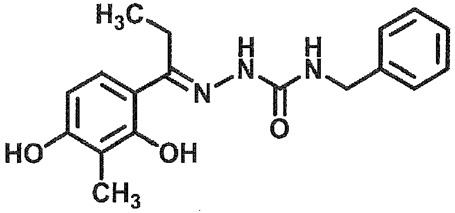 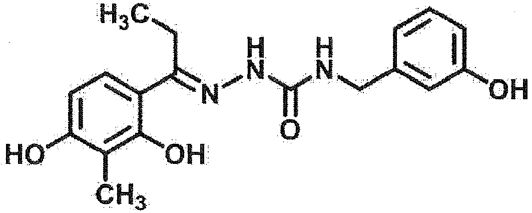 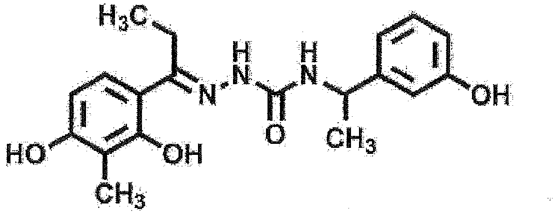
[18] The inhibitor, therapeutic agent or proliferation promoter of any of [1] to [12], [15] and [16], wherein the compound represented by the formula (I) is an optical isomer of the R form of the following compound: 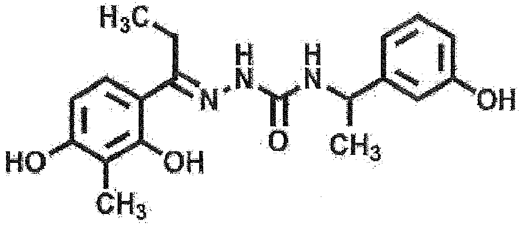
[19] A method for screening for a substance for treating and/or preventing a disease associated with promoted nuclear translocation of YAP and/or TAZ, comprising the following steps:
   (step 1) a step of culturing cells in a medium containing a compound represented by the following formula (I) or a salt thereof: 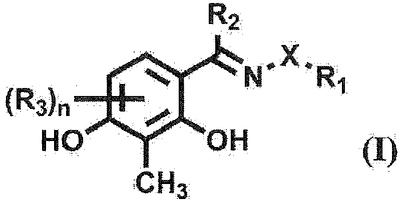 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2},
   (step 2) a step of measuring, in the presence of a test substance, an abundance of YAP and/or TAZ in the nucleus of the cell obtained in (step 1);
   (step 3) a step of determining the test substance as a substance for treating and/or preventing a disease associated with promoted nuclear translocation of YAP and/or TAZ when the abundance of YAP and/or TAZ in the nucleus measured in (step 2) is reduced compared to an abundance of YAP and/or TAZ in the absence of the test substance.
[20] An optically active form of a compound represented by the following formula (I), or a salt thereof: 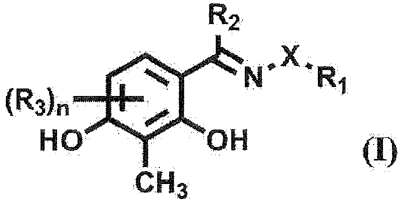 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2 (provided that when X is -NHCO-, R₂ is an ethyl group, and n is 0, then R₁ is not -CH₂-NH-C₆H₅)}.
[21] The optically active form or a salt thereof of [20], wherein X is -NHCO-.
[22] The optically active form or a salt thereof of [20] or [21], wherein R₂ is an alkyl group having 1 - 6 carbon atoms, and n is 0.
[23] The optically active form or a salt thereof of any of [20] to [22], wherein R₁ is -Y-W-Z-Ar, Y is a methylene group having an alkyl group having 1 - 6 carbon atoms, W is N(R₄), Z is a single bond, and Ar is an aryl group optionally having a halogen atom, a hydroxyl group, an alkyl group having 1 - 6 carbon atoms or an alkoxy group having 1 - 6 carbon atoms.
[24] The optically active form or a salt thereof of any of [20] to [23], wherein the optically active form is an R form.
[25] The optically active form or a salt thereof of any of [20] to [24], wherein the compound represented by the formula (I) is a compound selected from the group consisting of the following: 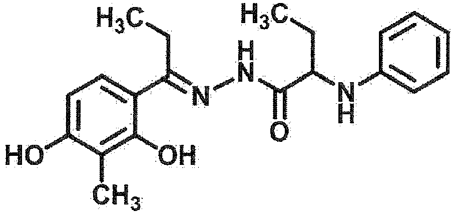 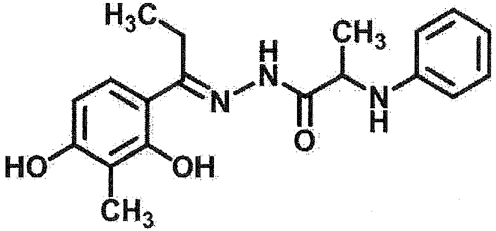 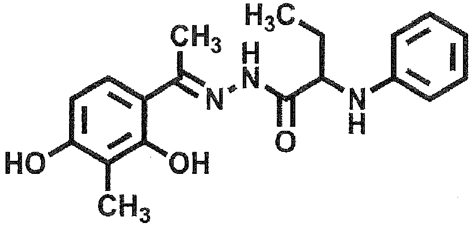 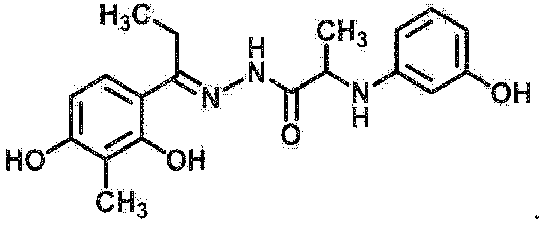
[26] The optically active form or a salt thereof of any of [20] to [22], wherein R₁ is -Y-W-Z-Ar, Y is a single bond, W is N(R₄), R₄ is a hydrogen atom, Z is a methylene group having an alkyl group having 1 - 6 carbon atoms, and Ar is an aryl group optionally having a halogen atom, a hydroxyl group, an alkyl group having 1 - 6 carbon atoms or an alkoxy group having 1 - 6 carbon atoms.
[27] The optically active form or a salt thereof of any of [20] to [22], and [26], wherein the optically active form is an R form.
[28] The optically active form or a salt thereof of any of [20] to [22], [26], and [27], wherein the compound represented by the formula (I) is the following compound:

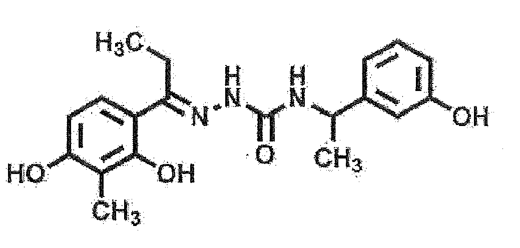

In one embodiment, the present invention is as follows.
[1'] A kinase inhibitor comprising a compound represented by the following formula (I') or a salt thereof: 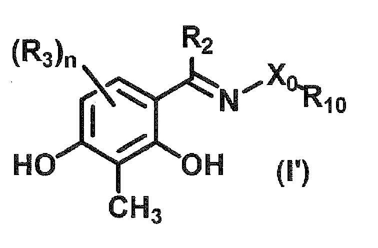 {wherein, X₀ is -NHCO-, R₁₀ is -Y-NH-Z-Ar, wherein Y and Z are each a single bond, or an alkylene group having 1 - 6 carbon atoms and optionally having substituent(s), Ar is an aryl group optionally having substituent(s), R₂ is an alkyl group having 1 - 6 carbon atoms and optionally having substituent(s), R₃ is a hydroxyl group, and n is an integer of 0, 1 or 2}.
[2'] A Hippo signal transduction pathway inhibitor comprising a compound represented by the following formula (I') or a salt thereof: 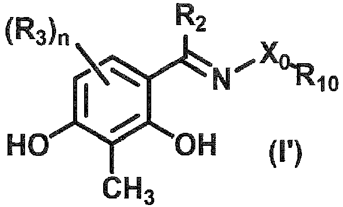 {wherein, X₀ is -NHCO-, R₁₀ is -Y-NH-Z-Ar, wherein Y and Z are each a single bond, or an alkylene group having 1 - 6 carbon atoms and optionally having substituent(s), Ar is an aryl group optionally having substituent(s), R₂ is an alkyl group having 1 - 6 carbon atoms and optionally having substituent(s), R₃ is a hydroxyl group, and n is an integer of 0, 1 or 2}.
[3'] A therapeutic agent for a disease or tissue damage associated with failure of cellular proliferation, comprising a compound represented by the following formula (I') or a salt thereof: 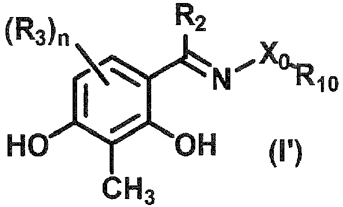 {wherein, X₀ is -NHCO-, R₁₀ is -Y-NH-Z-Ar, wherein Y and Z are each a single bond, or an alkylene group having 1 - 6 carbon atoms and optionally having substituent(s), Ar is an aryl group optionally having substituent(s), R₂ is an alkyl group having 1 - 6 carbon atoms and optionally having substituent(s), R₃ is a hydroxyl group, and n is an integer of 0, 1 or 2}.
[4'] A proliferation promoter of a cell or tissue for a transplantation treatment, comprising a compound represented by the following formula (I') or a salt thereof : 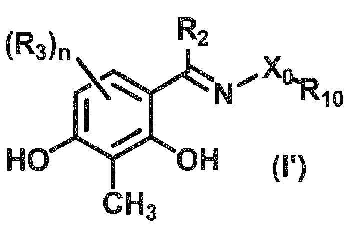 {wherein, X₀ is -NHCO-, R₁₀ is -Y-NH-Z-Ar, wherein Y and Z are each a single bond, or an alkylene group having 1 - 6 carbon atoms and optionally having substituent(s), Ar is an aryl group optionally having substituent(s), R₂ is an alkyl group having 1 - 6 carbon atoms and optionally having substituent(s), R₃ is a hydroxyl group, n is an integer of 0, 1 or 2}.
[5'] The inhibitor, therapeutic agent or proliferation promoter of any of [1'] to [4'], wherein R₂ is a methyl group, an ethyl group, or an isobutyl group,
   n is 0,
   Ar is a phenyl group optionally substituted by a hydroxyl group or a methyl group, and
   Y is a methylene group optionally substituted by a methyl group or an ethyl group.
[6'] The inhibitor, therapeutic agent or proliferation promoter of any of [1'] to [5'], wherein compound (I') is a compound selected from the group consisting of the following: 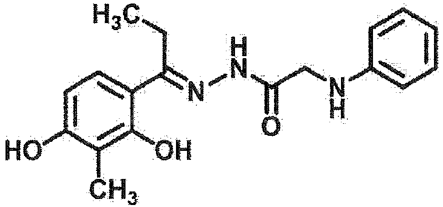 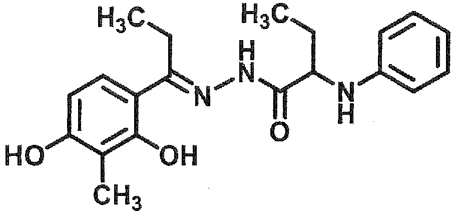 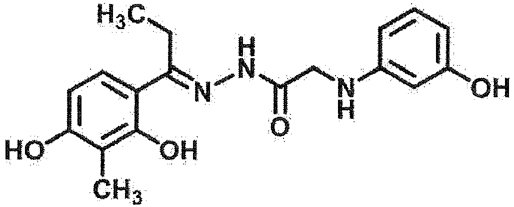 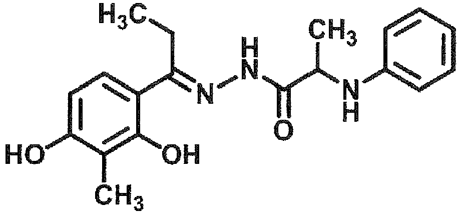 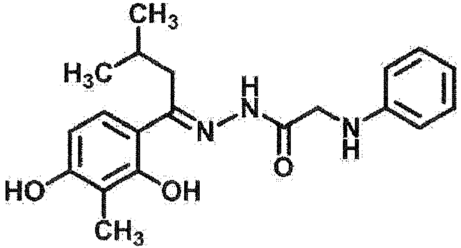 and, 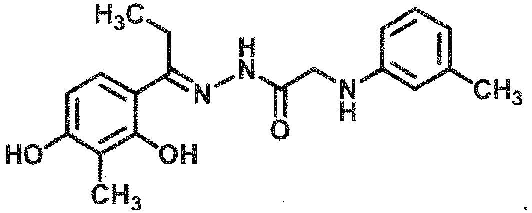
[7'] The inhibitor, therapeutic agent or proliferation promoter of any of [1'] to [5'], wherein compound (I') is a compound represented by the following: 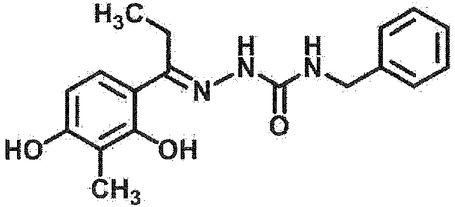 and, 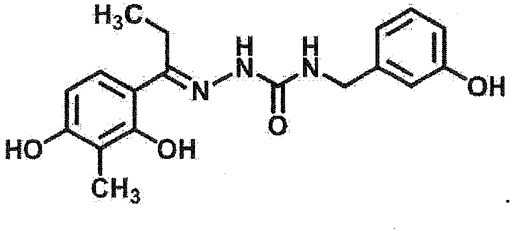
[8'] The inhibitor, therapeutic agent or proliferation promoter of any of [1'], [5'] to [7'], wherein the kinase is selected from the group consisting of CGK2, LATS2, MSK1, p70S6K, PKACα, PKACβ, SGK2, and SGK3.
[9'] The inhibitor, therapeutic agent or proliferation promoter of [8'], wherein the kinase is LATS2.
[10'] The inhibitor, therapeutic agent or proliferation promoter of any of [3'], [5'] to [7'], wherein the disease or tissue damage associated with failure of cellular proliferation is a disease associated with suppression of nuclear translocation of YAP and/or TAZ.
[11'] The inhibitor, therapeutic agent or proliferation promoter of any of [3'], [5'] to [7'], wherein the disease or tissue damage associated with failure of cellular proliferation is selected from the group consisting of inflammatory disease, neurodegenerative disease, immune-nerve disease, muscular dystrophy, myopathy, trauma, burn, spinal cord injury, muscle damage, liver failure, skin damage, brain edema, myocardial infarction, cerebral hemorrhage, and cerebral infarction.
[12'] A method for screening for a substance for treating and/or preventing a disease associated with promoted nuclear translocation of YAP and/or TAZ, comprising the following steps:
   (step 1) a step of culturing cells in a medium containing a compound represented by the following formula (I') or a salt thereof: 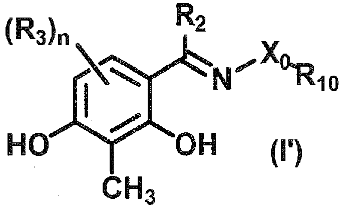 {wherein, X₀ is -NHCO-, R₁₀ is -Y-NH-Z-Ar, wherein Y and Z are each a single bond, or an alkylene group having 1 - 6 carbon atoms and optionally having substituent(s), Ar is an aryl group optionally having substituent(s), R₂ is an alkyl group having 1 - 6 carbon atoms and optionally having substituent(s), R₃ is a hydroxyl group, n is an integer of 0, 1 or 2};
   (step 2) a step of measuring, in the presence of a test substance, an abundance of YAP and/or TAZ in the nucleus of the cell obtained in (step 1);
   (step 3) a step of determining the test substance as a substance for treating and/or preventing a disease associated with promoted nuclear translocation of YAP and/or TAZ when the abundance of YAP and/or TAZ in the nucleus measured in (step 2) is reduced compared to an abundance of YAP and/or TAZ in the absence of the test substance.
[13'] A cell culture medium comprising the inhibitor, therapeutic agent or proliferation promoter of any of [1'], [2'], [4'] to [7'] .

Compound (I') corresponds to a compound represented by the formula (I) wherein X is -NHCO-, R₁ is -Y-W-Z-Ar wherein Y, and Z are each a single bond, or an alkylene group having 1 - 6 carbon atoms and optionally having substituent(s), W is NH, and Ar is an aryl group optionally having substituent(s)).

### [Advantageous Effects of Invention]

According to the present invention, it is possible to inhibit the activity of a plurality of kinases including LATS which is a major kinase in the Hippo signal transduction pathway. In particular, by inhibiting the activity of LATS kinase, it becomes possible to efficiently inhibit the Hippo signal transduction pathway. This promotes the translocation of the YAP protein into the cell nucleus, as a result of which the expression of the target gene group of the YAP or TAZ protein can be promoted. Inhibition of the Hippo signal transduction pathway in cells promotes cell proliferation, which makes it possible to treat diseases associated with failure of cellular proliferation (particularly diseases associated with activation of the Hippo signal transduction pathway) or tissue damage. According to the present invention, moreover, it becomes possible to conduct research on cell functions and diseases, in which the Hippo signal transduction pathway is involved, more quickly and efficiently than before. That is, selective inhibition of the Hippo signal transduction pathway heretofore requires deletion of the genes of MST1 and LATS2 (gene knockout) or suppression of the expression thereof (gene knockdown) by using molecular biological techniques. While these methods are superior in terms of selective inhibition, complicated operations and time are required to obtain cells in which the gene of interest is knocked down or knocked out, and these gene manipulations need to be applied to each cell of interest. However, using the specific compound of the present invention, the Hippo signal transduction pathway can be easily and selectively inhibited by simply adding the compound to the medium during cell culture. In addition, if the compound is removed from the medium, the inhibitory effect disappears. Thus, it is also ideal for the purpose of temporarily inhibiting the Hippo signal transduction pathway. Furthermore, according to the present invention, a therapeutic agent for a disease in which the Hippo signal transduction pathway is involved can be found. That is, when cells are cultured in a medium supplemented with the specific compound of the present invention, the translocation of the YAP into the cell nucleus is promoted, and the phenotype of the disease involving YAP appears more clearly. In such a situation, compounds and factors that inhibit the function of YAP can be searched for more efficiently than before.

### [Brief Description of Drawings]

Fig. 1 shows the amount of YAP, phosphorylated YAP and TAZ in SKOV3 cells cultured in medium supplemented with the specific compound of the present invention, observed by Western blotting method.
Fig. 2 shows the observed changes in liver weight in mice administered with the specific compound of the present invention.
Fig. 3 shows the observed changes in the length of the large intestine in mice administered with the specific compound of the present invention.
Fig. 4 shows the observed changes in the area of skin wounds in mice administered with the specific compound of the present invention.

### [Description of Embodiments]

The terms used in the present specification are defined in the following.

In the present specification, n- means normal, i- means iso, sec- means secondary and tert- means tertiary. In addition, in the present specification, o- means ortho, m-means meta and p- means para.

The "halogen atom" is a fluorine atom, a chlorine atom, a bromine atom, or an iodine atom. The "halogeno group" is fluoro, chloro, bromo, or iodo.

The "alkyl group" and "alkyl group having 1 - 6 carbon atoms" mean a straight chain or branched alkyl group, and specifically, groups such as methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned.

The "aryl group" is, for example, monocyclic, bicyclic, tricyclic or tetracyclic carbon cyclic group in which at least one ring is aromatic and each ring has 5 to 8 ring atoms. Specifically, phenyl, indenyl, naphthyl, fluorenyl and the like can be mentioned. Particularly, the aryl group may be C₆₋₁₀ aromatic phenyl, indenyl, or naphthyl.

The "alkylene group" and "alkylene group having 1 - 6 carbon atoms" mean a straight chain carbon. Specifically, groups such as methylene, ethylene, propylene, butylene, pentylene, hexylene and the like can be mentioned.

The "alkyl group", "aryl group" and "alkylene group" may have a substituent. Examples of such substituent include the following. For "alkyl group", the following (1) to (40) can be mentioned, and the following (1) to (41) can be mentioned for "aryl group" and "alkylene group".
(1) halogeno group,
(2) hydroxyl group,
(3) cyano group,
(4) nitro group,
(5) carboxyl group,
(6) alkenyl group (C₂₋₁₀ alkenyl group; e.g., vinyl, allyl, propenyl, butenyl, pentenyl, hexenyl, heptenyl, butadienyl, hexatrienyl, and each isomer thereof),
(7) alkynyl group (C₂₋₁₀ alkynyl group; e.g., ethynyl, propynyl, butynyl, pentynyl, hexynyl, and each isomer thereof),
(8) halogenoalkyl group (e.g., monofluoromethyl, difluoromethyl, trifluoromethyl, monofluoroethyl, difluoroethyl, trifluoroethyl, chloromethyl, chloroethyl, dichloroethyl, and each isomer thereof),
(9) cyclic alkyl group (optionally having hetero atom in the ring) (e.g., cyclopropyl, cyclobutyl, cyclopentyl, cyclohexyl, tetrahydrofuranyl, tetrahydropyranyl, aziridinyl, azetidinyl, pyrrolidinyl, piperidinyl, morpholinyl),
(10) aryl group (e.g., phenyl, naphthyl),
(11) heteroaryl group (e.g., pyridyl, pyridazinyl, pyrimidinyl, pyrazinyl, furyl, thiophenyl, pyrrolyl, pyrazolyl, imidazolyl, triazolyl (e.g., 1,2,3-triazolyl, 1,2,4-triazolyl), tetrazolyl, oxazolyl, isoxazolyl, thiazolyl, isothiazolyl, oxadiazolyl (e.g., 1,2,3-oxadiazolyl, 1,2,4-oxadiazolyl, 1,3,4-oxadiazolyl), thiadiazolyl (e.g., 1,2,3-thiadiazolyl, 1,2,4-thiadiazolyl, 1,3,4-thiadiazolyl), benzofuryl, benzothiophenyl, indolyl, isoindolyl, benzoxazolyl, benzothiazolyl, benzimidazolyl, indazolyl, benzisoxazolyl, benzisothiazolyl, benzooxadiazolyl, benzothiadiazolyl, purinyl, quinolinyl, isoquinolinyl, cinnolinyl, phthalazinyl, quinazolinyl, quinoxalinyl, pteridinyl, imidazooxazolyl, imidazothiazolyl, imidazoimidazolyl),
(12) alkoxy group (e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(13) alkylthio group (e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(14) alkoxy group (same as in the above-mentioned (12)) substituted by aryl group (same as in the above-mentioned (10)),
(15) alkylthio group (same as in the above-mentioned (13)) substituted by aryl group (same as in the above-mentioned (10)),
(16) alkoxy group (same as in the above-mentioned (12)) substituted by heteroaryl group (same as in the above-mentioned (11)),
(17) alkylthio group (same as in the above-mentioned (13)) substituted by heteroaryl group (same as in the above-mentioned (11)),
(18) cyclic alkyl(optionally having hetero atom in the ring)oxy group (e.g., cyclopropyloxy, cyclobutyloxy, cyclopentyloxy, cyclohexyloxy, tetrahydrofuranyloxy, tetrahydropyranyloxy, aziridinyloxy, azetidinyloxy, pyrrolidinyloxy, piperidinyloxy, morpholinyloxy),
(19) aryloxy group (e.g., group with aryl group (same as in the above-mentioned (10)) bonded to oxygen atom),
(20) heteroaryloxy group (e.g., group with heteroaryl group (same as in the above-mentioned (11)) bonded to oxygen atom),
(21) halogenoalkoxy group (e.g., group with halogenoalkyl group (same as in the above-mentioned (8)) bonded to oxygen atom),
(22) halogenoalkylthio group (e.g., group with halogenoalkyl group (same as in the above-mentioned (8)) bonded to sulfur atom),
(23) alkoxy group (same as in the above-mentioned (12)) substituted by hydroxyl group,
(24) alkoxy group (same as in the above-mentioned (12)) substituted by alkoxy group (same as in the above-mentioned (12)),
(25) amino group,
(26) amino group mono- or di-substituted by alkyl group,
   as used herein, the "alkyl group" is, for example, C₁₋₆ alkyl group. Specifically, methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned,
(27) carbamoyl group,
(28) carbamoyl group mono- or di-substituted by alkyl group (same as "alkyl group" in the above-mentioned (26)) (e.g., methylcarbamoyl, ethylcarbamoyl, dimethylcarbamoyl, diethylcarbamoyl, ethylmethylcarbamoyl),
(29) sulfamoyl group,
(30) sulfamoyl group mono- or di-substituted by alkyl group (same as "alkyl group" in the above-mentioned (26)) (e.g., methylsulfamoyl, ethylsulfamoyl, dimethylsulfamoyl, diethylsulfamoyl, ethylmethylsulfamoyl),
(31) alkanoyl group (e.g., carbonyl group with hydrogen atom or alkyl group (same as "alkyl group" in the above-mentioned (26)) bonded to carbon atom),
(32) aroyl group (e.g., carbonyl group with aryl group (same as in the above-mentioned (10)) bonded to carbon atom),
(33) alkylsulfonylamino group (e.g., sulfonylamino group substituted by alkyl group (same as "alkyl group" in the above-mentioned (26)))
(34) arylsulfonylamino group (e.g., sulfonylamino group substituted by aryl group (same as in the above-mentioned (10))),
(35) heteroarylsulfonylamino group (e.g., sulfonylamino group substituted by heteroaryl group (same as in the above-mentioned (11))),
(36) acylamino group (e.g., amino group substituted by acyl group),
   as used herein, the "acyl group" is an acyl group having a C₁₋₆ alkyl group, or a C₆₋₁₀ aryl group. As used herein, the "C₁₋₆ alkyl group" is the above-mentioned "alkyl group" having 1 - 6 carbon number, and "C₆₋₁₀ aryl group" is the above-mentioned "aryl group" having 6 - 10 carbon number. Specific examples of the acyl group include acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group and the like,
(37) alkoxycarbonylamino group (e.g., carbonylamino group substituted by alkoxy group (same as in the above-mentioned (12))),
(38) alkylsulfonyl group (e.g., sulfonyl group substituted by alkyl group (same as "alkyl group" in the above-mentioned (26))),
(39) alkylsulfinyl group (e.g., sulfinyl group substituted by alkyl group (same as "alkyl group" in the above-mentioned (26))),
(40) alkoxycarbonyl group (e.g., methoxycarbonyl group, ethoxycarbonyl group),
(41) alkyl group (C₁₋₆ alkyl group; e.g., methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl etc.) and the like.

When two or more substituents are present, they may be the same or different.

The compound of the formula (I) (hereinafter to be also referred to as a specific compound) may be in the form of a salt. Examples of the salt of the aforementioned compound represented by the formula (I) include salts with inorganic acids such as hydrochloric acid and hydrobromic acid, and salts with organic acids such as acetic acid, propionic acid, tartaric acid, fumaric acid, maleic acid, malic acid, oxalic acid, succinic acid, citric acid and benzoic acid.

The compound represented by the formula (I) may contain geometric isomers of an E-form having an E-steric configuration and Z-form having a Z-steric configuration depending on the type of the substituent. The present invention includes E-form, Z-form or a mixture containing E-form and Z-form in any ratio.

The compound represented by the formula (I) may have an optically active substance due to the presence of one or more asymmetric carbon atoms. The compound represented by the formula (I) includes all optically active or racemic compounds.

### [Synthesis of compound represented by the formula (I)]

The compound represented by the formula (I) is preferably obtained by, as shown in the following reaction scheme, using 1 equivalent each of a ketone compound and H₂N-X-R₁ wherein X and R₁ are as defined above, for example, hydrazide compound, semicarbazide compound etc.), and performing the reaction in a solvent such as toluene, 1,4-dioxane, N,N-dimethylformamide, dimethyl sulfoxide and the like at not less than 100°C for 1 hr to 3 days.

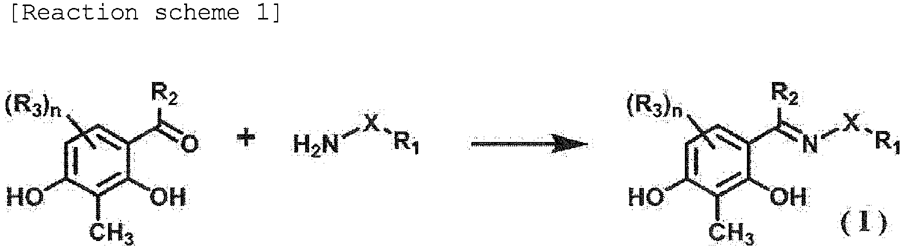

The reaction mixture after completion of the reaction is precipitated by adding distilled water, or when no precipitation occurs, a general post-treatment such as concentration after extraction with an organic solvent is performed to obtain the specific target compound to be used in the present invention. When purification is necessary, the compound can be separated and purified by any purification method such as recrystallization, column chromatography, thin-layer chromatography, liquid chromatography and the like.

### [Obtainment of optically active form]

Among the compounds synthesized by the aforementioned method, those having optical isomers may have an optically active form (eutomer). An optically active form can be obtained by a method known per se, such as a method by crystallization, a method using an enzymatic reaction, or a method using HPLC (e.g., an optically active support carrier method). Further, the optically active substance can also be prepared using an asymmetric synthesis method or the like. In addition, in the present specification, an "optically active form" means an optical isomer which is judged to have stronger activity at least in the activity of inhibiting the kinase activity of LATS 1 or 2.

### 1. Kinase inhibitor

The present invention provides a kinase inhibitor containing the following specific compound or a salt thereof (hereinafter sometimes referred to as "the kinase inhibitor of the present invention").

The compound contained in the kinase inhibitor of the present invention is a compound represented by the formula (I): 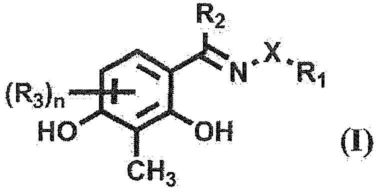 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}, or a salt thereof (hereinafter a compound represented by the formula (I) and a salt thereof are sometimes to be generically referred to simply as "the specific compound to be used in the present invention").

In one embodiment, when X in the formula (I) is -NHCO-, R₁ is -Y-W-Z-Ar, and Y is an alkylene group having 1 - 6 carbon atoms and optionally having substituent(s) (preferably, an alkylene group having 1 - 6 carbon atoms and optionally having an alkyl group having 1 - 6 carbon atoms, more preferably a methylene group optionally substituted by a methyl group or an ethyl group), then W is N(R₄) (more preferably, N(R₄) wherein R₄ is a hydrogen atom or an alkyl group having 1 - 6 carbon atoms, particularly preferably, N(R₄) wherein R₄ is a hydrogen atom), Z is a single bond, Ar is an aryl group optionally having substituent(s) (preferably, a phenyl group having a substituent, more preferably, a phenyl group substituted by a halogeno group, a hydroxyl group, a methyl group or an ethoxy group, or a naphthyl group), R₂ is an alkyl group having 1 - 6 carbon atoms and optionally having substituent(s) (more preferably, an alkyl group having 1 - 6 carbon atoms, particularly preferably, a methyl group, an ethyl group, or an isopropyl group), and n=0.

When X in the formula (I) is -NHCO-, and R₁ is -Y-W-Z-Ar, and Y is a single bond, then W is N(R₄) (more preferably, N(R₄), R₄ is a hydrogen atom or an alkyl group having 1 - 6 carbon atoms, particularly preferably, N(R₄), R₄ is a hydrogen atom), or an oxygen atom, Z is a single bond or an alkylene group optionally having substituent(s) (more preferably, an alkylene group optionally having a substituent, particularly preferably, a methylene group optionally having a substituent), Ar is an aryl group optionally having a substituent (a halogen atom, a hydroxyl group, a methyl group, or a methoxy group, etc.) (more preferably, an aryl group having a hydroxyl group or an aryl group not having a substituent, particularly preferably, a phenyl group having a hydroxyl group or a phenyl group), R₂ is an alkyl group having 1 - 6 carbon atoms and optionally having substituent(s) (more preferably, an alkyl group having 1 - 6 carbon atoms, particularly preferably, a methyl group, an ethyl group, or an isopropyl group), and n=0.

In one preferable embodiment, the compound represented by the formula (I) may be any of the compounds shown below:

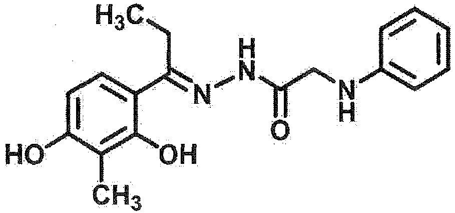

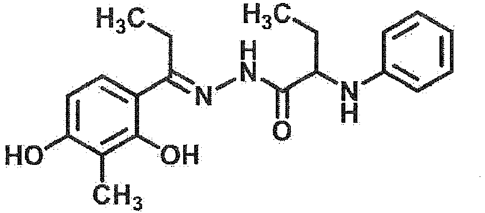

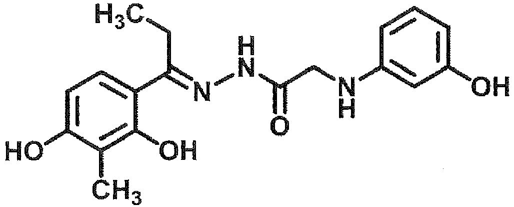

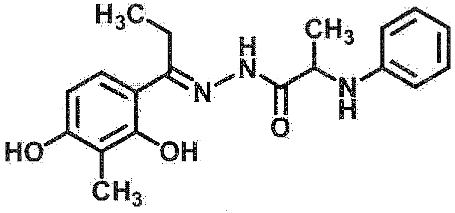

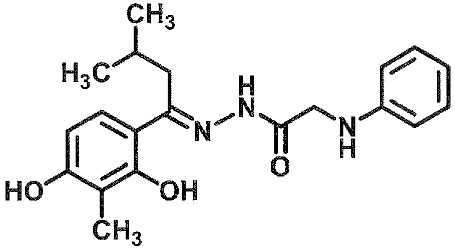

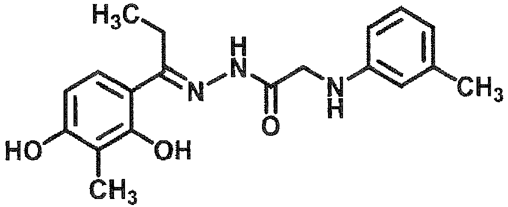

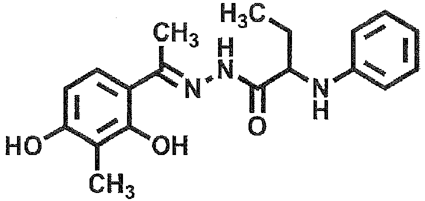

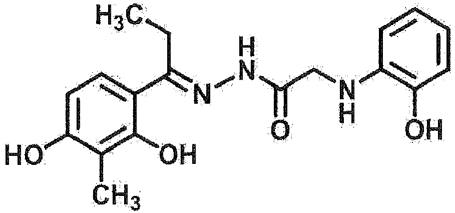

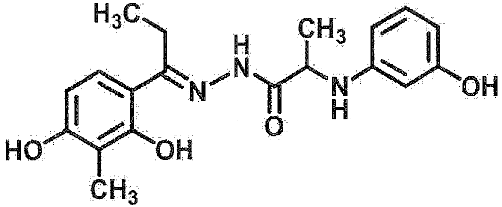

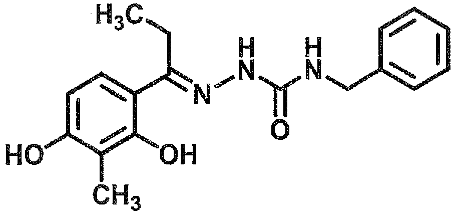

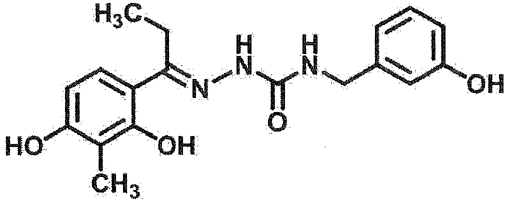

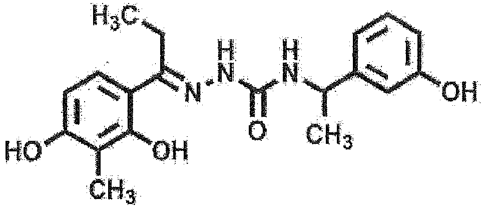

In one preferable embodiment, the compound represented by the formula (I) may be an R form among the optical isomers of the compounds shown below.

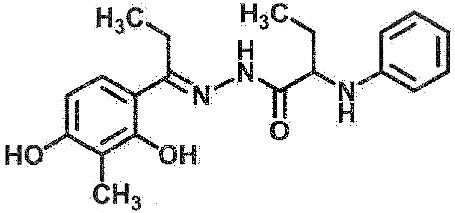

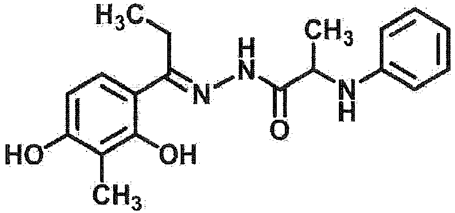

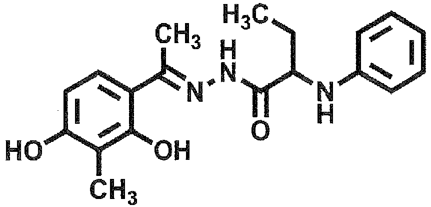

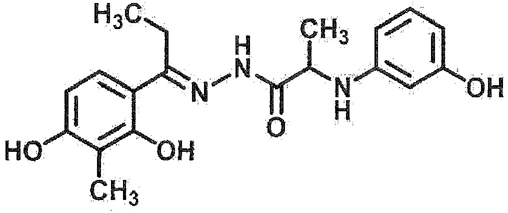

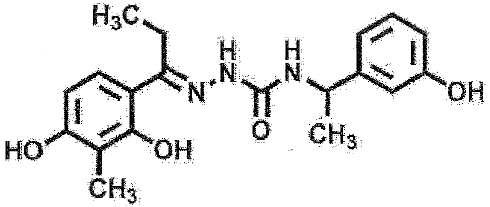

The amount of the specific compound of the present invention which is contained in the kinase inhibitor of the present invention is not particularly limited as long as the desired effect of the present invention is obtained. It may be generally 0.01 - 100 wt%, preferably 0.1 - 100 wt%, more preferably 1 - 100 wt%, further preferably 5 - 100 wt%, particularly preferably 10 - 100 wt%.

The kinase inhibitor of the present invention can have any shape during provision or preservation. The composition may be in the form of a formulated solid such as tablet, pill, capsule, granule, or a liquid such as a solution obtained by dissolving in an appropriate solvent using a solubilizer or a suspension, or may be bonded to a substrate or a carrier. Examples of the solvent used for formulating include aqueous solvents such as water, saline, dimethyl sulfoxide (DMSO), various alcohols (e.g., methanol, ethanol, butanol, propanol, glycerol, propylene glycol, butylene glycol and the like), and the like. Examples of the additive used formulating include preservatives such as p-hydroxybenzoic acid esters and the like; excipients such as lactose, glucose, sucrose, mannit and the like; lubricants such as magnesium stearate, talc and the like; binders such as poly(vinyl alcohol), hydroxypropylcellulose, gelatin and the like; surfactants such as fatty acid ester and the like; plasticizers such as glycerol and the like; and the like. These additives are not limited to those mentioned above, and can be selected freely as long as they are utilizable for those of ordinary skill in the art.

The kinase inhibitor of the present invention may be sterilized as necessary. The sterilization method is not particularly limited, and, for example, radiation sterilization, ethylene oxide gas sterilization, autoclave sterilization, filter sterilization and the like can be mentioned. When filter sterilization (hereinafter sometimes to be referred to as filtration sterilization) is to be performed, the material of the filter part is not particularly limited and, for example, glass fiber, nylon, PES (polyethersulfone), hydrophilic PVDF (polyvinylidene fluoride), cellulose mixed ester, celluloseacetate, polytetrafluoroethylene and the like can be mentioned. While the size of the pore in the filter is not particularly limited, it is preferably 0.1 µm to 10 µm, more preferably 0.1 µm to 1 µm, most preferably 0.1 µm to 0.5 µm. The sterilization treatment may be applied when the specific compound is in a solid state or a solution state.

In the kinase inhibitor of the present invention, "inhibiting kinase" means inhibiting the phosphorylation function of kinase to make its activity disappear or attenuate. Examples of the kind of kinase to be inhibited by the kinase inhibitor of the present invention include, but are not limited to, CGK2, LATS2, MSK1, p70S6K, PKACα, PKACβ, SGK2, and SGK3.
In the present specification, "attenuating the activity of kinase" means, for example, attenuating the activity of kinase by at least 5% or more, at least 10% or more, at least 15% or more, at least 20% or more, at least 25% or more, at least 30% or more, at least 35% or more, at least 40% or more, at least 50% or more, at least 55% or more, at least 60% or more, at least 65% or more, at least 70% or more, at least 75% or more, at least 80% or more, at least 85% or more, at least 90% or more, at least 95% or more, or at least 99% or more, as compared to the phosphorylation activity level of a given kinase to be the control. For the measurement of the kinase activity, a method known per se such as MSA method and the like can be used as explained in the following Examples. Alternatively, when the kinase is LATS2 or the like located at the upstream of the Hippo signal transduction pathway, the degree of attenuation of the kinase activity can also be indirectly evaluated by confirming the intracellular localization of YAP and/or TAZ, which are/is downstream effector(s) of LATS2 or the like and the target of phosphorylation, by a molecular biological method known per se.

In one embodiment of the present invention, when the kinase is LATS2, "inhibiting LATS2" means inhibiting the function of LATS2 as kinase to make its activity disappear or attenuate. LATS2 is evolutionarily conserved in a wide range of species from yeast to human. Therefore, LATS2 whose activity is inhibited by the kinase inhibitor of the present invention can be LATS2 in all organisms, and may be LATS of mammals such as rat, mouse, rabbit, guinea pig, squirrel, hamster, vole, duckbill, dolphin, whale, dog, cat, goat, bovine, horse, sheep, swine, elephant, Common marmoset, squirrel monkey, rhesus monkey, chimpanzee, human and the like. LATS2 to be inhibited by the kinase inhibitor of the present invention may be preferably human LATS. When simply referred to as "LATS" in the present specification, it is a concept including both "LATS1" and "LATS2" and a complex of LATS1 and LATS2.

Examples of the embodiment of use of the kinase inhibitor of the present invention include, but are not limited to, use for molecular biological tests and research purposes, and the like. In one embodiment of the use for molecular biological tests and research purposes, the kinase inhibitor of the present invention is administered to a cell that expresses a specific kinase such as LATS2 or the like, whereby a cell showing suppressed activity of the specific kinase can be easily prepared. The thus-obtained cells may be useful for functional analysis of a specific kinase, screening of candidate substances capable of regulating the function of the specific kinase, and the like.

In one embodiment, a cell expressing a specific kinase may be a cell derived from a mammal. Examples of the cell include, but are not limited to, somatic cells constituting the living body, normal cell lines, cancer cell lines, progenitor cells, stem cells, cells separated from the living body and applied with artificial genetic modification, cells separated from the living body wherein the nucleus is artificially exchanged and the like. While the derivation of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, rhesus monkey, chimpanzee, human and the like can be mentioned. The tissue or organ from which the cells are derived is not particularly limited. Examples of the tissue include tissues such as skin, kidney, spleen, adrenal gland, liver, lung, ovary, pancreas, uterus, stomach, colon, small intestine, large intestine, bladder, prostate, testis, thymus, muscle, bond tissue, bone, joints, blood vessel tissue, blood, heart, eye, brain, nerve tissue and the like. Examples of the aforementioned organ include organs such as liver, lung, kidney, heart, pancreas, stomach, spleen, small intestine, large intestine, reproductive organ and the like. In the present specification, the "stem cell" means a cell concurrently having an ability to replicate itself, and an ability to differentiate into other plural lineages. Examples thereof include, but are not limited to, embryonic stem cells (ES cells), embryonic tumor cells, embryonic germ stem cells, induced pluripotent stem cells (iPS cells), neural stem cells, hematopoietic stem cells, mesenchymal stem cells, liver stem cells, pancreas stem cells, muscle stem cells, germ stem cells, intestinal stem cells, cancer stem cells, hair follicle stem cells and the like. Examples of the pluripotent stem cells include ES cells, embryonic germ stem cells and iPS cells, from among the aforementioned stem cells. Progenitor cells are cells on the way to differentiate from the aforementioned stem cells into specific somatic cells or germ cells.

The kinase inhibitor of the present invention is administered to a cell that expresses a specific kinase by adding the kinase inhibitor of the present invention to a medium in which the cell is cultured. The amount of the kinase inhibitor of the present invention to be added is not particularly limited as long as the desired effect of the present invention is achieved. For example, the concentration of the specific compound to be used in the present invention in the medium is generally 0.001 - 100 µM, preferably 0.01 M - 50 uM, more preferably 0.1 - 30 µM, further preferably 1 - 20 µM, particularly preferably 5 - 10 µM.

When the kinase inhibitor of the present invention is used, the medium in which the cells are cultured is not particularly limited, and a commercially available medium can also be used. Preferable examples of the commercially available medium include, but are not limited to, Dulbecco's modified Eagle medium (Dulbecco's Modified Eagle's medium; DMEM), ham F12 medium (Ham's Nutrient MixtureF12), DMEM/F12 medium, McCoy's 5A medium (McCoy's 5A medium), Eagle MEM (Eagle's Minimum Essential medium; EMEM), αMEM (alpha Modified Eagle's Minimum Essential medium; αMEM), MEM (Minimum Essential medium), RPMI1640 medium, Iscove's Modified Dulbecco's medium (Iscove's Modified Dulbecco's medium; IMDM), MCDB131 medium, Williams' medium E, IPL41 medium, Fischer's medium, StemPro34 (manufactured by Invitrogen), X-VIVO 10 (manufactured by Cambrex), X-VIVO 15 (manufactured by Cambrex), HPGM (manufactured by Cambrex), StemSpan H3000 (manufactured by STEMCELL Technologies), StemSpanSFEM (manufactured by STEMCELL Technologies), StemlineII (manufactured by Sigma-Aldrich), QBSF-60 (manufactured by Quality Biological), StemProhESCSFM (manufactured by Invitrogen), Essential8 (registered trade mark) medium (manufactured by Gibco), mTeSR1 or 2 medium (manufactured by STEMCELL Technologies), TeSR-E8 medium (manufactured by STEMCELL Technologies), Repro FF or Repro FF2 (manufactured by ReproCELL), Primate ES Cell medium (manufactured by ReproCELL), PSGro hESC/iPSC medium (manufactured by System Biosciences), NutriStem (registered trade mark) medium (manufactured by Biological Industries), StemFit (registered trade mark) medium (manufactured by Ajinomoto Co., Inc.), CSTI-7 medium (manufactured by Cell Science & Technology Institute, Inc.), MesenPRO RS medium (manufactured by Gibco), MF- medium (registered trade mark) mesenchymal stem cell proliferation medium (manufactured by TOYOBO CO., LTD.), mesenchymal stem cell medium (manufactured by PromoCell GmbH), Sf-900II (manufactured by Invitrogen), Opti-Pro (manufactured by Invitrogen), keratinocyte basal medium 2 (manufactured by PromoCell), keratinocyte proliferation medium 2 (manufactured by PromoCell), normal human epidermal keratinocyte proliferation medium (manufactured by Kurabo Industries Ltd.), normal human epidermal keratinocyte basal medium (manufactured by Kurabo Industries Ltd.), human EpiVita proliferation medium (manufactured by TOYOBO CO., LTD.), EpiLife (registered trade mark) medium (manufactured by Thermo Fisher Scientific), cornea epithelial cell basal medium (ATCC (registered trade mark), PCS-700-030 (registered trade mark)) and the like.

In addition, it is possible to appropriately add, according to the purpose of test and researches, further substances such as sodium, potassium, calcium, magnesium, phosphorus, chlorine, various amino acids, various vitamins, antibiotics, serum, fatty acids, sugars, cell growth factors, differentiation inducing factors, cell adhesion factors, antibodies, enzymes, cytokines, hormones, lectins, extracellular matrices, bioactive substances, and the like to the above-mentioned medium. Specific examples include, but are not limited to, basic fibroblast growth factor, transforming growth factor-α, transforming growth factor-β, epidermal growth factor, insulin-like growth factor, Wnt protein, amphiregulin, interferons, interleukins, vascular endothelial cell growth factor, platelet derived from growth factor, hepatocyte growth factor, albumin, insulin, β-mercaptoethanol, Knockout Serum Replacement (KSR, manufactured by Thermo Fisher Scientific), Glutamax (manufactured by Thermo Fisher Scientific), hydrocortisone, epinephrine, L-glutamine, pyruvic acid, selenous acid and the like.

The cell culture conditions (e.g., temperature, carbon dioxide concentration, culture period etc.) may be those known per se, or may be appropriately modified according to the purpose. For example, the temperature for culturing cells is generally 25 - 39°C, preferably 33 - 39°C (e.g., 37°C). The carbon dioxide concentration is generally 4% - 10% by volume, preferably 4% - 6% by volume, in the atmosphere of culture. The culture period is generally 1 to 35 days, which can be appropriately set according to the purpose of the culture.

### 2. Hippo signal transduction pathway inhibitor

The present invention also provides a Hippo signal transduction pathway inhibitor containing the specific compound used in the present invention (hereinafter sometimes referred to as "the Hippo signal transduction pathway inhibitor of the present invention").

Hippo signal transduction pathway is a signal transduction pathway involved in cell proliferation, cell death, organ size, self-renewal, differentiation of stem cell and progenitor cell, wound therapy, and tissue regeneration. This pathway is evolutionarily conserved in various kinds of organisms, particularly highly conserved in mammals. Key factors in the Hippo signal transduction pathway in mammals include LATS1 and LATS2. LATS is activated by association with the scaffold protein Mob1A/B. LATS is also activated by phosphorylation by STE20 family protein kinases Mst1 and Mst2. LATS phosphorylates downstream effectors YAP and TAZ which are transcription co-factors. Phosphorylation of YAP and TAZ by LATS is a very important event in the Hippo signal transduction pathway. Phosphorylated YAP and TAZ transfer from the cell nucleus to the cytoplasm and are degraded by the ubiquitin proteasome system. Therefore, when the Hippo signal transduction pathway is in an activated state, YAP and/or TAZ are/is phosphorylated by LATS, transferred into the cytoplasm and degraded. On the other hand, when the Hippo signal transduction pathway is inactive, YAP and/or TAZ are/is not phosphorylated and are localized in the cell nucleus.

As described above, YAP and/or TAZ are/is transcription co-factor(s), and are known to induce the expression of various genes when localized in the cell nucleus. Many of the genes whose expression is induced by YAP and/or TAZ are known to mediate cell survival and proliferation. Examples of such gene include, but are not limited to, CCND1, CTGF, BIRC2, CYR61, AMOTL2, TGFB2, ANKRD1 and the like. The CCND1 gene (also referred to as CYCLIN D1, "PRAD1", etc.) is present on chromosome 11 in human. The protein encoded by the gene (295 amino acids, molecular weight: about 36 kDa) relates to promoting the cell cycle. The CTGF gene (connective tissue growth factor, also referred to as "CCN2", etc.) is present on chromosome 6 in human and encodes a polypeptide (349 amino acids, molecular weight: about 35 kDa) that induces promotion of cell growth and the like. The BIRC2 gene (baculoviral IAP repeat containing 2, also referred to as "API1", etc.) is present on chromosome 11 in human and encodes a protein that inhibits apoptosis by caspase-degrading activity (618 amino acids, molecular weight: about 70 kDa). The CYR61 gene (cysteine rich angiogenic inducer 61, also referred to as "CCN1", etc.) is present on chromosome 1 in human. The protein encoded by the gene (381 amino acids, molecular weight: about 39 kDa) plays an important role in angiogenesis, VEGF enhancement, and bone formation. The AMOTL2 gene (angiomotin like 2, also referred to as "LCCP") is present on chromosome 3 (3q22.2) in human. The protein encoded by the gene (837 amino acids, molecular weight: about 92 kDa) relates to angiomotin, which inhibits angiogenesis, and belongs to the motin protein family. The ANKRD1 gene (Ankyrin repeat domain-containing protein 1, Cardiac adriamycin-responsive protein, also referred to as "CARP") is present on chromosome 10 in human. The protein encoded by the gene (319 amino acids, molecular weight: about 36 kDa) is highly expressed in cardiac muscle and skeletal muscle and functions as a transcription factor.

The Hippo signal transduction pathway inhibitor of the present invention inhibits the kinase activity of LATS (particularly LATS2), and inhibits the phosphorylation of YAP and/or TAZ by LATS. Since YAP and/or TAZ are/is not phosphorylated, the nuclear translocation of YAP and/or TAZ is promoted. As a result, gene expression of genes whose expression is induced by YAP and/or TAZ is enhanced. Therefore, the Hippo signal transduction pathway inhibitor of the present invention can also be paraphrased as "nuclear translocation_ promoter of YAP and/or TAZ", or "promoter of gene expression induced by YAP and/or TAZ".

In the present specification, "promoting nuclear translocation of YAP and/or TAZ" means that the abundance of YAP and/or TAZ is increased in the nucleus rather than in the cytoplasm by inhibiting phosphorylation and proteolysis of YAP and/or TAZ. Increasing the abundance of YAP and/or TAZ means that, for example, the abundance of YAP and/or TAZ in the nucleus increases at least 1.3 times or more, at least 1.5 times or more, at least 2 times or more, at least 3 times or more, at least 4 times or more, at least 5 times or more, at least 6 times or more, at least 7 times or more, at least 8 times or more, at least 9 times or more, at least 10 times or more, as compared to the abundance of YAP and/or TAZ to be the control in the nucleus. The abundance of YAP and/or TAZ in the nucleus can be measured by a method known per se such as imaging analysis using an anti-YAP (or TAZ) antibody bound with a fluorescent label, as used in Examples described later, and the like. In addition, the abundance of YAP and/or TAZ in the nucleus can be indirectly measured by observing the phosphorylation state of YAP and/or TAZ by using a Western blotting method or the like.

In the present specification, "promotion of gene expression induced by YAP and/or TAZ" means that, as a result of promotion of transfer of YAP and/or TAZ into the cell nucleus, the level of gene expression induced by YAP and/or TAZ (e.g., at least one or more of CCND1, CTGF, BIRC2, CYR61, AMOTL2, TGFB2, ANKRD1) is enhanced. That the gene expression is promoted means, for example, that the expression level of at least one or more genes of the above-mentioned genes increases at least 1.3 times or more, at least 1.5 times or more, at least 2 times or more, at least 3 times or more, at least 4 times or more, at least 5 times or more, at least 6 times or more, at least 7 times or more, at least 8 times or more, at least 9 times or more, at least 10 times or more, as compared to the expression level of a given control amount (e.g., cell population free of addition of the Hippo signal transduction pathway inhibitor of the present invention). The promotion of gene expression may be evaluated at the transcription level by using a microarray method, a real-time PCR method, or the like, or at the protein translation level by using a Western blotting method or the like.

The amount, dosage form, sterilization treatment and the like of the specific compound used in the present invention in the Hippo signal transduction pathway inhibitor of the present invention are similar to those explained in 1. Kinase inhibitor of the present invention.

Examples of the embodiment of use of the Hippo signal transduction pathway inhibitor of the present invention include, but are not limited to, use for molecular biological tests and research purposes, and the like. As a specific example of the use for molecular biological tests and research purposes, a cell with inhibited Hippo signal transduction pathway can be easily prepared by administering the Hippo signal transduction pathway inhibitor of the present invention to a cell having the Hippo signal transduction pathway. As described above, the Hippo signal transduction pathway inhibitor of the present invention promotes transfer of YAP and/or TAZ into the cell nucleus, so that the phenotype of the disease involving YAP and/or TAZ appears more clearly in the cell. Therefore, by using cells having such properties, it is possible to efficiently search for a substance that can regulate the functions of YAP and/or TAZ.

In one embodiment, a cell having Hippo signal transduction pathway may be a cell derived from a mammal. Examples of such cell include, but are not limited to, somatic cells constituting the living body, normal cell line, cancer cell line, progenitor cells, stem cell, cells separated from the living body and applied with artificial genetic modification, cells separated from the living body wherein the nucleus is artificially exchanged and the like. While the derivation of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, rhesus monkey, chimpanzee, human and the like can be mentioned. The tissue or organ from which the cells are derived is not particularly limited. Examples of the tissue include tissues such as skin, kidney, spleen, adrenal gland, liver, lung, ovary, pancreas, uterus, stomach, colon, small intestine, large intestine, bladder, prostate, testis, thymus, muscle, bond tissue, bone, joints, blood vessel tissue, blood, heart, eye, brain, nerve tissue and the like. Examples of the organ include organs such as liver, lung, kidney, heart, pancreas, stomach, spleen, small intestine, large intestine, reproductive organ and the like.

The Hippo signal transduction pathway inhibitor of the present invention is administered to a cell having Hippo signal transduction pathway by adding the Hippo signal transduction pathway inhibitor of the present invention to a medium in which the cell is cultured. The amount of the kinase inhibitor of the present invention to be added is not particularly limited as long as the desired effect of the present invention is achieved. For example, the concentration of the specific compound to be used in the present invention in the medium is generally 0.001 - 100 µM, preferably 0.01 - 50 µM, more preferably 0.1 - 30 µM, further preferably 1 - 20 µM, particularly preferably 5 - 10 µM.

When the Hippo signal transduction pathway inhibitor of the present invention is used, the medium in which the cells are cultured is not particularly limited, and a commercially available medium can also be used. The commercially available medium that can be used when using the Hippo signal transduction pathway inhibitor of the present invention is the same as the commercially available medium that can be used when using the kinase inhibitor of the present invention. In addition, the cell culture conditions and the like are also the same as those described for the kinase inhibitor of the present invention.

### 3. Therapeutic agent for disease or tissue damage associated with failure of cellular proliferation

The present invention also provides a therapeutic agent for a disease or tissue damage associated with failure of cellular proliferation which contains the specific compound used in the present invention (hereinafter sometimes referred to as "the therapeutic agent for a disease or tissue damage associated with failure of cellular proliferation of the present invention").

Hippo signal transduction pathway is a signal transduction pathway involved in cell proliferation, cell death, organ size, self-renewal, differentiation of stem cell and progenitor cell, wound therapy, and tissue regeneration. Particularly, cell proliferation, wound therapy, and tissue regeneration were promoted by inhibiting the Hippo signal transduction pathway. Therefore, In one embodiment, the Hippo signal transduction pathway inhibitor of the present invention can also be used for diseases associated with failure of cellular proliferation and regeneration of damaged tissues.

The amount, sterilization treatment and the like of the specific compound used in the present invention in the therapeutic agent for a disease or tissue damage associated with failure of cellular proliferation of the present invention are similar to those explained in 1. Kinase inhibitor of the present invention.

The therapeutic agent for a disease or tissue damage associated with failure of cellular proliferation of the present invention may be used alone or may be used in combination with at least one kind of other therapeutic drug. In this case, the therapeutic agent for a disease or tissue damage associated with failure of cellular proliferation of the present invention may be administered simultaneously with the therapeutic drug, or may be administered prior to or after the administration of the therapeutic drug. The therapeutic agent for a disease or tissue damage associated with failure of cellular proliferation of the present invention may be administered by an administration route the same as or different from that of the therapeutic drug. Examples of the therapeutic drug include chemotherapy drugs, supporting therapeutic drugs, and a combination thereof.

In the present specification, "treating " means partially or substantially achieving one or more of partially or completely reducing the extent of a disease, improving or ameliorating clinical symptoms or indicators related to a disease, slowing, suppressing, or preventing the progression of a disease, and partially or completely slowing, suppressing, or preventing the onset or progression of a disease.

The animals to which the therapeutic agent for a disease or tissue damage associated with failure of cellular proliferation of the present invention is applied is generally mammal, preferably human, or may be pets (e.g., dog, cat, guinea pig, rabbit, squirrel etc.) or domestic animals (bovine, sheep, swine, horse etc.).

The therapeutic agent for a disease or tissue damage associated with failure of cellular proliferation of the present invention can be generally administered as oral administration agents such as tablet, capsule, powder, granule, pill, syrup and the like, rectal administration agent, percutaneous absorber or injection. The agent can be administered as a single therapeutic drug or as a mixture with other therapeutic drugs. They may be administered alone, or are generally administered in the form of pharmaceutical compositions. These preparations can be produced by a conventional method by adding pharmacologically and pharmaceutically acceptable additives. That is, additives such as general excipient, lubricant, binder, disintegrant, wetting agent, plasticizer, coating agent and the like can be used for the oral preparation. Oral solution may be in the form of aqueous or oily suspension, solution, emulsion, syrup, elixir, or the like, or may be provided as dry syrup to be prepared with water or other suitable solvent prior to use. The aforementioned liquids can contain general additives such as suspending agent, flavor, diluent and emulsifier. For intrarectal administration, they can be administered as suppository. Suppositories use suitable substances such as cocoa butter, lauric oil, macrogol, glycerol gelatin, Witepsol, sodium stearate or mixtures thereof as a base, and emulsifier, suspending agent, preservative and the like can be added as necessary. For injections, preparation components such as dissolving agent or solubilizing agent (e.g., distilled water for injection, physiological saline, 5% glucose solution, propylene glycol, and the like), pH adjuster, isotonicity agent, stabilizer and the like are used to form an aqueous dosage form or a dosage form for dissolution at the time of use. Examples of the topical administration agent include spray, aerosol, cream, ointment, lotion, liniment, and gel, preferably spray, cream, ointment, or gel. Where necessary, these preparations can contain additives such as absorption promoter, pH adjuster, preservative, flavor, dispersing agent, wetting agent, stabilizer, antiseptic, suspension, surfactant, antibacterial agent and the like. The therapeutic agent of the present invention can be used together with a coating material. Examples thereof include gauze, polyurethane film, hydrocolloid, polyurethane foam, alginate coating material, hydrogel, hydrofiber, hydropolymer and the like. In the following, examples of the preparation of the therapeutic agent for a disease or tissue damage associated with failure of cellular proliferation of the present invention and a preparation method thereof are illustrated; however, the present invention is not limited thereto.

### Preparation Example 1

Granules containing the following components are produced. Components

| | |
|---|---|
| compound represented by formula (I) | 10 mg |
| lactose | 700 mg |
| cornstarch | 274 mg |
| HPC-L | 16 mg |
| total | 1000 mg |

A compound represented by the formula (I) and lactose are passed through a 60-mesh sieve. Cornstarch is passed through a 120 mesh sieve. These are mixed in a V-type mixer. Low-viscosity hydroxypropyl cellulose (HPC-L) aqueous solution is added to the mixed powder, kneaded and granulated (extrusion-granulation pore size 0.5 - 1 mm), and then dried. The obtained dried granules are sieved with a vibrating sieve (12/60 mesh) to obtain granules.

### Preparation Example 2

Powder to be filled in a capsule and containing the following components is produced.

### Components

| | |
|---|---|
| compound represented by formula (I) | 10 mg |
| lactose | 79 mg |
| cornstarch | 10 mg |
| magnesium stearate | 1 mg |
| total | 100 mg |

A compound represented by the formula (I) and lactose are passed through a 60-mesh sieve. Cornstarch is passed through a 120 mesh sieve. These and magnesium stearate are mixed in a V-type mixer. The 10% powder (100 mg) is filled in a No. 5 hard gelatin capsule.

### Preparation Example 3

Granules to be filled in a capsule and containing the following components are produced.

### Components

| | |
|---|---|
| compound represented by formula (I) | 15 mg |
| lactose | 90 mg |
| cornstarch | 42 mg |
| HPC-L | 3 mg |
| total | 150 mg |

A compound represented by the formula (I) and lactose are passed through a 60-mesh sieve. Cornstarch is passed through a 120 mesh sieve. These are mixed in a V-type mixer. Low-viscosity hydroxypropyl cellulose (HPC-L) aqueous solution is added to the mixed powder, kneaded and granulated, and then dried. The obtained dried granules are sieved and screened with a vibrating sieve (12/60 mesh), and 150 mg thereof is filled in a No. 4 hard gelatin capsule.

### Preparation Example 4

Tablet containing the following components is produced.

### Components

| | |
|---|---|
| compound represented by formula (I) | 10 mg |
| lactose | 90 mg |
| microcrystalline cellulose | 30 mg |
| magnesium stearate | 5 mg |
| CMC-Na | 15 mg |
| total | 150 mg |

A compound represented by the formula (I), lactose, crystalline cellulose, and CMC-Na (carboxymethylcellulose sodium salt) are passed through a 60-mesh sieve and mixed. Magnesium stearate is added to the mixed powder to obtain a mixed powder for preparation. The mixed powder is directly compressed to obtain a 150 mg tablet.

### Preparation Example 5

Intravenous preparation is produced as follows.

| | |
|---|---|
| compound represented by the formula (I) | 100 mg |
| saturated fatty acid glycerides | 1000 mL |

A solution of the above-mentioned components is generally administered intravenously to patients at a rate of 1 mL per min.

### Preparation Example 6

Liquid is produced as follows.

| | |
|---|---|
| compound represented by formula (I) | 10 mg |
| ethanol | 1 mL |
| water | 99 mL |

The above-mentioned components are mixed.

### Preparation Example 7

Liquid is produced as follows.

| | |
|---|---|
| compound represented by formula (I) | 1 g |
| Pluronic F-68 | 10 g |
| propylene glycol | 20 mL |

| | |
|---|---|
| tris hydroxymethylaminomethane | 2.4 g |
| water | 970 mL |

The above-mentioned components are mixed.

When the therapeutic agent for a disease or tissue damage associated with failure of cellular proliferation of the present invention is administered to human, the dose thereof is determined based on the age and condition of the patient. Generally, in the case of an adult, the dose of the specific compound used in the present invention for oral preparation or intrarectal administration is about 0.1 - 1000 mg/human/day, and about 0.05 mg - 500 mg/human/day for injection. For topical agents, the dose is generally 0.01 mg/kg to 100 mg/kg per administration to an adult, and the number of administration is generally 1 to 6 times per day. These numerical values are mere examples, and the dose is determined according to the symptoms of the patients.

The specific compound used in the present invention that can be used as a prodrug is a derivative of the present invention which has a group that can be chemically or metabolically decomposed and which produces the pharmacologically active compound of the present invention by solvolysis or in vivo under physiological conditions. The method for selecting and producing a suitable prodrug is described, for example, in Design of Prodrugs (Elsevier, Amsterdam 1985). In the present invention, when the compound has a hydroxyl group, an acyloxy derivative obtained by reacting the compound and a suitable acyl halide or suitable acid anhydride is exemplified as a prodrug. Examples of acyloxy particularly preferable as a prodrug include -OCOC₂H₅, -OCO(t-Bu), -OCOC₁₅H₃₁, -OCO(m-CO₂Na-Ph), -OCOCH₂CH₂CO₂Na,-OCOCH(NH₂)CH₃, -OCOCH₂N(CH₃)₂ and the like. When the compound forming the present invention has an amino group, an amide derivative produced by reacting a compound having an amino group with a suitable acid halide or a suitable mixed acid anhydride is exemplified as a prodrug. Examples of amide particularly preferable as a prodrug include -NHCO(CH₂)₂₀OCH₃, - NHCOCH(NH₂)CH₃ and the like.

The diseases to which the therapeutic agent for a disease or tissue damage associated with failure of cellular proliferation of the present invention can be applied not particularly limited as long as they are diseases or tissue damages that can be treated as a result of promotion of cell proliferation, wound therapy, and tissue regeneration by inhibiting the Hippo signal transduction pathway. In one embodiment, the disease associated with failure of cellular proliferation includes a disease associated with abnormal activation of the Hippo signal transduction pathway. In another embodiment, the disease associated with failure of cellular proliferation includes a disease associated with suppression of nuclear translocation of YAP and/or TAZ. Examples of such disease or tissue damage associated with failure of cellular proliferation include, but are not limited to, inflammatory disease (e.g., inflammation, dermatitis, atopic dermatitis, hepatitis, nephritis, glomerulonephritis, pancreatitis, psoriasis, gout, Addison's disease, inflammatory bowel disease (e.g., Crohn's disease), ulcerative colitis, collagenous colitis, lymphocytic colitis, ischemic colitis, diverslon colitis, Behcet's syndrome, infective colitis, and indeterminate colitis and the like), neurodegenerative disease (e.g., Alzheimer's disease, Parkinson's disease, amyotrophic lateral sclerosis (ALS), Creutzfeldt-Jakob disease, Huntington's disease, spinocerebella degeneration (SCD), multiple system atrophy (MSA), spinal muscular atrophy (SMA), spinal and bulbar muscular atrophy and the like), immune-nerve disease (e.g., multiple sclerosis, Guillain-Barre syndrome, myasthenia gravis, polymyositis and the like), muscular dystrophy, myopathy, trauma, burn, chemical burn, skin ulcer, traumatic ulcer, lower leg ulcer, frostbite ulcer, immune-ulcer, postherpetic ulcer, radiation ulcer, pressure ulcer, diabetic skin ulcer, giant pigmented nevus, scar, disorder due to tattoo, vitiligo vulgaris, leukopathia, spinal cord damage, muscle damage, liver failure, drug-induced hepatopathy, alcohol-induced hepatopathy, ischemic hepatopathy, viral hepatopathy, autoimmune hepatopathy, acute hepatitis, chronic hepatitis, cirrhosis, skin damage, brain edema, myocardial infarction, cerebral hemorrhage, and cerebral infarction and the like.

In one embodiment, the therapeutic agent for a disease or tissue damage associated with failure of cellular proliferation of the present invention may be effective in promoting engraftment and recovery of cell, tissue, organ and the like transplanted to a mammal. Examples of the cell to be transplanted include, but are not limited to, stem cells (hematopoietic stem cells, mesenchymal stem cells, neural stem cells, etc.), β-cells and the like. Examples of the tissue to be transplanted include, but are not limited to, blood, erythrocytes, platelets, lymphocytes, bone marrow, cord blood, blood vessel, cornea, retina, eyeball, skin and the like. Examples of the organ to be transplanted include, but are not limited to, pancreas, lung, kidney, liver, heart, small intestine and the like. In this embodiment, the therapeutic agent for a disease or tissue damage associated with failure of cellular proliferation of the present invention can also be paraphrased as "an engraftment promoter in cell, tissue, and/or organ transplantation ".

### 4. Proliferation promoter of cell or tissue for transplantation treatment

The present invention also provides a proliferation promoter of a cell or tissue for a transplantation treatment containing the specific compound used in the present invention (hereinafter sometimes referred to as "the proliferation promoter of the present invention").

In cells in which the Hippo signal transduction pathway is suppressed or inhibited, the nuclear concentration of YAP and/or TAZ is increased, and as a result, cell proliferation is promoted. In addition, in cells in which the Hippo signal transduction pathway is suppressed or inhibited, cell functions relating to damage healing ability and tissue regeneration ability have also enhanced. Therefore, cells in which the Hippo signal transduction pathway is suppressed or inhibited or tissues containing the cells are suitable for transplantation treatment, and the time required for engraftment and/or recovery after transplantation into the target is expected to be shortened compared to cells or tissues free of suppression or inhibition of the Hippo signal transduction pathway.

The amount, sterilization treatment and the like of the specific compound used in the present invention and blended in the proliferation promoter of the present invention are similar to those explained in 1. Kinase inhibitor of the present invention.

By culturing cells in a medium supplemented with the proliferation promoter of the present invention, cells in which the Hippo signal transduction pathway is suppressed or inhibited, or tissues containing the cells can be prepared in vitro. In one embodiment, the cells cultured in the medium supplemented with the proliferation promoter of the present invention can be cells derived from mammals. Examples of the cell include, but are not limited to, somatic cells constituting the living body, normal cell lines, cancer cell lines, progenitor cells, stem cells, cells separated from the living body and applied with artificial genetic modification, cells separated from the living body wherein the nucleus is artificially exchanged and the like. While the derivation of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, rhesus monkey, chimpanzee, human and the like can be mentioned. The tissue or organ from which the cells are derived is not particularly limited. Examples of the tissue include tissues such as skin, kidney, spleen, adrenal gland, liver, lung, ovary, pancreas, uterus, stomach, colon, small intestine, large intestine, bladder, prostate, testis, thymus, muscle, bond tissue, bone, joints, blood vessel tissue, blood, heart, eye, brain, nerve tissue and the like. Examples of the aforementioned organ include organs such as liver, lung, kidney, heart, pancreas, stomach, spleen, small intestine, large intestine, reproductive organ and the like. In one embodiment, stem cells are preferable as the cells to be cultured. For example, if ES cells or iPS cells are cultured in vitro using a medium supplemented with the proliferation promoter of the present invention, the proliferation of the cells is promoted, and a large amount of ES cells or iPS cells can be efficiently prepared. By differentiating the thus obtained ES cells or iPS cells into a target tissue by a method known per se, they can be preferably used for transplantation treatment.

The amount of the proliferation promoter of the present invention to be added to a medium is not particularly limited as long as the Hippo signal transduction pathway is inhibited. For example, the concentration of the specific compound to be used in the present invention in the medium is generally 0.001 - 100 µM, preferably 0.01 M - 50 µM, more preferably 0.1 - 30 µM, further preferably 1 - 20 µM, particularly preferably 5 - 10 µM. The proliferation promoter of the present invention may be added to the medium at any timing during culture and amplification of the cells. From the aspect of the effect of promoting cell proliferation, it is preferable to add the proliferation promoter from the start of culture.

The medium to which the proliferation promoter of the present invention can be added is not particularly limited as long as it is a medium capable of maintaining or proliferating cells, and a commercially available medium can also be used. The commercially available medium that can be used when using the proliferation promoter of the present invention is the same as the commercially available medium that can be used when using the kinase inhibitor of the present invention.

The culture conditions in the medium to which the proliferation promoter of the present invention is added are not particularly limited as long as the cells can be maintained or amplified. The culture temperature is, for example, within the range of 30 - 40°C, preferably 36 - 38°C, more preferably 37°C. From the aspect of more preferably maintaining or amplifying cells, it is preferable to perform medium exchange and passage at a suitable time. The frequency of medium exchange is not particularly limited as long as the cells can be maintained or amplified. For example, it is performed every 1 to 5 days, preferably every 3 days. The frequency of passage is not particularly limited as long as the cells can be maintained or amplified, and can be appropriately performed at the timing when the cell population (preferably organoid) grows, for example, every 4 to 12 days, preferably every 6 to 10 days.

For the culture of cell and/or tissue, culture vessels generally used for cell culture such as schales, flasks, plastic bags, Teflon (registered trade mark) bags, dishes, schales, dishes for tissue culture, multidishes, microplates, microwell plates, multiplates, multiwell plates, chamber slides, tubes, trays, culture bags, roller bottles and the like can be used for cultivation. In one embodiment, as a culture vessel, one having a surface artificially treated to improve adhesiveness to cells (e.g., coating treatment with extracellular matrix and the like) may be used. Examples of such container include, but are not limited to, coated containers such as Matrigel (registered trade mark), Geltrex (registered trade mark), collagen, gelatin, poly-L-lysine, poly-D-lysine, laminin, iMatrix-511, iMatrix-411, iMatrix-221, fibronectin, vitronectin, tenascin, selectin, hyaluronic acid, fibrin and the like. A coating material for inhibiting adhesion of the cells and/or tissues to culture tools can also be used. Examples of the coating material include, but are not limited to, hydrogel, prevelex (registered trade mark), silicon, poly(2-hydroxymethylmethacrylate), poly(2-methacryloyloxyethylphosphoryl choline) and the like. In addition, a culture vessel having a surface artificially treated to reduce adhesiveness to cells may also be used. Examples of such culture container include, but are not limited to, Sumilon cell-tight plate (manufactured by SUMITOMO BAKELITE CO., LTD.), PrimeSurface (registered trade mark) plate (manufactured by SUMITOMO BAKELITE CO., LTD.), Ultra-low Attachment surface plate (manufactured by Corning Incorporated), Nunclon Spheraplate (manufactured by Thermo Fisher Scientific) and the like.

The cells and/or tissues can also be cultured by automatically conducting cell seeding, medium exchange, cell image obtainment, and recovery of cultured cells, under a mechanical control and under a closed environment while controlling pH, temperature, oxygen concentration and the like and using a bioreactor and an automatic incubator capable of high density culture. Examples of the method for supplying a new medium and feeding the required substances to the cells and/or tissues during the culture using such apparatuses include, but are not limited to, fed-batch culture, continuous culture and perfusion culture.

### 5. Screening method

According to the aforementioned Hippo signal transduction pathway inhibitor of the present invention, the Hippo signal transduction pathway of the desired cell can be inhibited. Cells with an inhibited Hippo signal transduction pathway mimic the cellular environment of disease associated with promoted nuclear translocation of YAP and/or TAZ. Therefore, by using such cells, candidate substances that can be applied to the treatment or prevention of the disease can be screened. That is, the present invention provides a method for screening for a substance for treating and/or preventing a disease associated with promoted nuclear translocation of YAP and/or TAZ, (hereinafter sometimes referred to as "the screening method of the present invention").

In the screening method of the present invention, first, cells are cultured in a medium supplemented with the specific compound used in the present invention (or the Hippo signal transduction pathway inhibitor of the present invention) (step 1). The cells, medium, amount of the specific compound added to the medium, and the like that can be used in step 1 are the same as those explained for the kinase inhibitor of the present invention.

Next, the test substance is brought into contact with the cells obtained in step 1. The test substance can be contacted by adding the test substance to the cell culture medium. After adding the test substance to the culture medium, the cells are generally maintained and cultured for 1 - 5 days (preferably 2 - 4 days). During the maintenance culture period, the Hippo signal transduction pathway inhibitor of the present invention is preferably added to the medium for maintenance culture so that the inhibition of the Hippo signal transduction pathway in cells can be maintained. After lapse of a given period, the abundance of YAP and/or TAZ in the nucleus of the cell contacted with the test substance is measured (step 2). When the abundance of YAP and/or TAZ in the nucleus is reduced compared to the abundance in the absence of the test substance, the test substance can be determined as an agent for the treatment and/or prophylaxis of a disease associated with promoted nuclear translocation of YAP and/or TAZ (step 3). That the abundance of YAP and/or TAZ decreases means that, for example, the abundance of YAP and/or TAZ in the nucleus decreases by at least 5% or more, at least 10% or more, at least 15% or more, at least 20% or more, at least 25% or more, at least 30% or more, at least 35% or more, at least 40% or more, at least 50% or more, at least 55% or more, at least 60% or more, at least 65% or more, at least 70% or more, at least 75% or more, at least 80% or more, at least 85% or more, at least 90% or more, at least 95% or more, or at least 99% or more, as compared to the abundance of YAP and/or TAZ to be the control in the nucleus. The abundance of YAP and/or TAZ in the cell nucleus can be measured by a method known per se such as imaging analysis using an anti-YAP (or TAZ) antibody to which a fluorescent label is bound, as used in Examples described later.

In the screening method of the present invention, a disease associated with promoted nuclear translocation of YAP and/or TAZ includes cancer. Specific examples include, but are not limited to, colorectal cancer, pancreatic cancer, lung cancer, ovarian cancer, liver cancer, breast cancer, kidney cancer, prostate cancer, digestive organ cancer, melanoma, cervix cancer, bladder cancer, glioblastoma, cervical cancer, and the like.

### 6. Method for inhibiting kinase

The present invention provides a method for inhibiting a specific kinase activity (hereinafter sometimes referred to as "a method for inhibiting the kinase of the present invention"), which includes contacting the specific compound used in the present invention with a cell. The method for inhibiting the kinase of the present invention can be preferably performed using the above-mentioned kinase inhibitor of the present invention. Thus, the embodiment thereof can also be applied to the present method. In one embodiment, the method for inhibiting the kinase of the present invention includes inhibiting the phosphorylation activity of LATS2 in cells.

### 7. Method for inhibiting Hippo signal transduction pathway

The present invention provides a method for inhibiting the Hippo signal transduction pathway, which includes contacting the specific compound used in the present invention with a cell (hereinafter sometimes referred to as "a method for inhibiting the Hippo signal transduction pathway of the present invention"). The method for inhibiting the Hippo signal transduction pathway of the present invention can be preferably performed using the above-mentioned Hippo signal transduction pathway inhibitor of the present invention. Thus, the embodiment thereof can also be applied to the present method.

### 8. Method for treating disease or tissue damage associated with failure of cellular proliferation

The present invention provides a method for treating a disease or tissue damage associated with failure of cellular proliferation, which includes administering the specific compound used in the present invention to the target (hereinafter sometimes referred to as "the treatment method of the present invention"). The treatment method of the present invention can be preferably performed using the above-mentioned therapeutic agent for a disease or tissue damage associated with failure of cellular proliferation of the present invention. Therefore, the embodiment thereof can also be applied to the present method. When the treatment method of the present invention is performed for the purpose of promoting engraftment and recovery particularly when cell, tissue, organ, or the like is transplanted into a mammal, the treatment method of the present invention can also be paraphrased as "a method for promoting engraftment in cell, tissue, and/or organ transplantation".

### 9. Method for promoting proliferation of cell or tissue for transplantation treatment

The present invention provides a method for promoting proliferation of a cell or tissue for a transplantation treatment, which includes administering the specific compound used in the present invention to a cell (hereinafter sometimes referred to as "a proliferation promoting method of the present invention"). The proliferation promoting method of the present invention can be preferably performed using the above-mentioned proliferation promoter of the present invention. Therefore, the embodiment thereof can also be applied to the present method. Since a cell or tissue for a transplantation treatment can be efficiently prepared by the proliferation promoting method of the present invention, the proliferation promoting method of the present invention can also be paraphrased as "a method for producing a cell or tissue for a transplantation treatment".

### 10. Medium

The present invention also provides a cell culture medium containing the kinase inhibitor of the present invention, the Hippo signal transduction pathway inhibitor of the present invention, or the proliferation promoter of a cell or tissue for a transplantation treatment of the present invention (hereinafter sometimes referred to as "the medium of the present invention"). The medium of the present invention is a medium prepared adding the kinase inhibitor of the present invention, the Hippo signal transduction pathway inhibitor of the present invention, and the proliferation promoter of a cell or tissue for a transplantation treatment of the present invention to a medium suitable for cell culture, as described for these agents. By culturing cells in the medium of the present invention, the effects exactly as described for the kinase inhibitor of the present invention, the Hippo signal transduction pathway inhibitor of the present invention, and the proliferation promoter of a cell or tissue for a transplantation treatment of the present invention can be obtained.

While the usefulness of the present invention is specifically described below in the following Examples, the present invention is not limited to them alone. The concentration (%) of CO₂ in the CO₂ incubator is shown by % by volume of CO₂ in the atmosphere. PBS means phosphate buffered saline (manufactured by Sigma-Aldrich Japan), and FBS means fetal bovine serum (manufactured by Biological Industries). In addition, (w/v) shows weight per volume.

### [Example]

The synthesis methods and structural formulas of the specific compounds to be used in the present invention are shown below. ¹H-NMR shows proton nuclear magnetic resonance spectrum which was measured at 270 MHz or 400 MHz in deuterodimethyl sulfoxide. As the chemical shift value, the value of deuterodimethyl sulfoxide is shown as 2.49 ppm. In addition, s shows singlet, and similarly, brs shows broad singlet, d shows doublet, dd shows double doublet, t shows triplet, q shows quartet, and m shows multiplet.

### [Synthetic Example 1] Synthesis of the compound of the present invention using ketone and hydrazide as starting materials (Materials and methods)

Compounds were synthesized from 4 kinds of ketones [1-(2,4-dihydroxy-3-methylphenyl)propan-1-one (hereinafter abbreviated as k-1), 1-(2,4-dihydroxy-3-methylphenyl)ethan-1-one (hereinafter abbreviated as k-2), 1-(2,4,6-trihydroxy-3-methylphenyl)propan-1-one (hereinafter abbreviated as k-3), 1-(2,4-dihydroxy-3-methylphenyl)-3-methylbutan-1-one (hereinafter abbreviated as k-5)] and 10 kinds of hydrazides [2-(phenylamino)acetohydrazide (hereinafter abbreviated as H-1), 2-(o-tolylamino)acetohydrazide (hereinafter abbreviated as H-2), 2-(m-tolylamino)acetohydrazide (hereinafter abbreviated as H-3), 2-(p-tolylamino)acetohydrazide (hereinafter abbreviated as H-4), 2-[(4-fluorophenyl)amino]acetohydrazide (hereinafter abbreviated as H-5), 2-(naphthalen-1-ylamino)acetohydrazide (hereinafter abbreviated as H-6), 2-(phenylamino)butanehydrazide (hereinafter abbreviated as H-7), 2-[(4-ethoxyphenyl)amino]acetohydrazide (hereinafter abbreviated as H-9), 2-[(4-hydroxyphenyl)amino]acetohydrazide (hereinafter abbreviated as D-2), 2-[(2-hydroxyphenyl)amino]acetohydrazide (hereinafter abbreviated as D-4)] shown in the following.

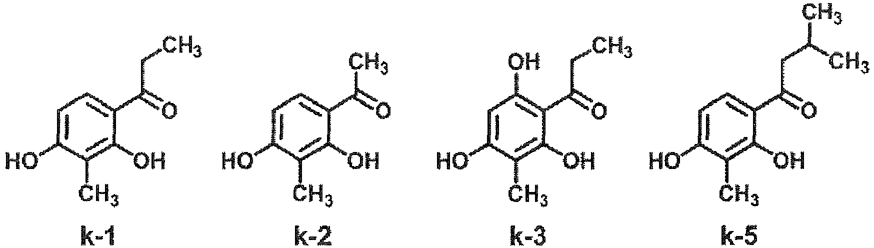

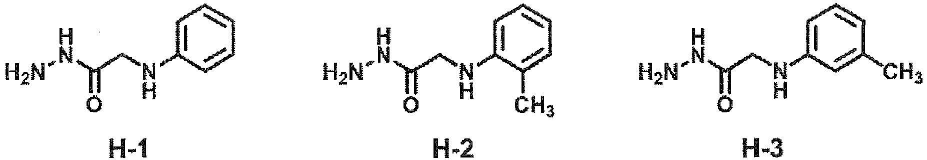

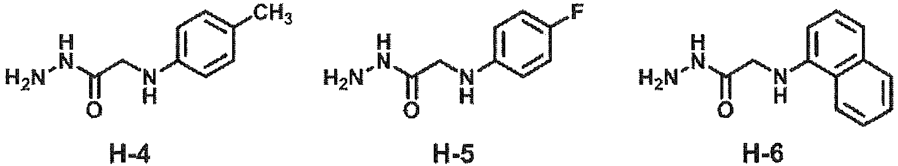

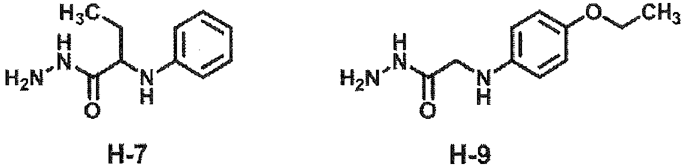

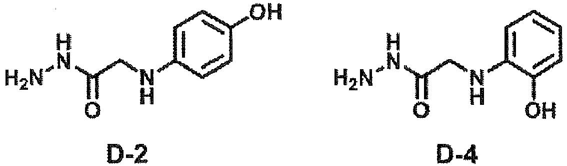

The synthesized 34 compounds are described in the first table. In the table, Me is methyl, and similarly, Et is ethyl, n-Pr is normal propyl, i-Bu is isobutyl, Ph is phenyl, and Naph is naphthyl. In (R₃)ₙ, "-" means unsubstituted, and the numbers indicated in the structural formulas show the substitutable position of (R₃)ₙ.

### [the first table]

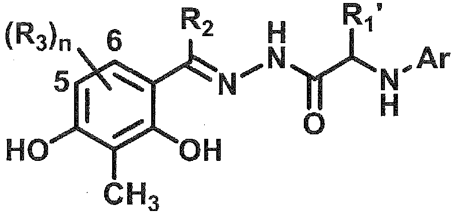

**[Table 1]**

| compound No. | R₂ | (R₃)ₙ | R₁' | Ar |
|---|---|---|---|---|
| k-1:H-1 | Et | - | H | Ph |
| k-1:H-2 | Et | - | H | 2-Me-Ph |
| k-1:H-3 | Et | - | H | 3-Me-Ph |
| k-1:H-4 | Et | - | H | 4-Me-Ph |
| k-1:H-5 | Et | - | H | 4-F-Ph |
| k-1:H-6 | Et | - | H | 1-Naph |
| k-1:H-7 | Et | - | Et | Ph |
| k-1:H-9 | Et | - | H | 4-EtO-Ph |
| k-1:D-2 | Et | - | H | 4-OH-Ph |
| k-1:D-4 | Et | - | H | 2-OH-Ph |
| k-2:H-1 | Me | - | H | Ph |
| k-2:H-2 | Me | - | H | 2-Me-Ph |
| k-2:H-3 | Me | - | H | 3-Me-Ph |
| k-2:H-4 | Me | - | H | 4-Me-Ph |
| k-2:H-5 | Me | - | H | 4-F-Ph |
| k-2:H-6 | Me | - | H | 1-Naph |
| k-2:H-7 | Me | - | Et | Ph |
| k-2:H-9 | Me | - | H | 4-EtO-Ph |
| k-3:H-1 | Et | 6-OH | H | Ph |
| k-3:H-2 | Et | 6-OH | H | 2-Me-Ph |
| k-3:H-3 | Et | 6-OH | H | 3-Me-Ph |
| k-3:H-4 | Et | 6-OH | H | 4-Me-Ph |
| k-3:H-5 | Et | 6-OH | H | 4-F-Ph |
| k-3:H-6 | Et | 6-OH | H | 1-Naph |
| k-3:H-7 | Et | 6-OH | Et | Ph |
| k-3:H-9 | Et | 6-OH | H | 4-EtO-Ph |
| k-5:H-1 | i-Bu | - | H | Ph |
| k-5:H-2 | i-Bu | - | H | 2-Me-Ph |
| k-5:H-3 | i-Bu | - | H | 3-Me-Ph |
| k-5:H-4 | i-Bu | - | H | 4-Me-Ph |
| k-5:H-5 | i-Bu | - | H | 4-F-Ph |
| k-5:H-6 | i-Bu | - | H | 1-Naph |
| k-5:H-7 | i-Bu | - | Et | Ph |
| k-5:H-9 | i-Bu | - | H | 4-EtO-Ph |

The measurement results of ¹H-NMR of the compounds described in the first table are shown in the second table.

### [the second table]

**[Table 2-1]**

| compound No. | ¹H-NMR (270 MHz) |
|---|---|
| k-1:H-1 | δ13.98(s, 1H), 11.13(s, 1H), 10.01(s, 1H), 7.58(d, J=8.1Hz, 1H), 7.41(t, J=8.1Hz, 2H), 6.95(d, J=8.1Hz, 2H), 6.89(t, J=8.1Hz, 1H), 6.72(d, J=8.1Hz, 1H), 6.29(t, J=8.1Hz, NH), 4.27(d, J=8.1Hz, 2H), 3.11(q, J=8.1Hz, 2H), 2.29(s, 3H), 1.37(t, J=8.1Hz, 3H). |
| k-1:H-2 | δ13.63 (s, 1H), 10.80(s, 1H), 9.67(s, 1H), 7.24(d, J=8.1Hz, 1H), 7.00(t, J=8.1Hz, 1H), 6.99(d, J=8.1Hz, 1H), 6.54(t, J=8.1Hz, 1H), 6.46(d, J=8.1Hz, 1H), 6.38(d, J=8.1Hz, 1H), 5.27(t, J=8.1Hz, NH), 3.98(d, J=5.4Hz, 2H), 2.76(q, J=5.4Hz, 2H), 2.14(s, 3H), 1.95(s, 3H), 1.02(t, J=8.1Hz, 3H). |
| k-1:H-3 | δ13.64 (s, 1H), 10.77 (brs, 1H), 9.68 (brs, 1H), 7.24(d, J=8.1Hz, 1H), 6.96(t, J=8.1Hz, 1H), 6.50-6.30(m, 4H), 5.86(t, J=5.4Hz, NH), 3.91(d, J=5.4Hz, 2H), 2.78(q, J=8.1Hz, 2H), 2.17(s, 3H), 1.95(s, 3H), 1.04(t, J=8.1Hz, 3H). |
| k-1:H-4 | δ13.65(s, 1H), 10.76(brs, 1H), 9.68(brs, 1H), 7.25(d, J=8.1Hz, 1H), 6.91(d, J=8.1Hz, 2H), 6.54(d, J=8.1Hz, 2H), 6.39(d, J=8.1Hz, 1H), 5.76(t, J=5.4Hz, NH), 3.90(d, J=5.4Hz, 2H), 2.78(q, J=8.1Hz, 2H), 2.14(s, 3H), 1.96(s, 3H), 1.04(t, J=8.1Hz, 3H). |
| k-1:H-5 | δ13.64(s, 1H), 10.80(brs, 1H), 9.67(brs, 1H), 7.24(d, J=10.8Hz, 1H), 6.93(m, 2H), 6.60(m, 2H), 6.38(d, J=10.8Hz, 1H), 5.93(t, J=8.1Hz, NH), 3.91(d, J=5.4Hz, 2H), 2.78(q, J=8.1Hz, 2H), 1.95(s, 3H), 1.04(t, J=8.1Hz, 3H). |
| k-1:H-6 | δ13.65(s, 1H), 10.91(brs, 1H), 9.67(brs, 1H), 8.16(d, J=8.1Hz, 1H), 7.77(d, J=8.1Hz, 1H), 7.50-7.20(m, 4H), 7.15(d, J=8.1Hz, 1H), 6.61(t, J=8.1Hz, NH), 6.45(d, J=5.4Hz, 1H), 6.38(d, J=10.8Hz, 1H), 4.14(d, J=5.4Hz, 2H), 2.78(q, J=8.1Hz, 2H), 1.95(s, 3H), 1.02(t, J=8.1Hz, 3H). |
| k-1:H-7 | δ13.64(s, 1H), 10.79(s, 1H), 9.67(s, 1H), 7.24(d, J=8.1Hz, 1H), 7.05(t, J=8.1Hz, 2H), 6.66(d, J=8.1Hz, 2H), 6.53(t, J=8.1Hz, 1H), 6.38(d, J=8.1Hz, 1H), 5.83(d, J=8.1Hz, NH), 4.13(m, J=8.1Hz, 1H), 2.80(q, J=8.1Hz, 2H), 1.94(s, 3H), 1.76(m, 2H), 1.02(t, J=8.1Hz, 3H), 0.98(t, J=8.1Hz, 3H). |
| k-1:H-9 | δ13.64(s, 1H), 10.73(s, 1H), 9.67(brs, 1H), 7.23(d, J=8.1Hz, 1H), 6.72(d, J=8.1Hz, 2H), 6.56(d, J=8.1Hz, 2H), 6.38(d, J=8.1Hz, 1H), 5.58(t, J=8.1Hz, NH), 3.87(q, J=8.1Hz, 2H), 3.86(d, J=8.1Hz, 2H), 2.76(q, J=8.1Hz, 2H), 1.95(s, 3H), 1.24(t, J=8.1Hz, 3H), 1.02(t, J=8.1Hz, 3H). |

**[Table 2-2]**

| compound No. | ¹H-NMR (270 MHz) |
|---|---|
| k-1:D-2 | δ13.65(s, 1H), 10.71(brs, 1H), 9.69(brs, 1H), 8.48(s, 1H), 7.25(d, J=8.1Hz, 1H), 6.57(d, J=8.1Hz, 2H), 6.49(d, J=8.1Hz, 2H), 6.39(d, J=10.8Hz, 1H), 5.40(t, J=8.1Hz, NH), 3.84(d, J=5.4Hz, 2H), 2.76(q, J=8.1Hz, 2H), 1.96(s, 3H), 1.02(t, J=8.1Hz, 3H). |
| k-1:D-4 | δ13.65(s, 1H), 10.88(brs, 1H), 9.70(s, 1H), 9.37(s, 1H), 7.26(d, J=8.1Hz, 1H), 6.69(d, J=8.1Hz, 1H), 6.65(t, J=8.1Hz, 1H), 6.50-6.35(m, 3H), 5.15(t, J=8.1Hz, NH), 3.95(d, J=5.4Hz, 2H), 2.78(q, J=8.1Hz, 2H), 1.97(s, 3H), 1.03(t, J=8.1Hz, 3H). |
| k-2:H-1 | 13.59(s, 1H), 10.82(brs, 1H), 9.67(brs, 1H), 7.25(d, J=8.1Hz, 1H), 7.08(t, J=8.1Hz, 2H), 6.62(d, J=8.1Hz, 2H), 6.56(t, J=8.1Hz, 1H), 6.38(d, J=8.1Hz, 1H), 5.93(t, J=8.1Hz, NH), 3.91(d, J=5.4Hz, 2H), 2.28(s, 3H), 1.96(s, 3H). |
| k-2:H-2 | δ13.58(s, 1H), 10.85(brs, 1H), 9.67(brs, 1H), 7.25(d, J=8.1Hz, 1H), 7.10-6.90 (m, 2H), 6.54(t, J=8.1Hz, 1H), 6.45(d, J=8.1Hz, 1H), 6.38(d, J=8.1Hz, 1H), 5.25(t, J=8.1Hz, NH), 3.97(d, J=8.1Hz, 2H), 2.27(s, 3H), 2.14(s, 3H), 1.96(s, 3H). |
| k-2:H-3 | δ13.59(s, 1H), 10.78(brs, 1H), 9.67(brs, 1H), 7.25(d, J=8.1Hz, 1H), 6.96(t, J=8.1Hz, 1H), 6.45-6.30(m, 4H), 5.82(t, J=8.1Hz, NH), 3.89(d, J=5.4Hz, 2H), 2.27(s, 3H), 2.18(s, 3H), 1.96(s, 3H). |
| k-2:H-4 | δ13.59(s, 1H), 10.78(brs, 1H), 9.67(brs, 1H), 7.25(d, J=8.1Hz, 1H), 6.90(d, J=8.1Hz, 2H), 6.52(d, J=8.1Hz, 2H), 6.38(d, J=8.1Hz, 1H), 5.72(t, J=8.1Hz, NH), 3.87(d, J=5.4Hz, 2H), 2.27(s, 3H), 2.14(s, 3H), 1.96(s, 3H). |
| k-2:H-5 | δ13.58(s, 1H), 10.80(brs, 1H), 9.67(brs, 1H), 7.25(d, J=8.1Hz, 1H), 6.95-6.80(m, 2H), 6.65-6.50(m, 2H), 6.38(d, J=8.1Hz, 1H), 5.90(t, J=8.1Hz, NH), 3.89(d, J=5.4Hz, 2H), 2.28(s, 3H), 1.96(s, 3H). |
| k-2:H-6 | δ13.60(s, 1H), 10.95(brs, 1H), 9.67(brs, 1H), 8.16(d, J=8.1Hz, 1H), 7.77(d, J=8.1Hz, 1H), 7.50-7.35(m, 2H), 7.35-7.20(m, 2H), 7.15(d, J=8.1Hz, 1H), 6.61(t, J=8.1Hz, NH), 6.44(d, J=8.1Hz, 1H), 6.38(d, J=8.1Hz, 1H), 4.12(d, J=5.4Hz, 2H), 2.30(s, 3H), 1.95(s, 3H). |
| k-2:H-7 | δ13.59(s, 1H), 10.83(brs, 1H), 9.67(brs, 1H), 7.24(d, J=8.1Hz, 1H), 7.06(t, J=8.1Hz, 2H), 6.65(d, J=8.1Hz, 2H), 6.53(t, J=8.1Hz, 1H), 6.37(d, J=8.1Hz, 1H), 5.80(d, J=8.1Hz, NH), 4.05(m, 1H), 2.28(s, 3H), 1.95(s, 3H), 1.75(m, 2H), 0.98(d, J=8.1Hz, 3H). |
| k-2:H-9 | δ13.59(s, 1H), 10.75(brs, 1H), 9.67(brs, 1H), 7.25(d, J=8.1Hz, 1H), 6.72(d, J=8.1Hz, 2H), 6.56(d, J=8.1Hz, 2H), 6.37(d, J=8.1Hz, 1H), 5.54(t, J=8.1Hz, NH), 3.87(q, J=8.1Hz, 2H), 3.85(d, J=8.1Hz, 2H), 2.26(s, 3H), 1.96(s, 3H), 1.25(t, J=8.1Hz, 3H). |

**[Table 2-3]**

| compound No. | ¹H-NMR (270 MHz) |
|---|---|
| k-3:H-1 | δ13.07(s, 1H), 10.62(s, 1H), 9.67(s, 1H), 9.45(s, 1H), 7.09(t, J=8.1Hz, 2H), 6.62(d, J=8.1Hz, 2H), 6.58(t, J=8.1Hz, 1H), 6.01(t, J=8.1Hz, NH), 5.95(s, 1H), 3.92(d, J=5.4Hz, 2H), 2.88(q, J=8.1Hz, 2H), 1.84(s, 3H), 1.00(t, J=8.1Hz, 3H). |
| k-3:H-2 | δ13.06(s, 1H), 10.62(s, 1H), 9.67(s, 1H), 9.45(s, 1H), 7.01(t, J=8.1Hz, 1H), 7.00(d, J=8.1Hz, 1H), 6.55(t, J=8.1Hz, 1H), 6.47(d, J=8.1Hz, 1H), 5.95(s, 1H), 5.33(t, J=8.1Hz, NH), 3.97(d, J=5.4Hz, 2H), 2.87(q, J=8.1Hz, 2H), 2.15(s, 3H), 1.84(s, 3H), 0.99(t, J=8.1Hz, 3H). |
| k-3:H-3 | δ13.07(s, 1H), 10.60(s, 1H), 9.67(s, 1H), 9.45(s, 1H), 6.97(t, J=8.1Hz, 1H), 6.45-6.30(m, 3H), 5.95(s, 1H), 5.89(t, J=8.1Hz, NH), 3.90(d, J=5.4Hz, 2H), 2.88(q, J=8.1Hz, 2H), 2.18(s, 3H), 1.84(s, 3H), 1.01(t, J=8.1Hz, 3H). |
| k-3:H-4 | δ13.07(s, 1H), 10.58(s, 1H), 9.67(s, 1H), 9.45(s, 1H), 6.92(d, J=8.1Hz, 2H), 6.54(d, J=8.1Hz, 2H), 5.95(s, 1H), 5.79(t, J=5.4Hz, NH), 3.88(d, J=5.4Hz, 2H), 2.87(q, J=8.1Hz, 2H), 2.15(s, 3H), 1.84(s, 3H), 1.00(t, J=8.1Hz, 3H). |
| k-3:H-5 | δ13.07(s, 1H), 10.63(s, 1H), 9.67(s, 1H), 9.45(s, 1H), 7.00-6.80(m, 2H), 6.70-6.65(m, 2H), 5.96(t, J=5.4Hz, NH), 5.95(s, 1H), 3.90(d, J=5.4Hz, 2H), 2.89(q, J=8.1Hz, 2H), 1.85(s, 3H), 1.01(t, J=8.1Hz, 3H). |
| k-3:H-6 | δ13.04(s, 1H), 10.71(s, 1H), 9.66(s, 1H), 9.43(s, 1H), 8.16(d, J=8.1Hz, 1H), 7.78(d, J=8.1Hz, 1H), 7.50-7.35(m, 2H), 7.27(t, J=8.1Hz, 1H), 7.14(t, J=8.1Hz, 1H), 6.62(t, J=5.4Hz, NH), 6.46(d, J=8.1Hz, 1H), 5.94(s, 1H), 4.12(d, J=5.4Hz, 2H), 2.87(q, J=8.1Hz, 2H), 1.83(s, 3H), 0.96(t, J=8.1Hz, 3H). |
| k-3:H-7 | δ13.09(s, 1H), 10.66(s, 1H), 9.67(s, 1H), 9.45(s, 1H), 7.06(t, J=10.8Hz, 2H), 6.67(d, J=8.1Hz, 2H), 6.55(t, J=8.1Hz, 1H), 5.95(s, 1H), 5.83(d, J=10.8Hz, NH), 4.07(m, 1H), 2.92(q, J=8.1Hz, 2H), 1.83(s, 3H), 1.77(m, 2H), 0.99(t, J=8.1Hz, 3H). |
| k-3:H-9 | δ13.08(s, 1H), 10.57(s, 1H), 9.67(s, 1H), 9.45(s, 1H), 6.75(d, J=10.8Hz, 2H), 6.57(d, J=8.1Hz, 2H), 5.95(s, 1H), 5.63(t, J=5.4Hz, NH), 3.88(q, J=8.1Hz, 2H), 3.86(d, J=8.1Hz, 2H), 2.86(q, J=8.1Hz, 2H), 1.84(s, 3H), 1.26(t, J=8.1Hz, 3H), 0.99(t, J=8.1Hz, 3H). |

**[Table 2-4]**

| compound No. | ¹H-NMR (270 MHz) |
|---|---|
| k-5:H-1 | δ13.71(s, 1H), 10.67(brs, 1H), 9.66(brs, 1H), 7.23(d, J=8.1Hz, 1H), 7.07(t, J=8.1Hz, 2H), 6.60(d, J=8.1Hz, 2H), 6.57(t, J=8.1Hz, 1H), 6.36(d, J=8.1Hz, 1H), 6.01(t, J=5.4Hz, NH), 3.91(d, J=5.4Hz, 2H), 2.69(d, J=5.4Hz, 2H), 1.95(s, 3H), 1.82(m.1H), 0.83(d, J=5.4Hz, 6H). |
| k-5:H-2 | δ13.71(s, 1H), 10.66(brs, 1H), 9.66(brs, 1H), 7.23(d, J=8.1Hz, 1H), 7.01(d, J=8.1Hz, 1H), 6.99(t, J=8.1Hz, 1H), 6.55(t, J=8.1Hz, 1H), 6.44(d, J=8.1Hz, 1H), 6.37(d, J=8.1Hz, 1H), 5.37(t, J=8.1Hz, NH), 3.97(d, J=5.4Hz, 2H), 2.68(d, J=5.4Hz, 2H), 2.16(s, 3H), 1.96(s, 3H), 1.79(m.1H), 0.81(d, J=5.4Hz, 6H). |
| k-5:H-3 | δ13.71(s, 1H), 10.66(brs, 1H), 9.65(brs, 1H), 7.23(d, J=8.1Hz, 1H), 6.95(t, J=8.1Hz, 1H), 6.45-6.30(m, 4H), 5.92(t, J=8.1Hz, NH), 3.90(d, J=8.1Hz, 2H), 2.69(d, J=5.4Hz, 2H), 2.16(s, 3H), 1.95(s, 3H), 1.82(m, 1H), 0.83(d, J=5.4Hz, 6H). |
| k-5:H-4 | δ13.71(s, 1H), 10.65(brs, 1H), 9.66(brs, 1H), 7.24(d, J=8.1Hz, 1H), 6.91(d, J=8.1Hz, 2H), 6.53(d, J=8.1Hz, 2H), 6.37(d, J=8.1Hz, 1H), 5.82(t, J=8.1Hz, NH), 3.89(d, J=8.1Hz, 2H), 2.69(d, J=5.4Hz, 2H), 2.14(s, 3H), 1.96(s, 3H), 1.82(m, 1H), 0.84(d, J=5.4Hz, 6H). |
| k-5:H-5 | δ13.71(s, 1H), 10.69(brs, 1H), 9.66(brs, 1H), 7.23(d, J=8.1Hz, 1H), 7.00-6.85(m, 2H), 6.65-6.55(m, 2H), 6.37(d, J=8.1Hz, 1H), 5.98(t, J=8.1Hz, NH), 3.89(d, J=8.1Hz, 2H), 2.70(d, J=8.1Hz, 2H), 1.95(s, 3H), 1.82(m, 1H), 0.84(d, J=5.4Hz, 6H). |
| k-5:H-6 | δ13.71(s, 1H), 10.75(brs, 1H), 9.65(brs, 1H), 8.17(d, J=8.1Hz, 1H), 7.78(d, J=8.1Hz, 1H), 7.50-7.40(m, 2H), 7.30-7.10(m, 3H), 6.69(t, J=8.1Hz, NH), 6.43(d, J=5.4Hz, 1H), 6.36(d, J=8.1Hz, 1H), 4.13(d, J=8.1Hz, 2H), 2.66(d, J=8.1Hz, 2H), 1.95(s, 3H), 1.76(m, 1H), 0.74(d, J=8.1Hz, 6H). |
| k-5:H-7 | δ 13.72(s, 1H), 10.75(brs, 1H), 9.66(brs, 1H), 7.23(d, J=8.1Hz, 1H), 7.05(t, J=8.1Hz, 2H), 6.65(d, J=8.1Hz, 2H), 6.55(t, J=8.1Hz, 1H), 6.37(d, J=8.1Hz, 1H), 5.87(d, J=8.1Hz, NH), 4.09(m, 1H), 2.75(d, J=5.4Hz, 2H), 1.95(s, 3H), 1.90-1.70 (m, 3H), 0.99(t, J=8.1Hz, 3H), 0.89(d, J=5.4Hz, 3H), 0.83(d, J=5.4Hz, 3H). |
| k-5:H-9 | δ13.71(s, 1H), 10.62(brs, 1H), 9.66(brs, 1H), 7.22(d, J=8.1Hz, 1H), 6.71(d, J=8.1Hz, 2H), 6.55(d, J=8.1Hz, 2H), 6.36(d, J=8.1Hz, 1H), 5.65(t, J=8.1Hz, NH), 3.88(q, J=8.1Hz, 2H), 3.85(d, J=8.1Hz, 2H), 2.67(d, J=8.1Hz, 2H), 1.95(s, 3H), 1.82(m, 1H), 1.24(t, J=8.1Hz, 3H), 0.82(d, J=5.4Hz, 6H). |

The synthesis methods of the specific compounds to be used in the present invention and synthesis intermediate compounds are described below.

In the 4 kinds of ketones, k-1, k-2 and k-5 can be synthesized by a known method (Sum TH et al., Tetrahedron. 2015 Jul 1; 71(26-27): 4557-4564.). In the 10 kinds of hydrazides, H-1, H-2, H-3, H-4, H-5, H-6, H-9, D-2 and D-4 can be synthesized by a known method (Samal RP et al., Chem Biol Drug Des. 2013 Jun; 81(6):715-29. etc.). Therefore, the synthesis methods of k-3 and H-7 are described in detail below.

### [Synthesis of k-3]

2,4,6-Trihydroxybenzaldehyde (2.22 g, 14.4 mmol) was dissolved in THF (40 mL), NaBH₃CN (2.7 g, 43 mmol) and acetic acid (8 mL) were added under ice-cooling and the mixture was stirred at room temperature for 2 hr. The reaction solution was diluted with ethyl acetate (50 mL), and washed successively with water (50 mLx2), saturated aqueous sodium hydrogen carbonate solution (50 mL), and brine (50 mL). The mixture was dried over anhydrous sodium sulfate, filtered, and concentrated under reduced pressure and the obtained residue was purified by moderate-pressure silica gel column chromatography (silica gel 50 g, ethyl acetate/hexane=20/80 - 50/50) to give an intermediate compound (1.20 g, 8.56 mmol, yield 59%) as a white solid.

The intermediate compound (1.04 g, 7.42 mmol) obtained as mentioned above was suspended in propionic acid (7 mL), propionic acid anhydride (1.15 mL, 8.90 mmol) and BF₃-Et₂O (1.12 mL, 8.90 mmol) were added and the mixture was heated under reflux at 130°C for 1 hr. The mixture was allowed to cool, and the reaction solution was diluted with ethyl acetate (100 mL), and washed successively with water (100 mLx3), saturated aqueous sodium hydrogen carbonate solution (100 mLx2), and brine (100 mL). The mixture was dried over anhydrous sodium sulfate, filtered, and concentrated under reduced pressure and the obtained residue was purified by moderate-pressure silica gel column chromatography (silica gel 100 g, ethyl acetate/hexane=10/90 - 45/55). The obtained solid was washed with hexane to give k-3 (0.41 g) as a light orange solid. The filtrate was concentrated under reduced pressure and the obtained residue was purified again by moderate-pressure silica gel column chromatography (silica gel 30 g, ethyl acetate/hexane=10/90 - 50/50) to give k-3 (0.70 g) as a light orange solid. In total, k-3 (1.11 g, 5.66 mmol, yield 76%) was obtained as a light orange solid.

### [Synthesis of H-7]

Methyl 2-bromobutyrate (8.0 g, 44 mmol) and aniline (8.0 mL, 88 mmol) were dissolved in toluene (10 mL), and the mixture was heated under reflux for 5 hr. The mixture was allowed to cool, and the reaction solution was washed successively with water (30 mL), 2 M hydrochloric acid (25 mL), water (30 mL), saturated aqueous sodium hydrogen carbonate solution (30 mL), and brine (30 mL), and dried over anhydrous sodium sulfate. The mixture was filtered, and concentrated under reduced pressure and the obtained residue was purified by moderate-pressure silica gel column chromatography (silica gel 100 g, ethyl acetate/hexane=1/99 - 10/90) to give an intermediate compound (5.11 g, 26.4 mmol, yield 60%) as a yellow liquid.

The intermediate compound (5.11 g, 26.4 mmol) obtained as mentioned above was dissolved in methanol (26 mL), hydrazine monohydrate (12.8 mL, 264 mmol) was added and the mixture was stirred at room temperature for 4.5 hr. Water (150 mL) was added and the mixture was extracted with methylene chloride (30 mLx5). The organic layer was washed with saturated brine (100 mL), dried over anhydrous magnesium sulfate, filtered, and concentrated under reduced pressure. The obtained solid was washed with IPE to give H-7 (4.60 g, 23.8 mmol, yield 90%) as a white solid.

### [Synthesis of k-1:H-1]

k-1 (100 mg, 0.555 mmol), H-1 (110 mg, 0.666 mmol) were dissolved in DMSO (1.1 mL), and the mixture was stirred at 100°C for 14 hr. The mixture was allowed to cool, distilled water (11 mL) was added, and the mixture was stirred again at 100°C and filtered while hot to give k-1:H-1 (70.1 mg, 0.214 mmol, yield 39%) as a light yellow solid.

### [Synthesis of k-1:H-2]

k-1 (50 mg, 0.28 mmol), H-2 (69 mg, 0.33 mmol) were dissolved in DMSO (0.55 mL), and the mixture was stirred at 100°C for 18 hr. The mixture was allowed to cool, distilled water (6 mL) was added, decantated, and the remaining solid was washed successively with methylene chloride, and ethyl acetate. The obtained residue was dissolved in DMSO (0.2 mL), water was added and the precipitated solid was washed with methanol to give k-1:H-2 (19.6 mg, 0.0574 mmol, yield 21%) as a light orange solid.

### [Synthesis of k-1:H-3]

k-1 (100 mg, 0.555 mmol), H-3 (119 mg, 0.666 mmol) were dissolved in DMSO (1.1 mL), and the mixture was stirred at 100°C for 14 hr. Distilled water (11 mL) was added at the same temperature and the mixture was allowed to cool. The precipitated solid was collected by filtration, and washed with methanol to give k-1:H-3 (98.9 mg, 0.290 mmol, yield 52%) as a white solid.

### [Synthesis of k-1:H-4]

k-1 (50 mg, 0.28 mmol), H-4 (65 mg, 0.36 mmol) were dissolved in DMSO (0.55 mL), and the mixture was stirred at 100°C for 19 hr. The mixture was allowed to cool, distilled water (6 mL) was added and the precipitated solid was collected by filtration, and washed with ethyl acetate to give k-1:H-4 (28.6 mg, 0.0838 mmol, yield 30%) as a light yellow solid.

### [Synthesis of k-1:H-5]

k-1 (100 mg, 0.555 mmol), H-5 (122 mg, 0.666 mmol) were dissolved in DMSO (1.1 mL), and the mixture was stirred at 100°C for 14 hr. Distilled water (11 mL) was added at the same temperature and the mixture was allowed to cool, and the precipitated solid was collected by filtration, and washed with methanol to give k-1:H-5 (55.6 mg, 0.161 mmol, yield 29%) as a light yellow solid.

### [Synthesis of k-1:H-6]

k-1 (100 mg, 0.555 mmol), H-6 (143 mg, 0.666 mmol) were dissolved in DMSO (1.1 mL), and the mixture was stirred at 100°C for 14 hr. The mixture was allowed to cool, distilled water (11 mL) was added and the precipitated solid was collected by filtration and washed with methylene chloride to give k-1:H-6 (86.5 mg, 0.229 mmol, yield 41%) as a brown solid.

### [Synthesis of k-1:H-7]

k-1 (50 mg, 0.28 mmol), H-7 (54 mg, 0.28 mmol) were dissolved in DMSO (0.55 mL), and the mixture was stirred at 100°C for 17 hr. The mixture was allowed to cool, distilled water (6 mL) was added and the mixture was decantated, and the residue was dissolved in methylene chloride (0.5 mL). Hexane (0.5 mL) was added and the precipitated solid was collected by filtration to give k-1:H-7 (56.8 mg, 0.160 mmol, yield 57%) as a white solid.

### [Synthesis of k-1:H-9]

k-1 (150 mg, 0.83 mmol), H-9 (226 mg, 1.08 mmol) was suspended in DMSO (0.55 mL) and the mixture was stirred at 100°C for 17 hr. The mixture was allowed to cool, distilled water (20 mL) was added and the mixture was decantated. The obtained residue was purified by moderate-pressure silica gel column chromatography (silica gel 10 g, ethyl acetate/hexane=10/90 - 60/40). The obtained solid was washed with methylene chloride to give k-1:H-9 (40.2 mg, 0.108 mmol, yield 13%) as a white solid.

### [Synthesis of k-2:H-1]

k-2 (80 mg, 0.48 mmol), H-1 (95.4 mg, 0.578 mmol) were dissolved in DMSO (1.0 mL), and the mixture was stirred at 100°C for 15 hr. The mixture was allowed to cool, distilled water (10 mL) was added and the precipitated solid was collected by filtration and washed with methylene chloride to give k-2:H-1 (80.4 mg, 0.257 mmol, yield 53%) as a yellow solid.

### [Synthesis of k-2:H-2]

k-2 (80 mg, 0.48 mmol), H-2 (112 mg, 0.625 mmol) were dissolved in DMSO (1 mL), and the mixture was stirred at 100°C for 19 hr. The mixture was allowed to cool, distilled water (30 mL), ethyl acetate (30 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 80/20) to give k-2:H-2 (34 mg, 0.10 mmol, yield 21%) as a light yellow solid.

### [Synthesis of k-2:H-3]

k-2 (80 mg, 0.48 mmol), H-3 (104 mg, 0.578 mmol) were dissolved in DMSO (1 mL), and the mixture was stirred at 100°C for 15 hr. The mixture was allowed to cool, distilled water (10 mL) was added and the mixture was decantated. The obtained residue was dissolved in methylene chloride (3 mL), hexane (3 mL) was added and the precipitated solid was collected by filtration. The obtained solid was purified by moderate-pressure silica gel column chromatography (silica gel 10 g, ethyl acetate/hexane=50/50 - 80/20) to give k-2:H-3 (46.9 mg, 0.143 mmol, yield 30%) as a light yellow solid.

### [Synthesis of k-2:H-4]

k-2 (80 mg, 0.48 mmol), H-4 (112 mg, 0.625 mmol) were dissolved in DMSO (1 mL), and the mixture was stirred at 100°C for 19 hr. The mixture was allowed to cool, distilled water (10 mL) was added, the mixture was decantated, and the residue was washed with methylene chloride to give k-2:H-4 (58.7 mg, 0.179 mmol, yield 37%) as a light yellow solid.

### [Synthesis of k-2:H-5]

k-2 (80 mg, 0.48 mmol), H-5 (106 mg, 0.578 mmol) were dissolved in DMSO (1 mL), and the mixture was stirred at 100°C for 15 hr. The mixture was allowed to cool, distilled water (10 mL) was added and the mixture was decantated. The obtained residue was dissolved in methylene chloride (3 mL), hexane (1 mL) was added and the precipitated solid was collected by filtration. The obtained solid was purified by moderate-pressure silica gel column chromatography (silica gel 10 g, ethyl acetate/hexane=50/50 - 80/20) to give k-2:H-5 (36.6 mg, 0.110 mmol, yield 23%) as a white solid.

### [Synthesis of k-2:H-6]

k-2 (100 mg, 0.602 mmol), H-6 (155 mg, 0.722 mmol) were dissolved in DMSO (1 mL), and the mixture was stirred at 100°C for 15 hr. The mixture was allowed to cool, distilled water (10 mL) was added and the mixture was decantated. The obtained residue was washed with methylene chloride to give k-2:H-6 (59.3 mg, 0.163 mmol, yield 27%) as a light brown solid.

### [Synthesis of k-2:H-7]

k-2 (80 mg, 0.48 mmol), H-7 (121 mg, 0.626 mmol) were dissolved in DMSO (0.55 mL), and the mixture was stirred at 100°C for 17 hr. The mixture was allowed to cool, distilled water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=5/95 - 50/50) to give k-2:H-7 (109 mg, 0.319 mmol, yield 66%) as a light yellow solid.

### [Synthesis of k-2:H-9]

k-2 (100 mg, 0.60 mmol), H-9 (164 mg, 0.784 mmol) were suspended in DMSO (1.2 mL) and the mixture was stirred at 100°C for 18 hr. The mixture was allowed to cool, distilled water (12 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 60/40), and the obtained solid was washed with methylene chloride to give k-2:H-9 (63.1 mg, 0.177 mmol, yield 30%) as a light yellow solid.

### [Synthesis of k-3:H-1]

k-3 (200 mg, 1.02 mmol), H-1 (253 mg, 1.53 mmol) were dissolved in DMSO (2.0 mL) and the mixture was stirred at 100°C for 3 days. The mixture was allowed to cool, distilled water (15 mL) was added and the mixture was decantated. The obtained residue was purified by moderate-pressure silica gel column chromatography (silica gel 30 g, ethyl acetate/hexane=40/60 - 70/30). The obtained solid was washed with methylene chloride to give k-3:H-1 (25.0 mg, 0.0728 mmol, yield 7.1%) as a light brown solid.

### [Synthesis of k-3:H-2]

k-3 (200 mg, 1.02 mmol), H-2 (237 mg, 1.33 mmol) were dissolved in DMSO (2 mL) and the mixture was stirred at 100°C for 4 days. The mixture was allowed to cool, distilled water (15 mL) was added and the precipitated solid was collected by filtration and washed with methanol. The obtained solid was purified by moderate-pressure silica gel column chromatography (silica gel 30 g, ethyl acetate/hexane=40/60 - 70/30). The obtained solid was washed with methylene chloride to give k-3:H-2 (10.3 mg, 0.0288 mmol, yield 2.8%) as a light brown solid.

### [Synthesis of k-3:H-3]

k-3 (200 mg, 1.02 mmol), H-3 (219 mg, 1.22 mmol) were dissolved in DMSO (2 mL) and the mixture was stirred at 100°C for 5 days. The mixture was allowed to cool, distilled water (15 mL) was added and the precipitated solid was collected by filtration and purified by moderate-pressure silica gel column chromatography (silica gel 30 g, ethyl acetate/hexane=40/60 - 70/30). The obtained solid was washed with methylene chloride to give k-3:H-3 (17.9 mg, 0.0501 mmol, yield 4.9%) as a light brown solid.

### [Synthesis of k-3:H-4]

k-3 (200 mg, 1.02 mmol), H-4 (237 mg, 1.33 mmol) were dissolved in DMSO (2 mL) and the mixture was stirred at 100°C for 4 days. The mixture was allowed to cool, distilled water (15 mL) was added and the precipitated solid was collected by filtration and purified by moderate-pressure silica gel column chromatography (silica gel 30 g, ethyl acetate/hexane=40/60 - 70/30). The obtained solid was washed with methylene chloride to give k-3:H-4 (17.9 mg, 0.0501 mmol, yield 4.9%) as a light brown solid.

### [Synthesis of k-3:H-5]

k-3 (200 mg, 1.02 mmol), H-5 (224 mg, 1.22 mmol) were dissolved in DMSO (2 mL) and the mixture was stirred at 100°C for 6 days. The mixture was allowed to cool, distilled water (15 mL) was added and the mixture was decantated. The obtained residue was dissolved in methylene chloride (3 mL), hexane (1 mL) was added and the precipitated solid was collected by filtration and purified by moderate-pressure silica gel column chromatography (silica gel 30 g, ethyl acetate/hexane=40/60 - 70/30). The obtained solid was washed with methylene chloride to give k-3:H-5 (13.7 mg, 0.0379 mmol, yield 3.7%) as a light brown solid.

### [Synthesis of k-3:H-6]

k-3 (100 mg, 0.510 mmol), H-6 (121 mg, 0.561 mmol) were dissolved in DMSO (2 mL) and the mixture was stirred at 100°C for 4 days. The mixture was allowed to cool, distilled water (15 mL) was added and the mixture was decantated. The obtained residue was purified by moderate-pressure silica gel column chromatography (silica gel 30 g, ethyl acetate/hexane=40/60 - 70/30). The obtained solid was washed with methylene chloride to give k-3:H-6 (17.0 mg, 0.0432 mmol, yield 8.5%) as an orange solid.

### [Synthesis of k-3:H-7]

k-3 (200 mg, 1.02 mmol), H-7 (256 mg, 1.33 mmol) were dissolved in DMSO (2 mL) and the mixture was stirred at 100°C for 3 days. The mixture was allowed to cool, distilled water (15 mL) was added and the mixture was decantated. The obtained residue was purified by moderate-pressure silica gel column chromatography (silica gel 30 g, ethyl acetate/hexane=40/60 - 70/30). The obtained solid was washed with methylene chloride to give k-3:H-7 (28.3 mg, 0.762 mmol, yield 7.5%) as a white solid.

### [Synthesis of k-3:H-9]

k-3 (200 mg, 1.02 mmol), H-9 (277 mg, 1.33 mmol) were dissolved in DMSO (2 mL) and the mixture was stirred at 100°C for 4 days. The mixture was allowed to cool, distilled water (15 mL) was added and the precipitated solid was filtered and purified by moderate-pressure silica gel column chromatography (silica gel 30 g, ethyl acetate/hexane=40/60 - 70/30). The obtained solid was washed with methylene chloride to give k-3:H-9 (11.2 mg, 0.0289 mmol, yield 2.8%) as a light brown solid.

### [Synthesis of k-5:H-1]

k-5 (100 mg, 0.48 mmol), H-1 (95.2 mg, 0.576 mmol) were dissolved in DMSO (1 mL) and the mixture was stirred at 100°C for 4 days. The mixture was allowed to cool, distilled water (10 mL) was added and the precipitated solid was collected by filtration and washed successively with methylene chloride and methanol to give k-5:H-1 (38.1 mg, 0.107 mmol, yield 22%) as a yellow solid.

### [Synthesis of k-5:H-2]

k-5 (100 mg, 0.48 mmol), H-2 (112 mg, 0.625 mmol) were dissolved in DMSO (1 mL) and the mixture was stirred at 100°C for 3 days. Distilled water (10 mL) was added and the mixture was decantated, and the residue was dissolved in methylene chloride (2 mL), dried over salt cake, filtered, and concentrated under reduced pressure. Methylene chloride (1 mL) was added to the obtained residue, and the mixture was subjected to ultrasonication and the precipitated solid was collected by filtration to give k-5:H-2 (18.6 mg, 0.0503 mmol, yield 10%) as a yellow solid.

### [Synthesis of k-5:H-3]

k-5 (100 mg, 0.480 mmol), H-3 (103 mg, 0.576 mmol) were dissolved in DMSO (1 mL) and the mixture was stirred at 100°C for 4 days. The mixture was allowed to cool, distilled water (10 mL) was added and the precipitated solid was collected by filtration and washed with methylene chloride to give k-5:H-3 (44.6 mg, 0.121 mmol, yield 25%) as a yellow solid.

### [Synthesis of k-5:H-4]

k-5 (100 mg, 0.480 mmol), H-4 (112 mg, 0.625 mmol) were dissolved in DMSO (1 mL) and the mixture was stirred at 100°C for 3 days. Distilled water (10 mL) was added and the precipitated solid was collected by filtration and washed with methylene chloride to give k-5:H-4 (44.4 mg, 0.120 mmol, yield 25%) as a yellow solid.

### [Synthesis of k-5:H-5]

k-5 (100 mg, 0.480 mmol), H-5 (106 mg, 0.576 mmol) were dissolved in DMSO (2 mL) and the mixture was stirred at 100°C for 4 days. The mixture was allowed to cool, distilled water (10 mL) was added and the precipitated solid was collected by filtration to give k-5:H-5 (37.4 mg, 0.100 mmol, yield 21%) as a yellow solid.

### [Synthesis of k-5:H-6]

k-5 (100 mg, 0.480 mmol), H-6 (124 mg, 0.576 mmol) were dissolved in DMSO (1 mL) and the mixture was stirred at 100°C for 5 days. The mixture was allowed to cool, distilled water (10 mL) was added and the precipitated solid was collected by filtration and purified by moderate-pressure silica gel column chromatography (silica gel 10 g, ethyl acetate/hexane=20/80 - 50/50). The obtained solid was washed with methanol to give k-5:H-6 (28.1 mg, 0.0693 mmol, yield 14%) as a light yellow solid.

### [Synthesis of k-5:H-7]

k-5 (100 mg, 0.480 mmol), H-7 (121 mg, 0.626 mmol) were dissolved in DMSO (1 mL) and the mixture was stirred at 100°C for 4 days. The mixture was allowed to cool, distilled water (10 mL) was added and the precipitated solid was collected by filtration and washed successively with ethyl acetate, methylene chloride, and methanol to give k-5:H-7 (43.6 mg, 0.114 mmol, yield 24%) as a white solid.

### [Synthesis of k-5:H-9]

k-5 (150 mg, 0.72 mmol), H-9 (196 mg, 0.937 mmol) were suspended in DMSO (1.4 mL) at 100°C for 3 days. The mixture was allowed to cool, distilled water (12 mL), methylene chloride (20 mL) were added and the mixture was partitioned. The organic layer was washed successively with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 50/50) to give k-5:H-9 (72.0 mg, 0.180 mmol, yield 25%) as a light yellow solid.

In addition, compound Nos. k-1:D-2 and k-1:D-4 can also be synthesized by methods according to the above-mentioned synthesis methods.

[Synthetic Example 2] Synthesis of k-1:A-1

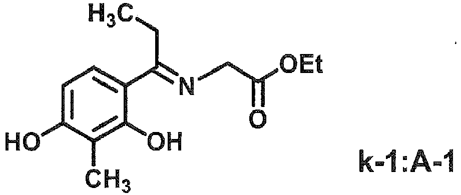

k-1 (100 mg, 0.55 mmol), glycine ethyl ester hydrochloride (A-1) (100 mg, 0.72 mmol), sodium acetate (64 mg, 0.78 mmol) were dissolved in DMSO (1.1 mL), and the mixture was stirred at 100°C for 2 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 50/50). The obtained solid was washed with IPE to give k-1:A-1 (21.9 mg, 0.0825 mmol, yield 15%) as a yellow solid.
¹H-NMR (270 MHz); δ9.69(s, 1H), 7.32(d, J=8.1Hz, 1H), 6.30(d, J=10.8Hz, 1H), 5.76(s, 1H), 4.48(s, 2H), 4.19(q, J=8.1Hz, 2H), 2.67(q, J=8.1Hz, 2H), 1.94(s, 3H), 1.24(t, J=8.1Hz, 3H), 1.08(t, J=8.1Hz, 3H).

### [Synthetic Example 3] Synthesis of k-1:A-2

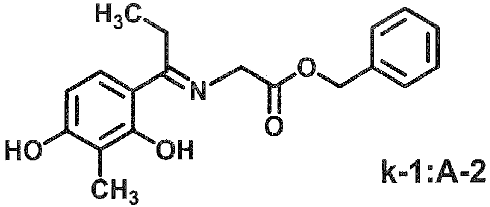

k-1 (100 mg, 0.55 mmol), glycine benzyl ester p-toluenesulfonate (A-2) (243 mg, 0.721 mmol), sodium acetate (64 mg, 0.78 mmol) were dissolved in DMSO (1.1 mL), and the mixture was stirred at 100°C for 2 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=5/95 - 50/50). The obtained solid was washed with IPE to give k-1:A-2 (25.0 mg, 0.0764 mmol, yield 14%) as a yellow solid.
¹H-NMR (270 MHz); δ9.69(s, 1H), 7.45-7.35 (m, 6H), 7.32(d, J=10.8Hz, 1H), 6.31(d, J=10.8Hz, 1H), 5.23(s, 2H), 4.56(s, 2H), 2.70(q, J=8.1Hz, 2H), 1.94(s, 3H), 1.08(t, J=8.1Hz, 3H).

### [Synthetic Example 4] Synthesis of k-1:A-3

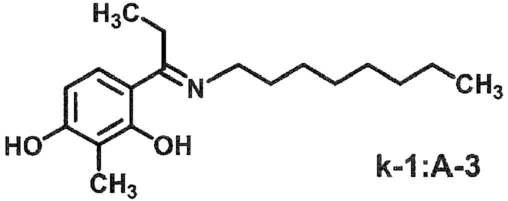

k-1 (100 mg, 0.55 mmol) was dissolved in DMSO (1.1 mL), n-octylamine (A-3) (120 mL, 0.72 mmol) was added and the mixture was stirred at room temperature for 2 hr and at 100°C for 17 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=0/100 - 20/80). The obtained solid was washed with IPE to give k-1:A-3 (60.1 mg, 0.206 mmol, yield 37%) as a yellow solid.
¹H-NMR (270 MHz); δ9.52(s, 1H), 7.25(d, J=8.1Hz, 1H), 6.20(d, J=8.1Hz, 1H), 5.31 (t, J=8.1Hz, 2H), 2.73(q, J=8.1Hz, 2H), 1.90(s, 3H), 1.64(m, 2H), 1.30-1.05(m, 10H), 1.11(t, J=8.1Hz, 3H), 0.86(t, J=8.1Hz, 3H).

### [Synthetic Example 5] Synthesis of k-1:A-5

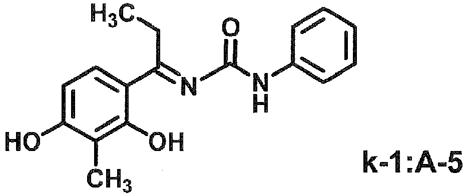

k-1 (300 mg, 1.7 mmol) was dissolved in 2 M ammonia/methanol solution (17 mL, 33 mmol), and the mixture was stirred at room temperature for 1 week. During that time, ammonia gas was bubbled every day for 15 min. The reaction solution was concentrated under reduced pressure to give a mixture of 4-(1-iminopropyl)-2-methylbenzene-1,3-diol (k-1') and k-1 (292 mg, k-1':k-1=1:0.2) as a yellow ocher solid. A mixture (292 mg) of k-1' and k-1 obtained as mentioned above was dissolved in THF (6.5 mL), and phenylisocyanate (A-5) (158 mL, 1.46 mmol) was added under ice-cooling. After stirring for 30 min at the same temperature, water (30 mL), ethyl acetate (30 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=5/95 - 45/55). The obtained solid was washed with IPE to give k-1:A-5 (160 mg, 0.536 mmol, 2 step yield 32%) as a light yellow solid.
¹H-NMR (270 MHz); δ13.74(s, 1H), 10.22(s, 1H), 10.11(s, 1H), 7.63(d, J=8.1Hz, 2H), 7.53 (d, J=8.1Hz, 1H), 7.32 (t, J=8.1Hz, 2H), 7.06 (t, J=8.1Hz, 1H), 6.49 (d, J=8.1Hz, 1H), 2.84(q, J=8.1Hz, 2H), 2.00(s, 3H), 1.24(t, J=8.1Hz, 3H).

### [Synthetic Example 6] Synthesis of k-1:H-10

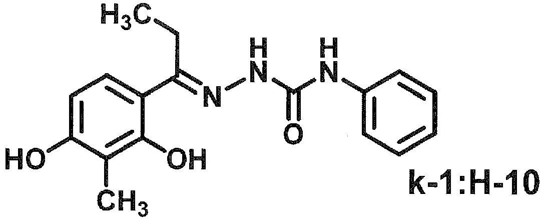

k-1 (100 mg, 0.55 mmol), 4-phenylsemicarbazide (H-10) (109 mg, 0.721 mmol) were dissolved in DMSO (1.1 mL), and the mixture was stirred at 100°C for 3.5 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 50/50). The obtained solid was washed with IPE to give k-1:H-10 (19.1 mg, 0.0610 mmol, yield 11%) as a white solid.
¹H-NMR (270 MHz) :δ13.50(s, 1H), 9.75(s, 1H), 9.59(s, 1H), 8.82(s, 1H), 7.49(d, J=8.1Hz, 2H), 7.30(t, J=8.1Hz, 2H), 7.21(d, J=8.0Hz, 1H), 6.99(t, J=8.1Hz, 1H), 6.39(d, J=8.1Hz, 1H), 2.70(q, J=8.1Hz, 2H), 1.99(s, 3H), 1.14(t, J=8.1Hz, 3H).

### [Synthetic Example 7] Synthesis of k-1:I-1

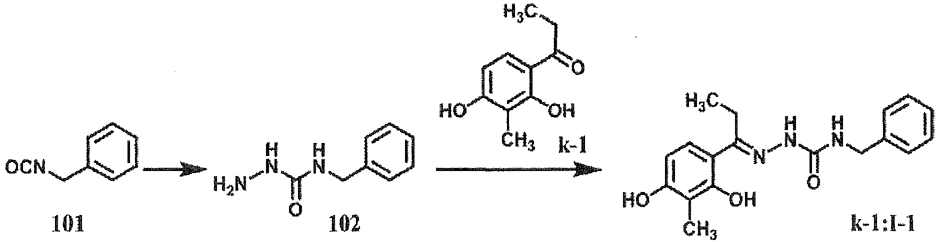

Hydrazine monohydrate (0.26 mL, 5.3 mmol) was dissolved in methylene chloride (5.3 mL), and benzyl isocyanate (101) (0.324 mL, 2.63 mmol) was slowly added under ice-cooling. The mixture was stirred at room temperature for 3 hr, and the precipitated solid was washed with IPE, and dried under reduced pressure to give 102 (352 mg, 2.13 mmol, yield 82%) as a white solid.

102 (155 mg, 0.938 mmol) obtained as mentioned above and k-1 (130 mg, 0.72 mmol) were dissolved in DMSO (1.4 mL), and the mixture was stirred at 100°C for 3.5 hr. The mixture was allowed to cool, distilled water (10 mL) was added, and the precipitated solid was collected by filtration, and purified by moderate-pressure silica gel column chromatography (silica gel 10 g, ethyl acetate/hexane=10/90 - 55/45). The obtained solid was washed with methylene chloride, and dried under reduced pressure to give k-1:I-1 (55 mg, 0.17 mmol, yield 24%) as a white solid.
¹H-NMR (400 MHz); δ9.59(s, 1H), 9.56(s, 1H), 7.40-7.25(m, 5H), 7.17(d, J=12.0Hz, 1H), 6.84(t, J=8.0Hz, NH), 6.37(d, J=12.0Hz, 1H), 4.34(d, J=8.0Hz, 2H), 2.65(q, J=8.0Hz, 2H), 1.97(s, 3H), 1.08(t, J=8.0Hz, 3H).(one signal of NH was not observed)

### [Synthetic Example 8] Synthesis of k-1:I-3

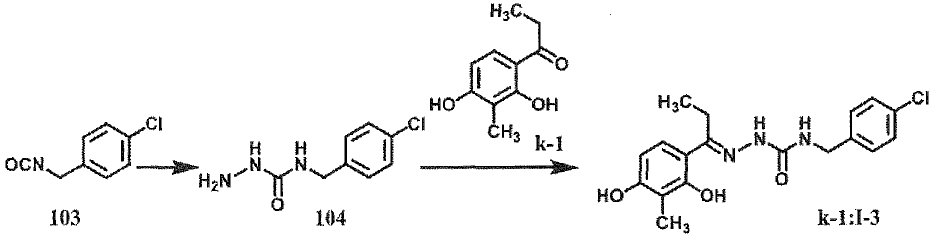

Hydrazine monohydrate (0.20 mL, 4.2 mmol) was dissolved in methylene chloride (4.2 mL), and 4-chlorobenzyl isocyanate (103) (0.278 mL, 2.09 mmol) was slowly added under ice-cooling. The mixture was stirred at room temperature for 1.5 hr, and the precipitated solid was collected by filtration and dried under reduced pressure to give 104 (315 mg, 1.58 mmol, yield 75%) as a white solid.

104 (187 mg, 0.937 mmol) obtained as mentioned above, k-1 (130 mg, 0.72 mmol) were dissolved in DMSO (1.4 mL), and the mixture was stirred at 100°C for 22 hr. The reaction solution was directly purified by moderate-pressure silica gel column chromatography (silica gel 10 g, ethyl acetate/hexane=10/90 - 65/35), the obtained purified product was dissolved in ethyl acetate, hexane was added and the precipitated solid was collected by filtration, and dried under reduced pressure to give k-1:I-3 (155 mg, 0.428 mmol, yield 59%) as a white solid.
¹H-NMR (400 MHz); δ9.63(s, 1H), 9.56(s, 1H), 7.41(d, J=8.0Hz, 2H), 7.34(d, J=8.0Hz, 2H), 7.17(d, J=8.0Hz, 1H), 6.89(t, J=8.0Hz, NH), 6.36(d, J=8.0Hz, 1H), 4.31(d, J=8.0Hz, 2H), 2.65(q, J=8.0Hz, 2H), 1.97(s, 3H), 1.08(t, J=8.0Hz, 3H).(one signal of NH was not observed)

### [Synthetic Example 9] Synthesis of k-1:I-6

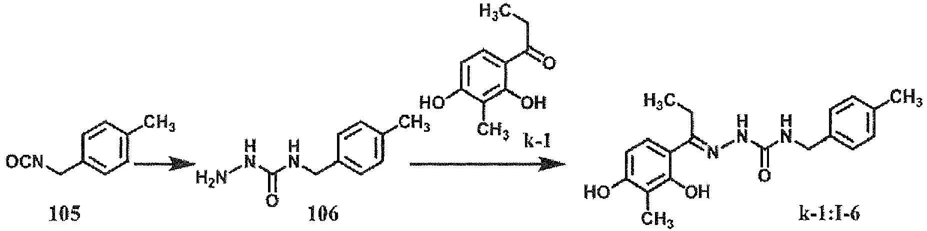

Hydrazine monohydrate (0.23 mL, 4.8 mmol) was dissolved in methylene chloride (8 mL), and 4-methylbenzyl isocyanate (105) (350 mg, 2.4 mmol) was slowly added under ice-cooling. The mixture was stirred at room temperature for 2 hr, and the precipitated solid was collected by filtration and dried under reduced pressure to give 106 (297 mg, 1.66 mmol, yield 69%) as a white solid.

106 (168 mg, 0.937 mmol) obtained as mentioned above, k-1 (130 mg, 0.72 mmol) were suspended in DMSO (2.8 mL) and the mixture was stirred at 100°C for 22 hr. The reaction solution was directly purified by moderate-pressure silica gel column chromatography (silica gel 10 g, ethyl acetate/hexane=10/90 - 60/40), and the obtained purified product was dissolved in ethyl acetate (30 mL). The mixture was washed successively with water (20 mL), saturated brine (30 mL), dried over anhydrous sodium sulfate, filtered, and concentrated under reduced pressure. The obtained solid was washed with methylene chloride, and dried under reduced pressure to give k-1:I-6 (118 mg, 0.346 mmol, yield 48%) as a white solid.

¹H-NMR (400 MHz); δ9.56(s, 1H), 9.55(s, 1H), 7.20(d, J=8.0Hz, 2H), 7.16(d, J=8.0Hz, 2H), 7.15(d, J=8.0Hz, 1H), 6.78(t, J=8.0Hz, NH), 6.36(d, J=8.0Hz, 1H), 4.29(d, J=8.0Hz, 2H), 2.64(q, J=8.0Hz, 2H), 2.28(s, 3H), 1.97(s, 3H), 1.07(t, J=8.0Hz, 3H).(one signal of NH was not observed)

### [Synthetic Example 10] Synthesis of k-1:I-7

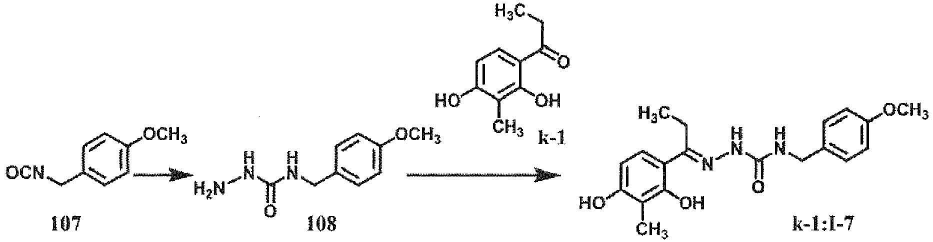

Hydrazine monohydrate (0.21 mL, 4.3 mmol) was dissolved in methylene chloride (4.3 mL), under ice-cooling 4-methoxybenzyl isocyanate (107) (350 mg, 2.15 mmol) was slowly added. The mixture was stirred at room temperature for 2 hr, and the precipitated solid was collected by filtration and dried under reduced pressure to give 108 (381 mg, 1.95 mmol, yield 93%) as a white solid.

108 (183 mg, 0.937 mmol) obtained as mentioned above, k-1 (130 mg, 0.72 mmol) were suspended in DMSO (2.8 mL) and the mixture was stirred at 100°C for 5 hr. The mixture was allowed to cool, distilled water (15 mL) was added, and the precipitated solid was collected by filtration, and purified by moderate-pressure silica gel column chromatography (silica gel 10 g, ethyl acetate/hexane=15/85 - 65/35). The obtained solid was washed with methylene chloride and dried under reduced pressure to give k-1:I-7 (55.0 mg, 0.154 mmol, yield 21%) as a white solid.
¹H-NMR (400 MHz); δ9.55(s, 2H), 7.25(d, J=8.0Hz, 2H), 7.16(d, J=8.0Hz, 1H), 6.91(d, J=8.0Hz, 2H), 6.75(t, J=8.0Hz, NH), 6.36(d, J=8.0Hz, 1H), 4.26(d, J=8.0Hz, 2H), 3.73(s, 3H), 2.63(q, J=8.0Hz, 2H), 1.97(s, 3H), 1.07(t, J=8.0Hz, 3H).(one signal of NH was not observed)

The compounds synthesized by methods according to the above-mentioned synthesis methods are shown in the third table.

### [the third table]

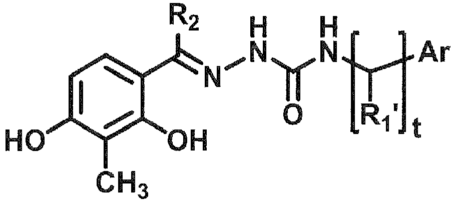

**[Table 3]**

| compound No. | R₂ | R₁' | t | Ar |
|---|---|---|---|---|
| k-1:I-8 | Et | H | 1 | 2-OH-Ph |
| k-1:I-9 | Et | H | 1 | 4-OH-Ph |
| k-1:I-10 | Et | Me | 1 | 3-OH-Ph |
| k-1:I-11 | Et | H | 2 | 3-OH-Ph |
| k-1:I-12 | Et | H | 1 | 3,5-di-OH-Ph |

The measurement results of ¹H-NMR of the compounds described in the third table are shown in the fourth table.

### [the fourth table]

**[Table 4]**

| compound No. | ¹H-NMR (270 MHz) |
|---|---|
| k-1:I-8 | δ13.46(s, 1H), 9.66(s, 1H), 9.63(s, 1H), 9.53(s, 1H), 7.20-7.05(m, 3H), 6.85-6.70(m, 3H), 6.37(d, J=8.1Hz, 1H), 4.26(d, J=5.4Hz, 2H), 2.64(q, J=8.1Hz, 2H), 1.97(s, 3H), 1.07(t, J=8.1Hz, 3H). |
| k-1:I-9 | δ13.47(s, 1H), 9.53(s, 1H), 9.51(s, 1H), 9.31(s, 1H), 7.16(d, J=8.1Hz, 1H), 7.13(d, J=8.1Hz, 2H), 6.73(d, J=8.1Hz, 2H), 6.71(t, J=8.1Hz, NH), 6.36(d, J=8.1Hz, 1H), 4.20(d, J=8.1Hz, 2H), 2.62(q, J=8.1Hz, 2H), 1.97(s, 3H), 1.06(t, J=8.1Hz, 3H). |
| k-1:I-10 | δ13.50 (s, 1H), 9.51(s, 1H), 9.46(s, 1H), 9.37(s, 1H), 7.16(d, J=8.1Hz, 1H), 7.13(d, J=8.1Hz, 1H), 6.80-6.70(m, 3H), 6.65(d, J=8.1Hz, NH), 6.35(d, J=8.1Hz, 1H), 4.74(m, 1H), 2.63(q, J=8.1Hz, 2H), 1.96(s, 3H), 1.38(d, J=5.4Hz, 3H), 1.06(t, J=8.1Hz, 3H). |
| k-1:I-11 | δ13.48(s, 1H), 9.53(s, 2H), 9.31(s, 1H), 7.15(d, J=8.1Hz, 1H), 7.09(d, J=8.1Hz, 1H), 6.70-6.60(m, 3H), 6.38(d, J=8.1Hz, 1H), 6.34(t, J=8.1Hz, NH), 3.40-3.30(m, 2H), 2.69(q, J=8.1Hz, 2H), 2.60-2.50(m, 2H), 1.97(s, 3H), 1.06(t, J=8.1Hz, 3H). |
| k-1:I-12 | δ13.47 (s, 1H), 9.56(s, 1H), 9.54(s, 1H), 9.19(s, 2H), 7.17(d, J=8.1Hz, 1H), 6.70(t, J=8.1Hz, NH), 6.37(d, J=8.1Hz, 1H), 6.17(s, 1H), 6.16(s, 1H), 6.08(s, 1H), 4.15(d, J=5.4Hz, 2H), 2.65(q, J=8.1Hz, 2H), 1.97(s, 3H), 1.08(t, J=8.1Hz, 3H). |

### [Synthetic Example 11] Synthesis of k-1:B-1

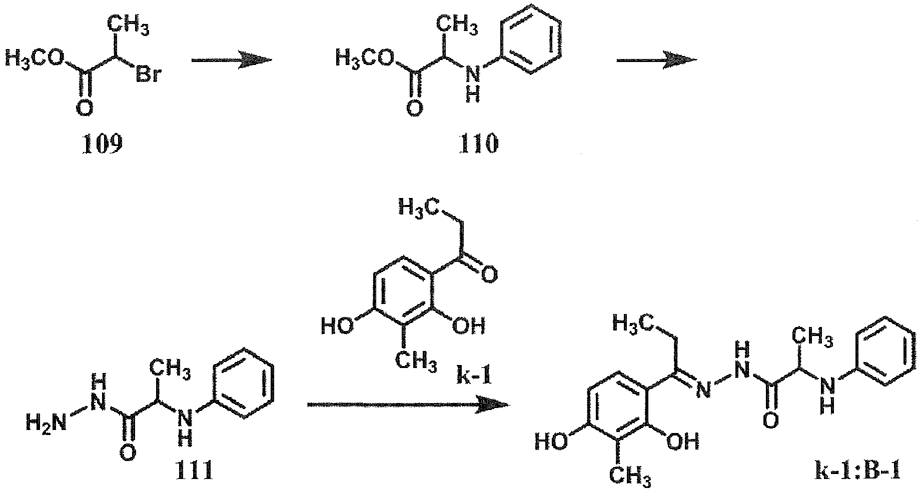

Methyl 2-bromopropionate (109) (500 mg, 3.0 mmol) was dissolved in DMSO (6 mL), aniline (0.36 mL, 3.9 mmol), potassium carbonate (0.54 g, 3.9 mmol) were added and the mixture was stirred at room temperature for 21 hr. Ethyl acetate (30 mL), water (50 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel chromatography (silica gel 30 g, ethyl acetate/hexane=2/98 - 15/85) to give 110 (343 mg, 1.91 mmol, yield 64%) as a light yellow liquid.

110 (340 mg, 1.9 mmol) obtained as mentioned above was dissolved in methanol (3.8 mL), hydrazine monohydrate (0.18 mL, 3.8 mmol) was added, and the mixture was stirred at 60°C for 24 hr. Hydrazine monohydrate (0.36 mL, 7.4 mmol) was added and the mixture was further stirred at 60°C for 17 hr. The reaction solution was concentrated under reduced pressure. The obtained residue was purified by moderate-pressure silica gel chromatography (aminesilica gel 10 g, ethyl acetate/methylene chloride=0/100 - 20/80) to give 111 (321 mg, 1.79 mmol, yield 94%) as a white solid.

111 (168 mg, 0.937 mmol) obtained as mentioned above, k-1 (130 mg, 0.72 mmol) were dissolved in DMSO (1.4 mL), and the mixture was stirred at 100°C for 19 hr. The mixture was allowed to cool, distilled water (15 mL) was added, and the precipitated solid was collected by filtration, and dried. The obtained yellow solid was washed with methylene chloride, and dried under reduced pressure to give k-1:B-1 (173 mg, 0.507 mmol, yield 70%) as a light yellow solid.
¹H-NMR (400 MHz):δ10.82(s, 1H), 9.71(s, 1H), 7.25(d, J=8.0Hz, 1H), 7.07(t, J=8.0Hz, 2H), 6.63(d, J=8.0Hz, 2H), 6.56(t, J=8.0Hz, 1H), 6.39(d, J=8.0Hz, 1H), 5.93(d, J=12Hz, NH), 4.28(m, 1H), 2.80(q, J=8.0Hz, 2H), 1.95(s, 3H), 1.40(d, J=8.0Hz, 3H), 1.03(t, J=8.0Hz, 3H). (one signal of NH was not observed)

### [Synthetic Example 12] Synthesis of compound GA-002A and compound GA-002B

k-1:B-1 (racemate) synthesized in the aforementioned Synthetic Example 11 was optically resolved by supercritical fluid chromatography (SFC) manufactured by Waters according to the following conditions to obtain the following two enantiomers. The specific optical rotation of the both enantiomers was measured using an optical rotation meter P-1020 (manufactured by JASCO Corporation). The steric configuration (R form, S form) of the enantiomers was determined by X-ray crystal structure analysis.
compound GA-002A (levorotatory enantiomer of k-1:B-1); retention time 11.95 min, fraction 475.1 mg, optical purity 99.9ee%, purity 99.0%, specific optical rotation [α]²²_{D} -10.5 (c=0.1019, ethanol), steric configuration R form compound GA-002B (dextrorotatory enantiomer of k-1:B-1); retention time 14.85 min, fraction 474.0 mg, optical purity 99.9ee%, purity 99.1%, specific optical rotation [α]²²_{D} +12.6 (c=0.1012, ethanol), steric configuration S form

### <Conditions>

column: CHIRALPAK IA <manufactured by Daicel Corporation, 20^{*}250 mm, 5 µm>, weak solvent: CO₂ (70%), strong solvent: MeOH (30%), column temperature: 40°C, total charge: 1 g, flow rate: 15 mL/min

### [Synthetic Example 13] Synthesis of k-1:B-2

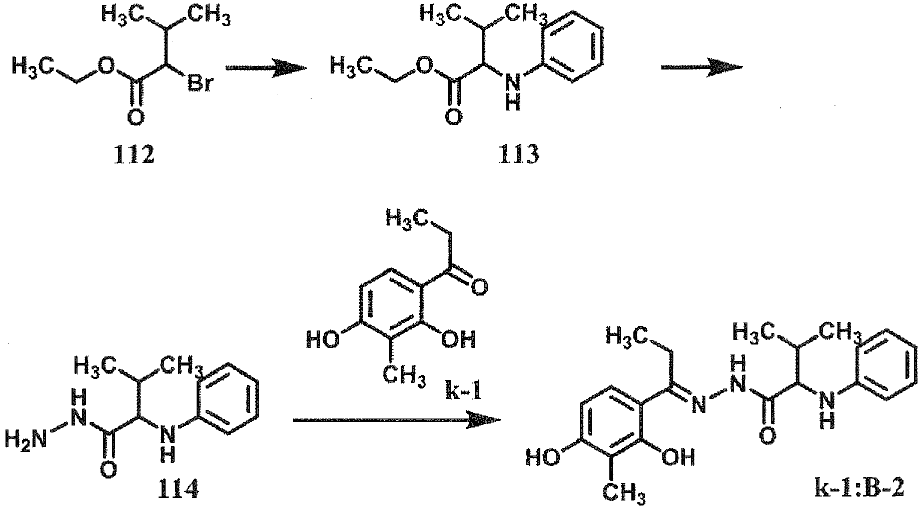

Ethyl 2-bromoisovalerate (112) (700 mg, 3.3 mmol) was dissolved in DMSO (7 mL), aniline (0.40 mL, 4.4 mmol), potassium carbonate (0.60 g, 4.4 mmol) were added, and the mixture was stirred at room temperature for 21 hr, at 80°C for 6 hr, and at 120°C for 20 hr. Ethyl acetate (30 mL), water (50 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel chromatography (silica gel 30 g, ethyl acetate/hexane=0/100 - 5/95) to give 113 (110 mg, 0.497 mmol, yield 15%) as a yellow liquid.

113 (96 mg, 0.43 mmol) obtained as mentioned above was dissolved in methanol (1 mL), hydrazine monohydrate (0.042 mL, 0.87 mmol) was added and the mixture was stirred at 50°C for 4 hr. Hydrazine monohydrate (0.2 mL, 4.1 mmol) was added and the mixture was stirred for 20 hr, hydrazine monohydrate (0.1 mL, 2.1 mmol) was added and the mixture was further stirred for 23 hr. The reaction solution was concentrated under reduced pressure, and the obtained residue was purified by moderate-pressure silica gel chromatography (aminesilica gel 10 g, ethyl acetate/methylene chloride=0/100 - 5/95) to give 114 (80.7 mg, 0.389 mmol, yield 90%) as a white solid.

114 (75 mg, 0.36 mmol) obtained as mentioned above, k-1 (50 mg, 0.28 mmol) were dissolved in DMSO (0.6 mL), and the mixture was stirred at 100°C for 18 hr. The mixture was allowed to cool, and the reaction solution was directly purified by moderate-pressure silica gel column chromatography (silica gel 10 g, ethyl acetate/hexane=10/90 - 75/25). The obtained solid was washed with methylene chloride/IPE, and dried under reduced pressure to give k-1:B-2 (37.6 mg, 0.102 mmol, yield 36%) as a white solid.
¹H-NMR (400 MHz):δ10.83(s, 1H), 9.72(s, 1H), 7.26(d, J=8.0Hz, 1H), 7.05(t, J=8.0Hz, 2H), 6.71(d, J=8.0Hz, 2H), 6.53(t, J=8.0Hz, 1H), 6.39(d, J=8.0Hz, 1H), 5.78(d, J=12Hz, NH), 3.97(m, 1H), 2.82(q, J=8.0Hz, 2H), 2.03(m, 1H), 1.95(s, 3H), 1.06(t, J=8.0Hz, 3H), 0.97(d, J=8.0Hz, 6H).(one signal of NH was not observed)

### [Synthetic Example 14] Synthesis of k-1:B-3

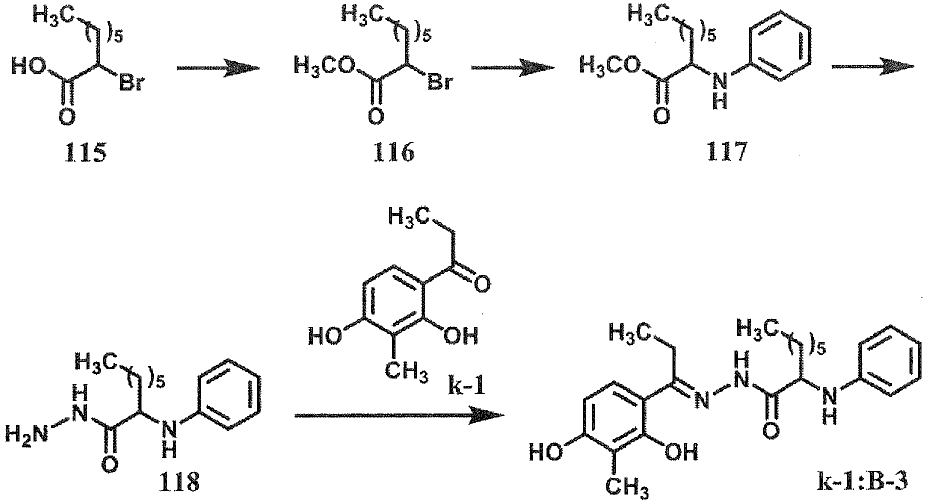

2-Bromo-n-octanoic acid (115) (600 mg, 2.7 mmol) was dissolved in methanol (5.5 mL), concentrated hydrochloric acid (3 drops) was added, and the mixture was stirred at 50°C for 18 hr. The mixture was allowed to cool, diethyl ether (30 mL), saturated aqueous sodium hydrogen carbonate solution (20 mL) were added and the mixture was partitioned. The organic layer was washed with saturated brine (20 mL), dried over anhydrous sodium sulfate, filtered, and concentrated under reduced pressure to give 116 (588 mg, 2.48 mmol, yield 92%) as a yellow liquid.

116 (585 mg, 2.47 mmol) obtained as mentioned above was dissolved in DMSO (5 mL), aniline (0.29 mL, 3.2 mmol), potassium carbonate (0.44 g, 3.2 mmol) were added, and the mixture was stirred at room temperature for 21 hr, and at 60°C for 16 hr. Ethyl acetate (30 mL), water (50 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel chromatography (silica gel 30 g, ethyl acetate/hexane=1/99 - 5/95) to give 117 (277 mg, 1.11 mmol, yield 45%) as a yellow liquid.

117 (256 mg, 1.08 mmol) obtained as mentioned above was dissolved in methanol (2.2 mL), hydrazine monohydrate (0.10 mL, 2.2 mmol) was added and the mixture was stirred at 50°C for 4 hr. Hydrazine monohydrate (0.50 mL, 10.3 mmol) was added and the mixture was stirred for 20 hr, hydrazine monohydrate (0.25 mL, 5.2 mmol) was added and the mixture was further stirred for 23 hr. The reaction solution was concentrated under reduced pressure, and the obtained residue was purified by moderate-pressure silica gel chromatography (aminesilica gel 10 g, ethyl acetate/methylene chloride=0/100 - 5/95) to give 118 (268 mg, 1.07 mmol, yield 99%) as a light yellow solid.

118 (144 mg, 0.58 mmol) obtained as mentioned above, k-1 (80 mg, 0.44 mmol) were dissolved in DMSO (0.9 mL), and the mixture was stirred at 100°C for 18 hr. The mixture was allowed to cool, and the reaction solution was directly purified by moderate-pressure silica gel column chromatography (silica gel 10 g, ethyl acetate/hexane=5/95 - 75/25), and continuously purified by moderate-pressure silica gel column chromatography (silica gel 10 g, ethyl acetate/methylene chloride=1/99 - 5/95). The obtained solid was washed with methylene chloride/IPE, and dried under reduced pressure to give k-1:B-3 (88.7 mg, 0.216 mmol, yield 49%) as a white solid.

¹H-NMR (400 MHz):δ10.82(s, 1H), 9.72(s, 1H), 7.25(d, J=8.0Hz, 1H), 7.06(t, J=8.0Hz, 2H), 6.65(d, J=8.0Hz, 2H), 6.54(t, J=8.0Hz, 1H), 6.39(d, J=8.0Hz, 1H), 5.87(d, J=8.0Hz, NH), 4.19(m, 1H), 2.81(q, J=8.0Hz, 2H), 1.95(s, 3H), 1.73(q, J=8.0Hz, 2H), 1.33(m, 4H), 1.27(m, 4H), 1.03(t, J=8.0Hz, 3H), 0.86(t, J=8.0Hz, 3H).(one signal of NH was not observed)

### [Synthetic Example 15] Synthesis of k-1:B-5

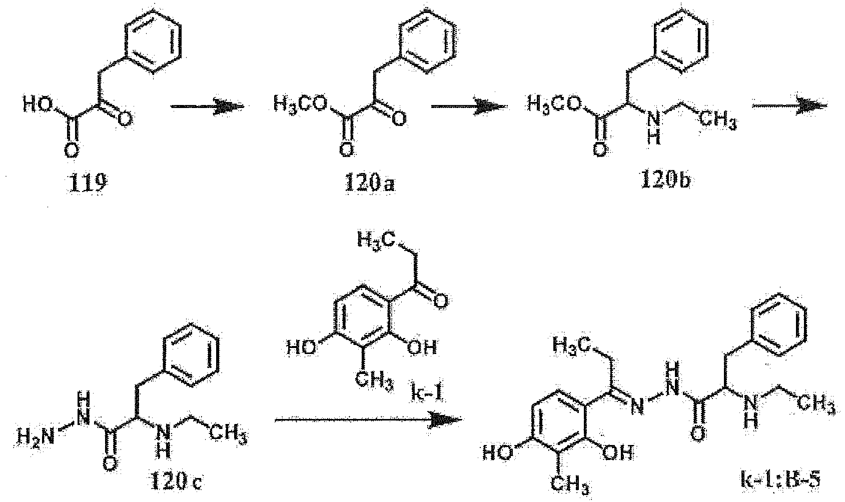

Phenylpyruvic acid (119) (1.0 g, 6.1 mmol) was dissolved in DMF (5 mL), DBU (1.0 mL, 6.7 mmol), methyl iodide (0.42 mL, 6.7 mmol) were added under ice-cooling and the mixture was stirred at room temperature for 3 hr. Ethyl acetate (30 mL), 1 M hydrochloric acid (50 mL) were added and the mixture was partitioned. The organic layer was washed with saturated brine (50 mL), dried over anhydrous sodium sulfate, filtered, and concentrated under reduced pressure. The obtained residue was purified by moderate-pressure silica gel chromatography (silica gel 30 g, ethyl acetate/hexane=0/100 - 13/87) to give 120a (298 mg, 1.67 mmol, yield 27%) as a white solid.

120a (295 mg, 1.66 mmol) obtained as mentioned above was dissolved in methanol (6.6 mL), acetic acid (0.66 mL), 2 M ethylamine THF solution (0.87 mL, 1.7 mmol), picoline borane (0.35 g, 3.3 mmol) was added under ice-cooling and the mixture was stirred at room temperature for 2.5 hr. 4 M hydrochloric acid (3 mL) was added and the mixture was heated at 50°C for 30 min, and allowed to cool. Ethyl acetate (20 mL), saturated aqueous sodium hydrogen carbonate solution (50 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel chromatography (silica gel 10 g, ethyl acetate/hexane=2/98 - 30/70) to give 120b (48.9 mg, 0.236 mmol, yield 14%).

120b (45 mg, 0.22 mmol) obtained as mentioned above was dissolved in methanol (0.9 mL), hydrazine monohydrate (0.021 mL, 0.43 mmol) was added and the mixture was stirred at 50°C for 3 hr. Methanol (0.9 mL), hydrazine monohydrate (0.021 mL, 0.43 mmol) were added and the mixture was stirred for 12 hr, methanol (0.9 mL), hydrazine monohydrate (0.021 mL, 0.43 mmol) were added and the mixture was further stirred for 3 hr. The reaction solution was concentrated under reduced pressure, and the obtained residue was purified by moderate-pressure silica gel chromatography (aminesilica gel 10 g, ethyl acetate/methylene chloride=0/100 - 20/80) to give 120c (32 mg, 0.15 mmol, yield 68%) as a light yellow liquid.

120c (30 mg, 0.14 mmol) obtained as mentioned above, k-1 (25 mg, 0.14 mmol) were dissolved in DMSO (0.3 mL), and the mixture was stirred at 100°C for 18 hr. The mixture was allowed to cool, and the reaction solution was directly purified by moderate-pressure silica gel column chromatography (silica gel 10 g, ethyl acetate/methylene chloride=10/90 - 60/40). The obtained solid was washed with IPE, and dried under reduced pressure to give k-1:B-5 (18.4 mg, 0.0498 mmol, yield 36%) as a white solid.
¹H-NMR (400 MHz) :δ9.72(s, 1H), 7.30-7.10(m, 6H), 6.38(d, J=8.0Hz, 1H), 3.62(m, J=8.0Hz, 1H), 2.86(q, J=8.0Hz, 2H), 2.66(q, J=8.0Hz, 2H), 1.97(s, 3H), 1.04(d, J=8.0Hz, 2H), 0.98(t, J=8.0Hz, 3H), 0.92(t, J=8.0Hz, 3H).(two signals of NH and one signal of OH were not observed)

### [Synthetic Example 16] Synthesis of k-1:C-1

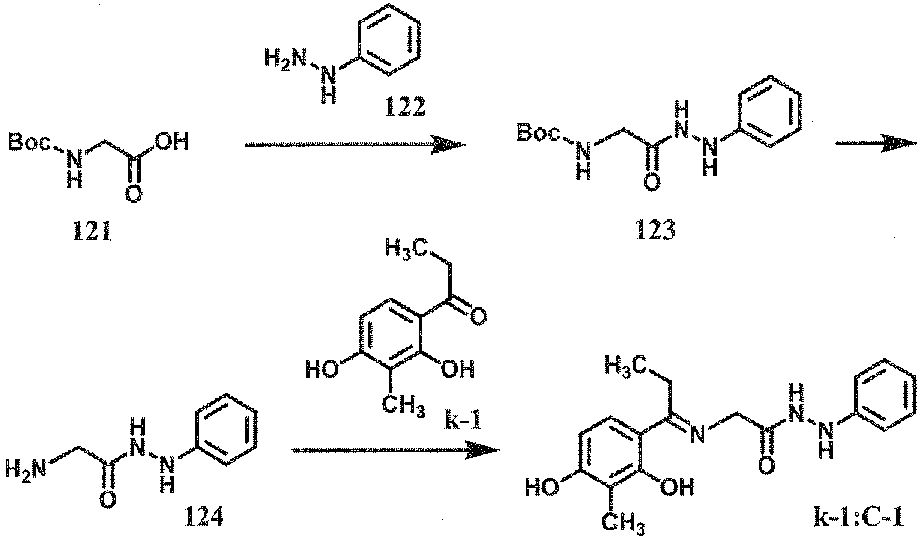

Boc-Gly-OH (121) (0.70 g, 4.0 mmol) was dissolved in methylene chloride (13 mL), WSC (0.84 g, 4.4 mmol), phenylhydrazine (122) (0.47 mL, 4.8 mmol) were added and the mixture was stirred at room temperature for 4 hr. Water (20 mL) was added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 30 g, ethyl acetate/hexane=15/85 - 65/35) to give 123 (853 mg, 3.22 mmol, yield 81%) as a colorless amorphous form.

To 123 (0.64 g, 2.4 mmol) obtained as mentioned above was added 4 M hydrochloric acid/dioxane (6 mL) and the mixture was stirred at room temperature for 3 hr. The precipitated solid was collected by filtration and purified by moderate-pressure silica gel column chromatography (aminesilica gel 30 g, methanol/methylene chloride=0/100 - 8/92) to give 124 (290 mg, 1.76 mmol, yield 73%) as a light yellow solid.

124 (120 mg, 0.72 mmol) obtained as mentioned above, k-1 (100 mg, 0.55 mmol) were dissolved in DMSO (1.1 mL), and the mixture was stirred at 100°C for 22 hr. The mixture was allowed to cool, and the reaction solution was directly purified by moderate-pressure silica gel column chromatography (silica gel 10 g, ethyl acetate/hexane=20/80 - 90/10). The obtained solid was washed with methylene chloride, and dried under reduced pressure to give k-1:C-1 (20.2 mg, 0.0617 mmol, yield 11%) as a yellow solid.
¹H-NMR (400 MHz):δ9.89 (s, 1H), 9.67(s, 1H), 7.83(s, 1H), 7.25(d, J=8.0Hz, 1H), 7.14(t, J=8.0Hz, 2H), 6.77(d, J=8.0Hz, 2H), 6.71(t, J=8.0Hz, 1H), 6.29(d, J=8.0Hz, 1H), 4.35(s, 2H), 2.74(q, J=8.0Hz, 2H), 1.92(s, 3H), 1.13(t, J=8.0Hz, 3H).(one signal of NH was not observed)

### [Synthetic Example 17] Synthesis of k-1:C-2

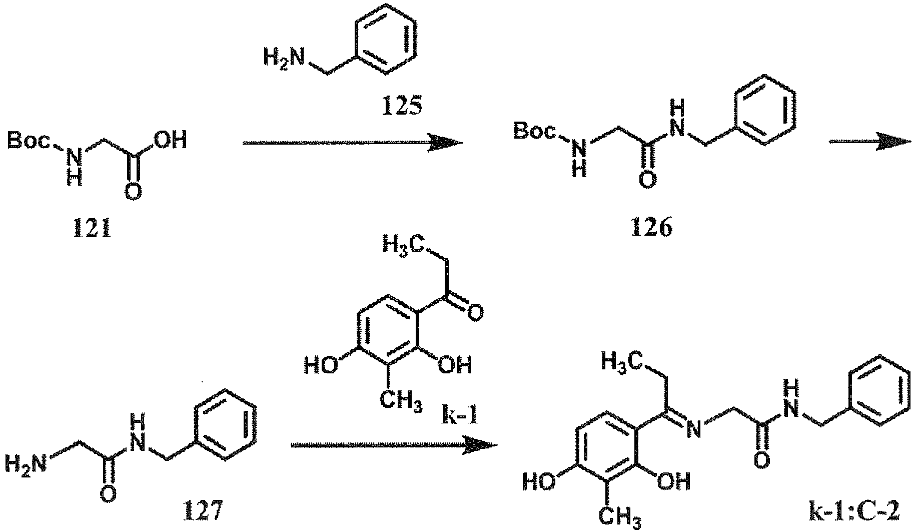

Boc-Gly-OH (121) (0.70 g, 4.0 mmol) was dissolved in methylene chloride (13 mL), WSC (0.84 g, 4.4 mmol), benzylamine (125) (0.52 mL, 4.8 mmol) were added and the mixture was stirred at room temperature for 3 hr. Water (20 mL), methylene chloride (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 30 g, ethyl acetate/hexane=10/90 - 65/35) to give 126 (937 mg, 3.54 mmol, yield 89%) as a colorless liquid.

To 126 (0.93 g, 3.5 mmol) obtained as mentioned above was added TFA (7 mL) and the mixture was stirred at room temperature for 3.5 hr. The reaction solution was concentrated under reduced pressure. The obtained residue was purified by moderate-pressure silica gel column chromatography (aminesilica gel 30 g, ethyl acetate/methylene chloride=10/90 - 95/5) to give 127 (457 mg, 2.78 mmol, yield 79%) as a light yellow liquid.

127 (120 mg, 0.72 mmol) obtained as mentioned above, k-1 (100 mg, 0.55 mmol) were dissolved in DMSO (1.1 mL), and the mixture was stirred at 100°C for 22 hr. The mixture was allowed to cool, and the reaction solution was directly purified by moderate-pressure silica gel column chromatography (silica gel 10 g, ethyl acetate/hexane=10/90 - 80/20). The obtained solid was washed with IPE, methylene chloride, and dried under reduced pressure to give k-1:C-2 (29.9 mg, 0.0908 mmol, yield 17%) as a yellow solid.
¹H-NMR (400 MHz):δ9.63 (s, 1H), 8.55(m, NH), 7.40-7.20(m, 6H), 6.25(d, J=8.0Hz, 1H), 4.32(d, J=8.0Hz, 2H), 4.29(s, 2H), 2.70(q, J=8.0Hz, 2H), 1.91(s, 3H), 1.09(t, J=8.0Hz, 3H).(one signal of OH was not observed)

### [Synthetic Example 18] Synthesis of k-1:D-1

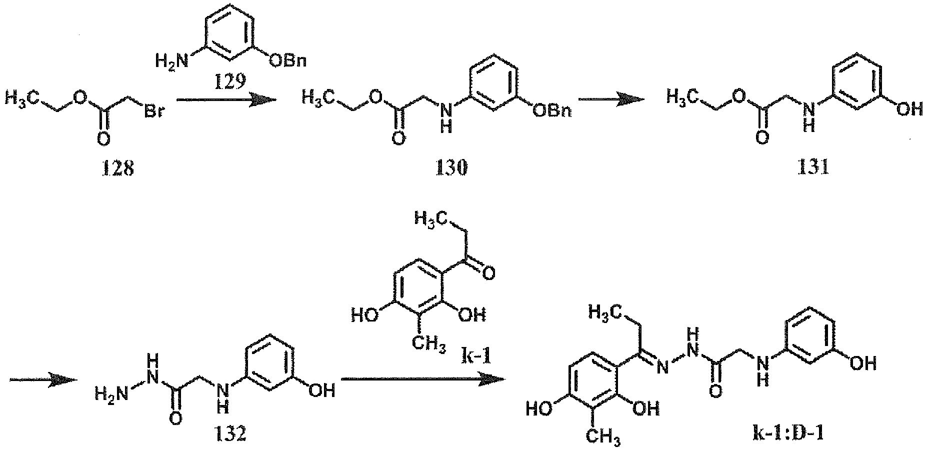

3-Benzyloxyaniline (129) (2.44 g, 12.2 mmol) was dissolved in DMF (24 mL), sodium acetate (1.10 g, 13.5 mmol), ethyl bromoacetate (128) (1.49 mL, 13.5 mmol) were added and the mixture was stirred at room temperature for 4 hr. Water (300 mL), ethyl acetate (150 mL) were added and the mixture was partitioned. The organic layer was washed with saturated brine (100 mL), dried over anhydrous sodium sulfate, filtered, and concentrated under reduced pressure. The obtained residue was purified by moderate-pressure silica gel column chromatography (silica gel 50 g, ethyl acetate/hexane=2/98 - 10/90) to give 130 (2.82 g, 9.88 mmol, yield 81%) as a light yellow solid.

130 (1.0 g, 3.5 mmol) obtained as mentioned above was suspended in methanol (18 mL), 10% Pd/C (0.1 g) was added and the mixture was stirred under a hydrogen atmosphere at room temperature for 5 hr. The reaction solution was filtered through celite, and the filtrate was concentrated under reduced pressure. The obtained residue was purified by moderate-pressure silica gel column chromatography (silica gel 30 g, ethyl acetate/hexane=3/97 - 35/65) to give 131 (295 mg, 1.51 mmol, yield 43%) as a light yellow liquid.

131 (0.29 g, 1.5 mmol) obtained as mentioned above was dissolved in ethanol (3.5 mL), hydrazine monohydrate (0.32 mL, 6.5 mmol) was added, and the mixture was stirred at 60°C for 18 hr. The reaction solution was concentrated under reduced pressure, and the obtained residue was purified by moderate-pressure silica gel chromatography (aminesilica gel 10 g, methanol/methylene chloride=1/99 - 10/90). The obtained solid was washed with IPE, and dried under reduced pressure to give 132 (239 mg, 1.32 mmol, yield 88%) as a light yellow solid.

132 (130 mg, 0.72 mmol) obtained as mentioned above, k-1 (100 mg, 0.55 mmol) were dissolved in DMSO (1.1 mL), and the mixture was stirred at 100°C for 21 hr. The mixture was allowed to cool, and the reaction solution was directly purified by moderate-pressure silica gel column chromatography (silica gel 10 g, ethyl acetate/methylene chloride=5/95 - 50/50). The obtained solid was washed with water and IPE, and dried under reduced pressure to give k-1:D-1 (95.9 mg, 0.279 mmol, yield 51%) as a white solid.
¹H-NMR (270 MHz); δ 13.66(s, 1H), 10.76(s, 1H), 9.70(s, 1H), 8.98(s, 1H), 7.25(d, J=10.8Hz, 1H), 6.86(t, J=10.8Hz, 1H), 6.40(d, J=8.1Hz, 1H), 6.20-5.95(m, 3H), 5.87(t, J=5.4Hz, NH), 3.88(d, J=5.4Hz, 2H), 2.78(q, J=8.0Hz, 2H), 1.97(s, 3H), 1.05(t, J=8.1Hz, 3H).

### [Synthetic Example 19] Synthesis of k-1:D-3

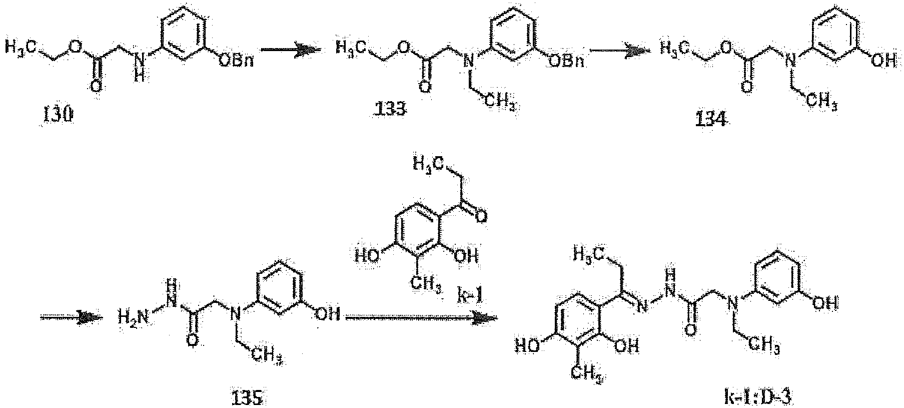

130 (1.2 g, 4.2 mmol) obtained in the step of Synthetic Example 18 was dissolved in DMF (12 mL), potassium carbonate (1.16 g, 8.4 mmol), iodoethane (1.35 mL, 16.8 mmol) were added and the mixture was stirred at 100°C for 3 hr. Iodoethane (0.7 mL, 8.7 mmol) was added and the mixture was stirred for 1.5 hr, and at room temperature for 20 hr. Water (100 mL), ethyl acetate (50 mL) were added and the mixture was partitioned. The organic layer was washed with saturated brine (50 mL), dried over anhydrous sodium sulfate, filtered, and concentrated under reduced pressure. The obtained residue was purified by moderate-pressure silica gel column chromatography (silica gel 10 g, ethyl acetate/hexane=2/98 - 10/90) to give 133 (1.21 g, 3.86 mmol, yield 92%) as a colorless liquid.

Compound 133 (1.2 g, 3.9 mmol) obtained as mentioned above was dissolved in methanol (19 mL), 10% Pd/C (0.1 g) was added and the mixture was stirred under a hydrogen atmosphere at room temperature for 15 hr. The reaction solution was filtered through celite, and the filtrate was concentrated under reduced pressure. The obtained residue was purified by moderate-pressure silica gel column chromatography (silica gel 30 g, ethyl acetate/hexane=5/95 - 25/75) to give 134 (194 mg, 0.869 mmol, yield 22%).

134 (0.20 g, 0.90 mmol) obtained as mentioned above was dissolved in ethanol (2.2 mL), hydrazine monohydrate (0.22 mL, 4.5 mmol) was added and the mixture was stirred at 50°C for 18 hr, hydrazine monohydrate (0.22 mL, 4.5 mmol) was added and the mixture was further stirred at 60°C for 24 hr. The reaction solution was concentrated under reduced pressure, and the obtained residue was purified by moderate-pressure silica gel chromatography (aminesilica gel 10 g, methanol/methylene chloride=1/99 - 6/94). The obtained solid was washed with IPE, and dried under reduced pressure to give 135 (151 mg, 0.722 mmol, yield 80%) as a light yellow solid.

135 (150 mg, 0.72 mmol) obtained as mentioned above, k-1 (100 mg, 0.55 mmol) were dissolved in DMSO (1.1 mL), and the mixture was stirred at 100°C for 23 hr. The mixture was allowed to cool, and the reaction solution was directly purified by moderate-pressure silica gel column chromatography (silica gel 10 g, ethyl acetate/hexane=15/85 - 80/20). The obtained solid was washed with a mixed solvent of IPE/methylene chloride (1/1), and dried under reduced pressure to give k-1:D-3 (118 mg, 0.318 mmol, yield 58%) as a yellow solid.
¹H-NMR (270 MHz); δ 13.68(s, 1H), 10.78(s, 1H), 9.69(s, 1H), 9.00(s, 1H), 7.26(d, J=8.1Hz, 1H), 6.91(t, J=8.1Hz, 1H), 6.40(d, J=8.1Hz, 1H), 6.11(d, J=8.1Hz, 1H), 6.10-6.05(m, 2H), 4.12(s, 2H), 3.42(q, J=8.1Hz, 2H), 2.79(q, J=8.1Hz, 2H), 1.96(s, 3H), 1.13(t, J=8.1Hz, 3H), 1.05(t, J=8.1Hz, 3H).

### [Synthetic Example 20] Synthesis of k-1:E-1

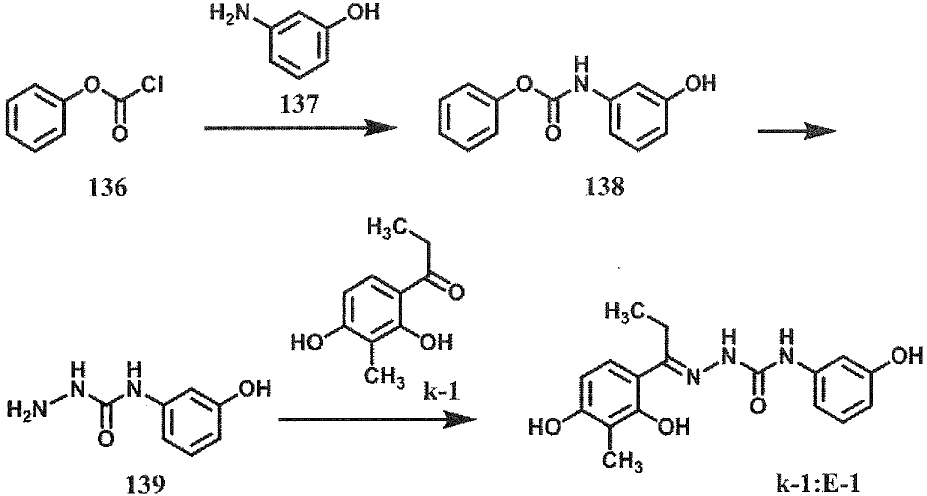

Aminophenol (137) (500 mg, 4.6 mmol) was dissolved in THF (5 mL), water (5 mL), sodium hydrogen carbonate (0.77 g, 9.2 mmol) was added and phenyl chloroformate (136) (0.61 mL, 4.8 mmol) was slowly added dropwise under ice-cooling. The mixture was stirred at the same temperature for 2 hr, ethyl acetate (20 mL), 2 M hydrochloric acid (20 mL) were added and the mixture was partitioned. The organic layer was washed with saturated brine (20 mL), dried over anhydrous sodium sulfate, filtered, and concentrated under reduced pressure. The obtained solid was washed with IPE, and dried under reduced pressure to give 138 (0.94 g, 4.1 mmol, yield 89%) as a white solid.

138 (0.94 g, 4.1 mmol) obtained as mentioned above was dissolved in acetonitrile (4 mL), hydrazine monohydrate (1.0 mL, 21 mmol) was added, and the mixture was stirred at 60°C for 23 hr. The reaction solution was concentrated under reduced pressure, and the obtained residue was purified by moderate-pressure silica gel chromatography (aminesilica gel 30 g, methanol/methylene chloride=0/100 - 12/88) to give 139 (664 mg, 3.97 mmol, yield 97%) as a light yellow solid.

139 (120 mg, 0.72 mmol) obtained as mentioned above, k-1 (100 mg, 0.55 mmol) were dissolved in DMSO (1.1 mL), and the mixture was stirred at 100°C for 16 hr. The mixture was allowed to cool, and the reaction solution was directly purified by moderate-pressure silica gel column chromatography (silica gel 10 g, ethyl acetate/hexane=10/90 - 80/20), and continuously by moderate-pressure silica gel column chromatography (silica gel 10 g, methanol/methylene chloride=1/99 - 5/95). The obtained solid was washed with IPE to give k-1:E-1 (19.7 mg, 0.0598 mmol, yield 11%) as a white solid.
¹H-NMR (400 MHz); δ 9.70(s, 1H), 9.60(s, 1H), 9.38(s, 1H), 8.73(s, 1H), 7.21(d, J=8.0Hz, 1H), 7.06(t, J=8.0Hz, 1H), 7.05(s, 1H), 6.82(d, J=8.0Hz, 1H), 6.39(d, J=8.0Hz, 2H), 5.76(s, 1H), 2.69(q, J=8.0Hz, 2H), 1.99(s, 3H), 1.13(t, J=8.0Hz, 3H).

### [Synthetic Example 21] Synthesis of k-1:F-1

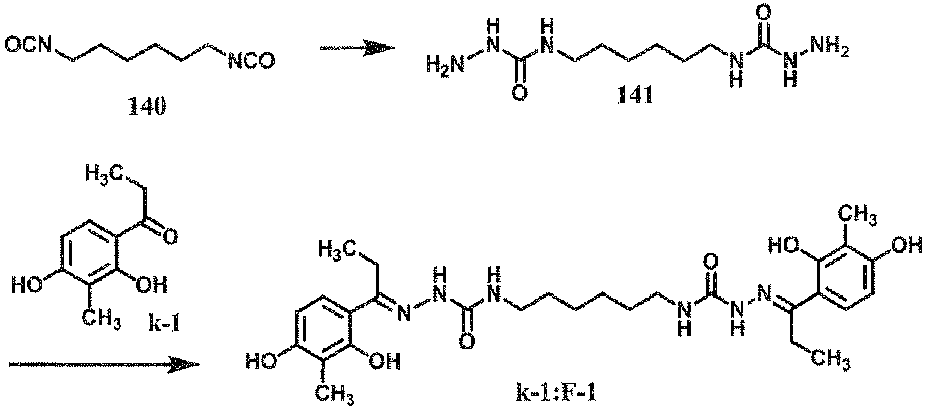

Hydrazine monohydrate (0.58 mL, 12 mmol) was dissolved in methylene chloride (6 mL), hexamethylene diisocyanate (140) (0.48 mL, 3.0 mmol) was added under ice-cooling and the mixture was stirred at room temperature for 1.5 hr. Hydrazine monohydrate (0.29 mL, 6.0 mmol) was added and the mixture was stirred for 2 hr. The resulting solid was collected by filtration, and dried under reduced pressure to give 141 (0.60 g, 2.6 mmol, yield 87%) as a white solid.

141 (100 mg, 0.43 mmol) obtained as mentioned above, k-1 (233 mg, 1.29 mmol) were suspended in DMSO (1.4 mL) and the mixture was stirred at 100°C for 20 hr. The mixture was allowed to cool, methylene chloride was added, an insoluble material was filtered off, and the filtrate was concentrated under reduced pressure. The obtained solid was washed with methylene chloride and water, and dried under reduced pressure to give k-1:F-1 (88.6 mg, 0.159 mmol, yield 37%) as a light yellow solid.
¹H-NMR (400 MHz); δ 9.44(s,4H), 7.15(d, J=8.0Hz, 2H), 6.40-6.30(m, 2H), 3.13(q, J=8.0Hz, 4H), 2.63 (q, J=8.0Hz, 4H),1.97(s, 6H), 1.47(m, 4H), 1.38(m, 4H),1.07(t, J=8.0Hz, 6H).

### [Synthetic Example 22] Synthesis of k-1:G-1

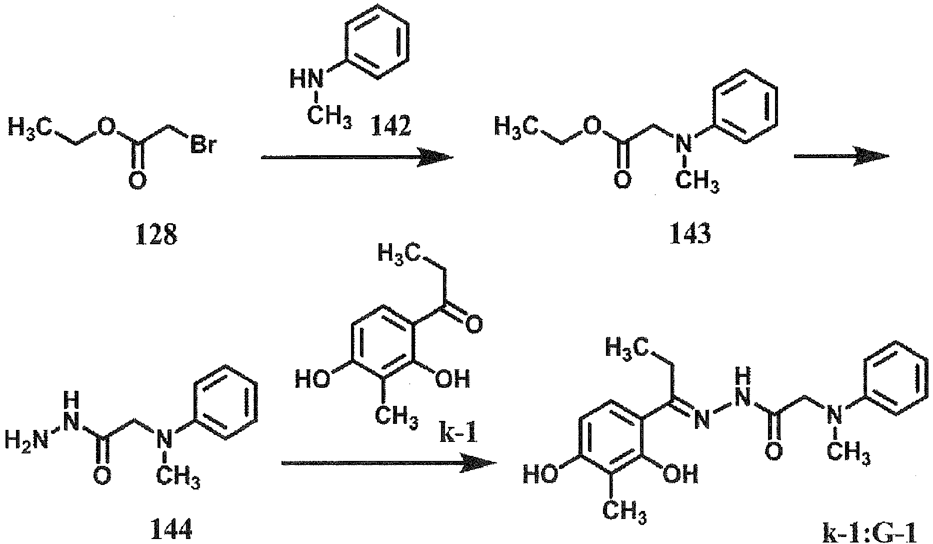

N-methyl-aniline (142) (0.50 g, 4.7 mmol) was dissolved in ethanol (9 mL), potassium carbonate (0.97 g, 7.0 mmol), ethyl bromoacetate (128) (0.57 mL, 5.1 mmol) were added, and the mixture was stirred at 60°C for 21 hr. Insoluble materials were filtered off, and the filtrate was concentrated under reduced pressure. To the obtained residue were added ethyl acetate (30 mL), water (30 mL) and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=0/100 - 5/95) to give 143 (496 mg, 2.57 mmol, yield 55%) as a light yellow liquid.

143 (0.49 g, 2.6 mmol) obtained as mentioned above was dissolved in methanol (5 mL), hydrazine monohydrate (0.25 mL, 5.1 mmol) was added, and the mixture was stirred at 55°C for 15 hr. Hydrazine monohydrate (0.50 mL, 10 mmol) was added and the mixture was further stirred at 60°C for 18 hr. The reaction solution was concentrated under reduced pressure, and the obtained solid was washed with IPE, and dried under reduced pressure to give 144 (389 mg, 2.17 mmol, yield 83%) as a white solid.

144 (130 mg, 0.72 mmol) obtained as mentioned above, k-1 (100 mg, 0.55 mmol) were dissolved in DMSO (1.1 mL), and the mixture was stirred at 100°C for 17 hr. The mixture was allowed to cool, and the reaction solution was directly purified by moderate-pressure silica gel column chromatography (silica gel 10 g, ethyl acetate/hexane=10/90 - 80/20). The obtained solid was washed with IPE, and dried under reduced pressure to give k-1:G-1 (92 mg, 0.27 mmol, yield 49%) as a white solid.
¹H-NMR (270 MHz); δ 13.64(s, 1H), 10.86(s, 1H), 9.67(s, 1H), 7.24(d, J=10.8Hz, 1H), 7.15(t, J=10.8Hz, 2H), 6.71(d, J=8.1Hz, 2H), 6.62(t, J=8.1Hz, 1H), 6.38(d, J=8.1Hz, 1H), 4.23(s, 2H), 3.04 (s, 3H), 2.89(q, J=8.1Hz, 2H), 1.93(s, 3H), 1.05(t, J=8.1Hz, 3H).

### [Synthetic Example 23] Synthesis of k-1:L-1

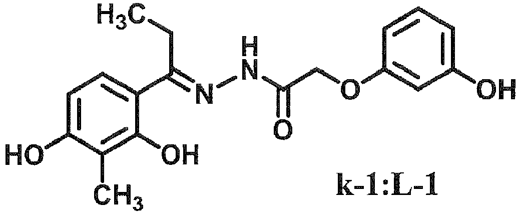

k-1:L-1 (65 mg, white solid) was synthesized by a method according to the above-mentioned Synthetic Example 22, and using resorcinol as a starting material.
¹H-NMR (270 MHz); δ 13.63(s, 1H), 10.99(s, 1H), 9.74(s, 1H), 9.46(s, 1H), 7.28(d, J=8.1Hz, 1H), 7.07(t, J=8.1Hz, 1H), 6.45-6.35(m, 4H), 4.72(s, 2H), 2.81(q, J=8.1Hz, 2H), 1.97(s, 3H), 1.08 (t, J=8.1Hz, 3H).

### [Synthetic Example 24] Synthesis of k-1:M-1

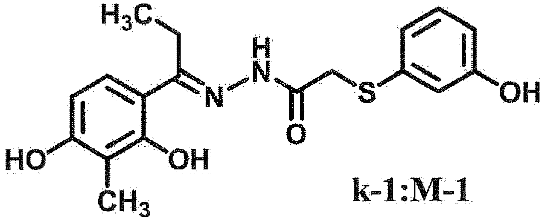

k-1:M-1 (119 mg, white solid) was synthesized by a method according to the above-mentioned Synthetic Example 22, and using 3-mercaptophenol as a starting material.
¹H-NMR (270 MHz); δ13.64(s, 1H), 10.96(s, 1H), 9.72(s, 1H), 9.57(s, 1H), 7.26(d, J=8.1Hz, 1H), 7.11(t, J=8.1Hz, 1H), 6.82(d, J=8.1Hz, 1H), 6.80 (s, 1H), 6.62(d, J=8.1Hz, 1H), 6.40(d, J=8.1Hz, 1H), 3.88(s, 2H), 2.78(q, J=8.1Hz, 2H), 1.97(s, 3H), 1.06(t, J=8.1Hz, 3H).

### [Synthetic Example 25] Synthesis of k-1:G-2

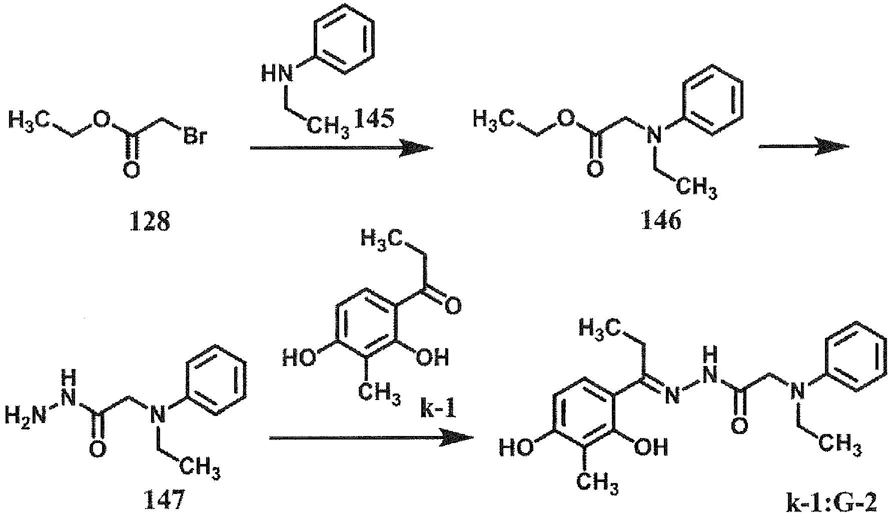

N-ethyl-aniline (145) (0.50 g, 4.1 mmol) was dissolved in ethanol (8 mL), potassium carbonate (0.86 g, 6.2 mmol), ethyl bromoacetate (128) (0.50 mL, 4.1 mmol) were added, and the mixture was stirred at 60°C for 21 hr. Insoluble materials were filtered off, and the filtrate was concentrated under reduced pressure. To the obtained residue were added ethyl acetate (30 mL), water (30 mL) and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=0/100 - 5/95) to give 146 (473 mg, 2.28 mmol, yield 56%) as a light yellow liquid.

146 (0.47 g, 2.3 mmol) obtained as mentioned above was dissolved in methanol (4.5 mL), hydrazine monohydrate (0.22 mL, 4.5 mmol) was added, and the mixture was stirred at 55°C for 15 hr. Hydrazine monohydrate (0.44 mL, 9.1 mmol) was added and the mixture was further stirred at 60°C for 18 hr. The reaction solution was concentrated under reduced pressure, and the obtained solid was washed with IPE, and dried under reduced pressure to give 147 (309 mg, 1.60 mmol, yield 70%) as a white solid.

147 (140 mg, 0.72 mmol) obtained as mentioned above, k-1 (100 mg, 0.55 mmol) were dissolved in DMSO (1.1 mL), and the mixture was stirred at 100°C for 17 hr. The mixture was allowed to cool, and the reaction solution was directly purified by moderate-pressure silica gel column chromatography (silica gel 10 g, ethyl acetate/hexane=10/90 - 80/20). The obtained solid was washed with IPE, and dried under reduced pressure to give k-1:G-2 (96 mg, 0.27 mmol, yield 49%) as a white solid.
¹H-NMR (270 MHz); δ 13.66(s, 1H), 10.82(s, 1H), 9.68(s, 1H), 7.25(d, J=8.1Hz, 1H), 7.13(t, J=8.1Hz, 2H), 6.65(d, J=8.1Hz, 2H), 6.59(t, J=8.1Hz, 1H), 6.38(d, J=10.8Hz, 1H), 4.17(s, 2H),3.46(q, J=8.1Hz, 2H), 2.79(q, J=8.1Hz, 2H), 1.94(s, 3H), 1.13(t, J=8.1Hz, 3H), 1.03(t, J=8.1Hz, 3H).

### [Synthetic Example 26] Synthesis of k-1:J-1

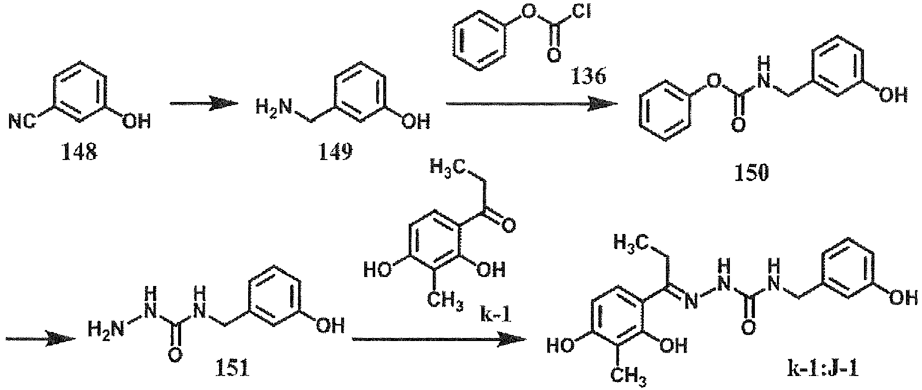

To ice-cooled THF (10 mL) were successively added hydrogenated aluminum (1.0 g, 26 mmol), 3-cyanophenol (148) (0.63 g, 5.3 mmol), and the mixture was stirred at room temperature for 1.5 hr, and at 60°C for 3.5 hr. The mixture was allowed to cool, hydrogenated aluminum (1.0 g, 26 mmol), THF (10 mL) were added and the mixture was further stirred at 60°C for 16 hr. The reaction solution was ice-cooled, water (1.5 mL), 15% aqueous sodium hydroxide solution (1.5 mL), water (4.5 mL) were successively added and the mixture was stirred at room temperature for 3 hr. The suspended solution was filtered through celite, and the filtrate was concentrated under reduced pressure. The obtained residue was purified by moderate-pressure silica gel column chromatography (aminesilica gel 10 g, methanol/methylene chloride=0/100 - 8/92). The obtained solid was washed with IPE, and dried under reduced pressure to give 149 (477 mg, 3.87 mmol, yield 73%) as a white solid.

149 (200 mg, 1.6 mmol) obtained as mentioned above was dissolved in methylene chloride (2 mL), water (2 mL), sodium hydrogen carbonate (0.27 g, 3.2 mmol) was added and phenyl chloroformate (136) (0.22 mL, 1.7 mmol) was slowly added dropwise under ice-cooling. The mixture was stirred at room temperature for 20 hr, ethyl acetate (20 mL), water (20 mL) were added and the mixture was partitioned. The organic layer was washed with saturated brine (20 mL), dried over anhydrous magnesium sulfate, filtered, and concentrated under reduced pressure. The obtained residue was purified by moderate-pressure silica gel column chromatography (silica gel 10 g, ethyl acetate/hexane=5/95 - 35/65) to give 150 (369 mg, 1.52 mmol, yield 95%) as a colorless liquid.

150 (365 mg, 1.50 mmol) obtained as mentioned above was suspended in acetonitrile (3.8 mL), hydrazine monohydrate (0.18 mL, 3.8 mmol) was added and the mixture was stirred at room temperature for 2.5 hr. Hydrazine monohydrate (0.18 mL, 3.8 mmol) was added and the mixture was further stirred at 55°C for 20 hr. The reaction solution was concentrated under reduced pressure, and the obtained solid was washed with IPE/methylene chloride (3/1), and dried under reduced pressure to give 151 (233 mg, 1.29 mmol, yield 86%) as a white solid.

151 (130 mg, 0.72 mmol) obtained as mentioned above, k-1 (100 mg, 0.55 mmol) were dissolved in DMSO (1.1 mL), and the mixture was stirred at 100°C for 15 hr. The mixture was allowed to cool, and the reaction solution was directly purified by moderate-pressure silica gel column chromatography (silica gel 10 g, ethyl acetate/methylene chloride=10/90 - 55/45). To the obtained purified product was added water and the precipitated solid was collected by filtration and dried under reduced pressure to give k-1:J-1 (102 mg, 0.297 mmol, yield 54%) as a white solid.
¹H-NMR (270 MHz); δ13.45 (brs, 1H), 9.57(s, 1H), 9.53(s, 1H), 9.36(s, 1H), 7.17(d, J=8.1Hz, 1H), 7.11(d, J=8.1Hz, 1H), 6.80-6.60(m, 4H), 6.37(d, J=8.1Hz, 1H), 4.25(d, J=5.4Hz, 2H), 2.65(q, J=8.1Hz, 2H), 1.97(s, 3H), 1.08(t, J=8.1Hz, 3H).

### [Synthetic Example 27] Synthesis of k-1:J-5

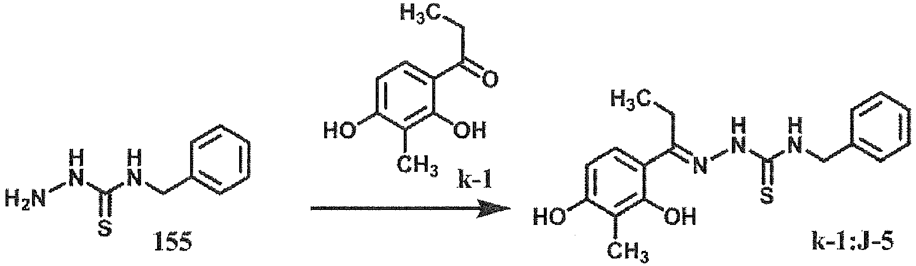

4-Benzyl-3-thiosemicarbazide (155) (130 mg, 0.72 mmol), k-1 (100 mg, 0.55 mmol) were dissolved in DMSO (1.1 mL), and the mixture was stirred at 100°C for 17 hr. The mixture was allowed to cool, and the reaction solution was directly purified by moderate-pressure silica gel column chromatography (silica gel 10 g, ethyl acetate/hexane=5/95 - 80/20), and continuously by moderate-pressure silica gel column chromatography (silica gel 10 g, ethyl acetate/hexane=10/90 - 50/50). The obtained solid was washed with IPE, and dried under reduced pressure to give k-1:J-5 (49.9 mg, 0.145 mmol, yield 26%) as a white solid.
¹H-NMR (400 MHz); δ10.61(brs, 1H), 9.69(s, 1H), 8.43 (brs, 1H), 7.40-7.20(m, 6H), 6.40 (d, J=8.0Hz, 1H), 4.78(d, J=8.0Hz, 2H), 2.74(q, J=8.1Hz, 2H), 1.99(s, 3H), 1.09(t, J=8.1Hz, 3H). (one signal of NH was not observed)

### [Synthetic Example 28] Synthesis of k-1:J-6

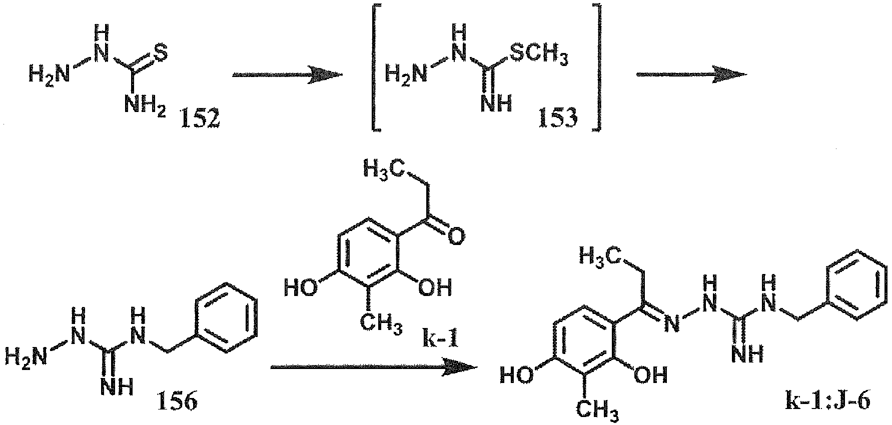

Thiosemicarbazide (152) (0.50 g, 5.5 mmol) was suspended in methanol (5.5 mL), methyl iodide (0.41 mL, 6.6 mmol) was added and the mixture was stirred at 60°C for 1.5 hr, and at 70°C for 50 min with the lid of the reaction container opened. The mixture was allowed to cool, benzylamine (0.61 mL, 5.5 mmol) was added, and the mixture was stirred at 55°C for 17 hr. The reaction solution was concentrated under reduced pressure, and the obtained residue was washed with a mixed solvent of IPE/methylene chloride (1/4). The obtained crude purified product was purified by moderate-pressure silica gel column chromatography (aminesilica gel 30 g, methanol/methylene chloride=0/100 - 15/85), and the obtained solid was washed with methylene chloride and dried under reduced pressure to give 156 (626 mg, 3.81 mmol, yield 69%) as an orange solid.

156 (120 mg, 0.72 mmol) obtained as mentioned above, k-1 (100 mg, 0.55 mmol) were dissolved in DMSO (1.1 mL), and the mixture was stirred at 100°C for 27 hr. The mixture was allowed to cool, and the reaction solution was directly purified by moderate-pressure silica gel column chromatography (silica gel 10 g, ethyl acetate/methylene chloride=10/90 - 80/20). To the obtained crude purified product were added methylene chloride, water and the mixture was partitioned. The organic layer was dried over anhydrous magnesium sulfate, filtered, and concentrated under reduced pressure. The obtained residue was purified by moderate-pressure silica gel column chromatography (silica gel 10 g, ethyl acetate/hexane=10/90 - 60/40) to give k-1:J-6 (29.9 mg, 0.0916 mmol, yield 17%) as a yellow amorphous form.
¹H-NMR (270 MHz); δ14.51 (brs, 1H), 9.39(s, 1H), 7.36-7.05 (m, 6H), 6.32(d, J=8.1Hz, 1H), 6.20 (m, NH), 5.30 (brs, NH), 4.34(d, J=5.4Hz, 2H), 2.84(q, J=8.1Hz, 2H), 1.96(s, 3H), 0.96(t, J=8.1Hz, 3H).

### [Synthetic Example 29] Synthesis of k-1:N-1

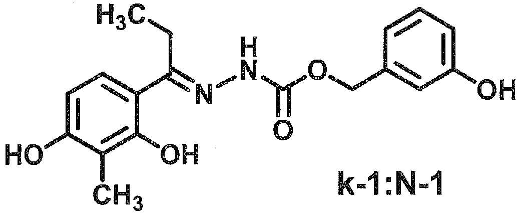

2-Hydroxybenzyl alcohol (1.0 g, 8.1 mmol) was suspended in methylene chloride (10 mL), water (10 mL) and sodium hydrogen carbonate (2.0 g, 24 mmol) was added, and phenyl chloroformate (2.0 mL, 16 mmol) was slowly added dropwise under ice-cooling. The temperature was gradually rose to room temperature and the mixture was stirred for 5.5 hr. Methylene chloride (20 mL), water (20 mL) were added and the mixture was partitioned. The organic layer was washed with saturated brine (30 mL), dried over anhydrous magnesium sulfate, filtered, and concentrated under reduced pressure. The obtained residue was purified by moderate-pressure silica gel column chromatography (silica gel 30 g, ethyl acetate/hexane=2/98 - 35/65) to give 3-(hydroxymethyl)phenyl phenyl carbonate (1.45 g, 5.94 mmol, yield 73%) as a white solid.

3-(Hydroxymethyl)phenyl phenyl carbonate obtained as mentioned above was dissolved in methylene chloride (7 mL), pyridine (0.72 mL, 8.9 mmol) and a small amount of DMAP were added, and phenyl chloroformate (0.90 mL, 7.1 mmol) was slowly added dropwise under ice-cooling. The mixture was stirred at room temperature for 2 hr, methylene chloride (10 mL), water (30 mL) were added and the mixture was partitioned. The organic layer was washed with saturated brine (30 mL), dried over anhydrous magnesium sulfate, filtered, and concentrated under reduced pressure. The obtained residue was purified by moderate-pressure silica gel column chromatography (silica gel 30 g, ethyl acetate/hexane=3/97 - 25/75) to give 3-[(phenoxycarbonyl)oxy]benzyl phenyl carbonate (2.20 g, 6.04 mmol, yield 102%) as a colorless liquid.

3-[(Phenoxycarbonyl)oxy]benzyl phenyl carbonate (2.2 g, 6.0 mmol) obtained as mentioned above was suspended in ethanol (10 mL), hydrazine monohydrate (2.9 mL, 60 mmol) was added, and the mixture was stirred at 60°C for 21 hr. Insoluble materials were filtered off, and the filtrate was concentrated under reduced pressure. The obtained residue was .purified by moderate-pressure silica gel column chromatography (silica gel 30 g, methanol/methylene chloride=0/100 - 10/90). The obtained solid was suspension washed with methylene chloride to give 3-hydroxybenzyl hydrazinecarboxylate (808 mg, 4.44 mmol, yield 74%) as a white solid.

3-Hydroxybenzyl hydrazinecarboxylate (130 mg, 0.72 mmol) obtained as mentioned above, 2',4'-dihydroxy-3'-methylpropiophenone (100) (100 mg, 0.55 mmol) were suspended in DMSO (1.1 mL) and the mixture was stirred at 100°C for 22 hr. The mixture was allowed to cool, and the reaction solution was directly purified by moderate-pressure silica gel column chromatography (silica gel 10 g, ethyl acetate/methylene chloride=2/98 - 30/70). To the obtained purified product was added water and the precipitated solid was collected by filtration to give k-1:N-1 (85.5 mg, 0.248 mmol, yield 45%) as a white solid.
¹H-NMR (270 MHz); δ13.45(brs, 1H), 10.78(brs, 1H), 9.63 (s, 1H), 9.49 (s, 1H), 7.21(d, J=8.1Hz, 1H), 7.18(t, J=8.1Hz, 1H), 6.84(d, J=8.1Hz, 1H), 6.83(s, 1H), 6.73(d, J=8.1Hz, 1H), 6.38(d, J=8.1Hz, 1H), 5.14(s, 2H), 2.75(q, J=8.1Hz, 2H), 1.97(s, 3H), 1.03(t, J=8.1Hz, 3H).

### [Synthetic Example 30] Synthesis of GA-005A

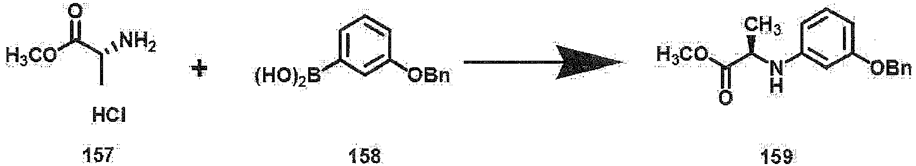

wherein Bn is a benzyl group.

D-alanine methyl ester hydrochloride (157) (1.40 g, 10.0 mmol), 3-benzyloxyphenylboronic acid (158) (3.43 g, 15.1 mmol), copper acetate (II) monohydrate (3.00 g, 15.1 mmol), and molecular sieve 4A (MS4A; 20 g) were dissolved in methylene chloride (200 mL), triethylamine (4.17 mL, 30.1 mmol) was added and the mixture was stirred under oxygen atmosphere at room temperature for 23 hr. The reaction solution was filtered through celite, and the filtrate was concentrated to half under reduced pressure. Saturated aqueous sodium hydrogen carbonate solution (50 mL) was added and the mixture was partitioned. The organic layer was washed with saturated brine (20 mL), dried over anhydrous sodium sulfate, filtered, and concentrated under reduced pressure. The obtained residue was purified by moderate-pressure silica gel chromatography (silica gel 30 g, ethyl acetate/hexane=1/99 - 10/90) to give 159 (595 mg, 2.09 mmol, yield 21%) as a yellow liquid. 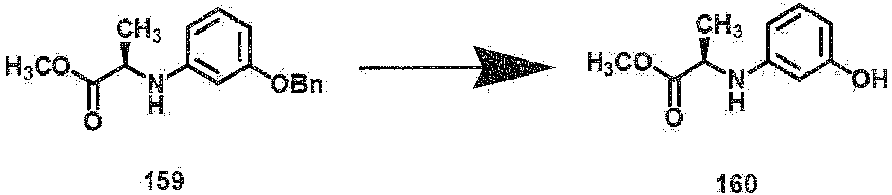 wherein Bn is a benzyl group.

159 (595 mg, 2.09 mmol) obtained as mentioned above was dissolved in methanol (21 mL), palladium carbon ethylenediamine complex (241 mg) was added and the mixture was stirred under hydrogen atmosphere at room temperature for 3.5 hr. Palladium carbon was filtered off and the filtrate was concentrated under reduced pressure. The obtained residue was purified by moderate-pressure silica gel column chromatography (silica gel 30 g, ethyl acetate/hexane=10/90 - 30/70) to give 160 (413 mg, 2.12 mmol, quant.) as a yellow liquid.

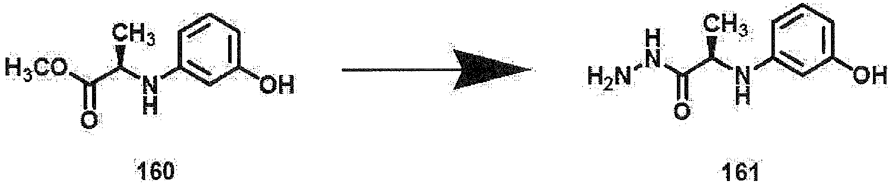

160 (413 mg, 2.12 mmol) obtained as mentioned above was dissolved in methanol (4.2 mL), hydrazine monohydrate (1.0 mL, 21 mmol) was added and the mixture was stirred at 60°C for 3.5 hr. The reaction solution was concentrated under reduced pressure, azeotropically distilled with toluene, and the obtained residue was purified by moderate-pressure silica gel column chromatography (silica gel 10 g, methanol/methylene chloride=1/99 - 10/90) to give 161 (391 mg, 2.00 mmol, yield 95%) as a brown amorphous form.

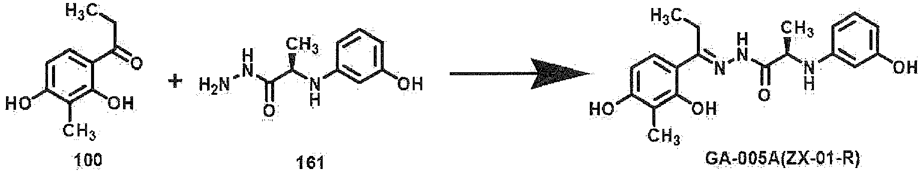

161 (72 mg, 0.37 mmol) obtained as mentioned above and 2',4'-dihydroxy-3'-methylpropiophenone (100) (60 mg, 0.33 mmol) were dissolved in DMSO (0.67 mL), and the mixture was stirred at 100°C for 3 days. The reaction solution was directly purified by moderate-pressure silica gel column chromatography (silica gel 10 g, ethyl acetate/methylene chloride=5/95 - 50/50), and the obtained purified product was successively suspended and washed with distilled water, isopropyl alcohol (IPA)/hexane, distilled water to give GA-005A (ZX-01-R (40.5 mg, 0.113 mmol, yield 34%)) as a pale-brown solid. The specific optical rotation was measured by a polarimeter P-1020 (manufactured by JASCO Corporation).
¹H-NMR (270 MHz); δ 13.65 (s, 1H), 10.75 (s, 1H), 9.70 (s, 1H), 8.97 (s, 1H), 7.25 (d, J=8.1Hz, 1H), 6.83 (t, J=8.1Hz, 1H), 6.39 (d, J=8.1Hz, 1H), 6.15-5.95 (m, 3H), 5.81 (d, J=8.1Hz, 1H), 4.18 (m, 1H), 2.78 (q, J=8.1Hz, 2H), 1.95 (s, 3H), 1.37 (d, J=8.1Hz, 3H), 1.03 (t, J=8.1Hz, 3H). compound GA-005A; optical purity 99.9ee%, purity 99.2%, specific optical rotation [α]²²_{D}-11.4 (c=0.0264, ethanol), steric configuration R form

### [Synthetic Example 31] Synthesis of GA-005B

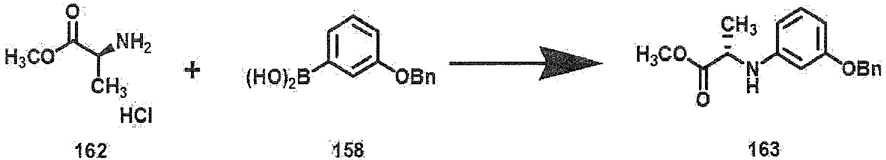

wherein Bn is a benzyl group.

L-alanine methyl ester hydrochloride (162) (700 mg, 5.02 mmol), 3-benzyloxyphenylboronic acid (158) (1.72 g, 7.52 mmol), copper acetate (II) monohydrate (1.50 g, 7.52 mmol), and MS4A (10 g) were dissolved in methylene chloride (100 mL), triethylamine (2.09 mL, 15.1 mmol) was added and the mixture was stirred at room temperature for 18 hr. The reaction solution was filtered through celite, and the filtrate was concentrated to half under reduced pressure. Methylene chloride (25 mL) and saturated aqueous sodium hydrogen carbonate solution (25 mL) were added and the mixture was partitioned. The organic layer was washed with saturated brine (10 mL), dried over anhydrous sodium sulfate, filtered, and concentrated under reduced pressure. The obtained residue was purified by moderate-pressure silica gel column chromatography (silica gel 30 g, ethyl acetate/hexane=1/99 - 10/90) to give 163 (255 mg, 0.894 mmol, yield 18%) as a pale-yellow liquid. 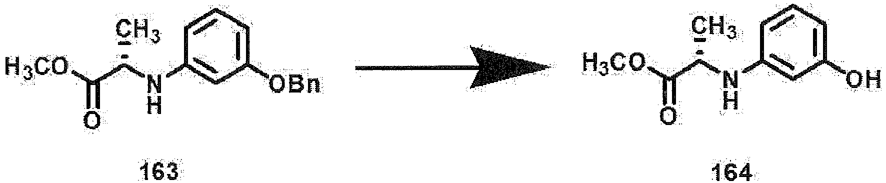 wherein Bn is a benzyl group.

163 (255 mg, 0.894 mmol) obtained as mentioned above was dissolved in methanol (9 mL), 5% palladium carbon (190 mg) was added and the mixture was stirred under hydrogen atmosphere at room temperature for 2 hr. Palladium carbon was filtered off, the filtrate was concentrated under reduced pressure and the obtained residue was purified by moderate-pressure silica gel column chromatography (silica gel 10 g, ethyl acetate/hexane=10/90 - 100/0) to give 164 (92.0 mg, 0.471 mmol, yield 53%) as a pale-brown liquid.

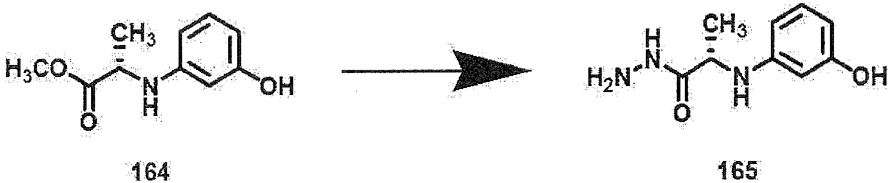

164 (75.5 mg, 0.389 mmol) obtained as mentioned above was dissolved in ethanol (0.8 mL), hydrazine monohydrate (0.19 mL, 3.9 mmol) was added and the mixture was stirred at 60°C for 2 hr. The reaction solution was concentrated under reduced pressure, azeotropically distilled with toluene and the obtained residue was purified by moderate-pressure silica gel column chromatography (silica gel 10 g, methanol/methylene chloride=1/99 - 4/96) to give 165 (62.2 mg, 0.319 mmol, yield 82%) as a colorless amorphous form.

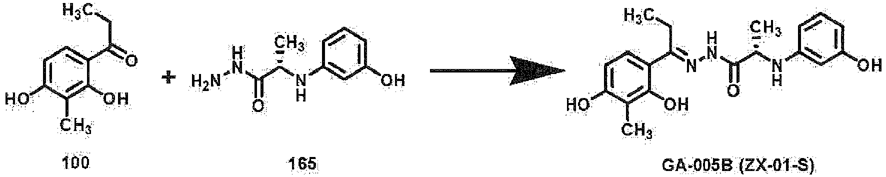

165 (60 mg, 0.31 mmol) obtained as mentioned above, 2',4'-dihydroxy-3'-methylpropiophenone (100) (50 mg, 0.28 mmol) were dissolved in DMSO (0.55 mL) and the mixture was stirred at 100°C for 3 days. The mixture was allowed to cool, and the reaction solution was directly purified by moderate-pressure silica gel column chromatography (silica gel 10 g, ethyl acetate/methylene chloride=3/97 - 50/50). The obtained solid was dissolved in IPA, hexane was added, and the precipitated solid was collected by filtration to give GA-005B (ZX-01-S (30.1 mg, 0.0842 mmol, yield 30%)) as a pale-brown solid. ¹H-NMR (270 MHz); δ 13.64 (s, 1H), 10.73 (s, 1H), 9.69 (s, 1H), 8.96 (s, 1H), 7.24 (d, J=8.1Hz, 1H), 6.82 (t, J=8.1Hz, 1H), 6.38 (d, J=8.1Hz, 1H), 6.15-5.95 (m, 3H), 5.80 (d, J=8.1Hz, 1H), 4.18 (m, 1H), 2.78 (q, J=8.1Hz, 2H), 1.95 (s, 3H), 1.36 (d, J=8.1Hz, 3H), 1.01 (t, J=8.1Hz, 3H).
compound GA-005B; optical purity 99.9ee%, purity 97.8%, specific optical rotation [α]²²_{D} +11.3 (c=0.0230, ethanol), steric configuration S form

### [Example 1] Kinase inhibitory activity evaluation 1

For compound k-1:B-1, inhibitory activity evaluation (calculation of 50% inhibitory concentration (IC₅₀)) was performed on 8 kinds of kinases (CGK2, LATS2, MSK1, p70S6K, PKACα, PKACβ, SGK2, SGK3). The activity was measured using the Off-chip Mobility Shift Assay (MSA method) described below. The compound was added such that the test concentration of k-1:B-1 was in 10 steps from the final concentration of 10 µM to 0.3 nM.

As an analysis method of the data, the mean signal of the control well containing all reaction components was set to 0% inhibition, the mean signal without enzyme addition was set to 100% inhibition, and the inhibition rate was calculated from the mean signal of 2 wells of each test substance test. The IC₅₀ value was obtained by approximating a plot based on the test substance concentration and inhibition rate to a 4 parameter logistics curve by using the Excel Solver add-in and by the non-linear least squares method.

### MSA method:

5 µL of k-1:B-1 solution at 4-fold concentration, 5 µL of substrate/ATP/metal solution at 4-fold concentration, and 10 µL of kinase solution at 2-fold concentration, each prepared with assay buffer (20 mM HEPES, 0.01% Triton X-100, 2 mM DTT, pH 7.5), were mixed in the wells of a polypropylene 384-well plate and reacted at room temperature for 1 or 5 hr. Successively, 70 µL of Termination Buffer (QuickScout Screening Assist MSA; Carna Biosciences) was added to discontinue the reaction. Then, the substrate peptide and the phosphorylated peptide in the reaction solution were separated and quantified by the LabChip system (Perkin Elmer). The kinase reaction was evaluated by the resultant product ratio (P/(P+S)) calculated from the substrate peptide peak height (S) and the phosphorylated peptide peak height (P).

As the result of this test, the IC₅₀ values of k-1:B-1 are shown in Table 5. It was found that k-1:B-1 inhibited CGK2, LATS2, MSK1, p70S6K, PKACα, PKACβ, SGK2, and SGK3 kinases.

**[Table 5]**

| **kinase** | IC50 (nM) |
|---|---|
| CGK2 | 29.9 |
| LATS2 | 5.12 |
| MSK1 | 17.4 |
| p70S6K | 41.5 |
| PKAC*α* | 23.4 |
| PKAC*β* | 29.3 |
| SGK2 | 13.2 |
| SGK3 | 63.3 |

### [Example 2] Kinase inhibitory activity evaluation 2

For compounds k-1:H-7, k-1:H-1, k-1:D-1, k-1:J-1, and k-1:B-1, inhibitory rate at 100 nM was calculated on 8 kinds of kinases (CGK2, LATS2, MSK1, p70S6K, PKACα, PKACβ, SGK2, SGK3). The activity was measured using the MSA method. As an analysis method of the data, the mean signal of the control well containing all reaction components was set to 0% inhibition, the mean signal without enzyme addition was set to 100% inhibition, and the inhibition rate was calculated from the mean signal of 2 wells of each test substance test.

### MSA method:

5 µL of a compound solution at 4-fold concentration, 5 µL of substrate/ATP/metal solution at 4-fold concentration, and 10 µL of kinase solution at 2-fold concentration, each prepared with assay buffer (20 mM HEPES, 0.01% Triton X-100, 2 mM DTT, pH 7.5), were mixed in the wells of a polypropylene 384-well plate and reacted at room temperature for 1 or 5 hr. Successively, 70 µL of Termination Buffer (QuickScout Screening Assist MSA; Carna Biosciences) was added to discontinue the reaction. Then, the substrate peptide and the phosphorylated peptide in the reaction solution were separated and quantified by the LabChip system (Perkin Elmer). The kinase reaction was evaluated by the resultant product ratio (P/(P+S)) calculated from the substrate peptide peak height (S) and the phosphorylated peptide peak height (P).

As the result of this test, compounds k-1:H-7, k-1:H-1, k-1:D-1, k-1:J-1, and k-1:B-1 showed not less than 40% of activity inhibition rate at 100 nM against 8 kinds of kinases (CGK2, LATS2, MSK1, p70S6K, PKACα, PKACβ, SGK2, and SGK3).

**[Table 6]**

| | K-1:H-1 | k-1:H-7 | k-1:B-1 | k-1:D-1 | k-1:J-1 |
|---|---|---|---|---|---|
| **kinase** | **inhibition (%)** | | | | |
| CGK2 | 77.6 | 80.8 | 83.2 | 97.2 | 51.3 |
| LATS2 | 92.1 | 95.8 | 93.8 | 100.9 | 96.2 |
| MSK1 | 99.7 | 94.9 | 87.7 | 101.4 | 99.6 |
| p70S6K | 82 | 70.3 | 67.6 | 97.9 | 78.3 |
| PKAC *α* | 92.1 | 77.7 | 82.8 | 99.7 | 94 |
| PKAC *β* | 87.7 | 72 | 80.1 | 98.5 | 89.7 |
| SGK2 | 93.6 | 84.3 | 87.3 | 99.9 | 95.1 |
| SGK3 | 73.7 | 44.4 | 65.9 | 93.6 | 82.5 |

### [Example 3] Kinase inhibitory activity evaluation 3

For compounds k-1:I-1, k-1:D-1, k-1:J-1, k-1:H-1, k-1:H-7, k-1:B-1, k-5:H-1, k-1:H-3, k-1:D-4 and k-1:I-10, inhibitory activity evaluation (calculation of IC₅₀) was performed on LATS2. The activity was measured using the Off-chip Mobility Shift Assay (MSA method) described below. The compounds were added such that the test concentration of each compound was in 10 steps from the final concentration of 10 µM to 0.3 nM.

As an analysis method of the data, the mean signal of the control well containing all reaction components was set to 0% inhibition, the mean signal without enzyme addition was set to 100% inhibition, and the inhibition rate was calculated from the mean signal of 2 wells of each test substance test. The IC₅₀ value was obtained by approximating a plot based on the test substance concentration and inhibition rate to a 4 parameter logistics curve by using the Excel Solver add-in and by the non-linear least squares method.

### MSA method:

5 µL of k-1:I-1, k-1:D-1, k-1:J-1, k-1:H-1, k-1:H-7, k-1:B-1, k-5:H-1, k-1:H-3, k-1:D-4 and k-1:I-10 solutions at 4-fold concentration, 5 µL of substrate/ATP/metal solution at 4-fold concentration, and 10 µL of kinase solution at 2-fold concentration, each prepared with assay buffer (20 mM HEPES, 0.01% Triton X-100, 2 mM DTT, pH 7.5), were mixed in the wells of a polypropylene 384-well plate and reacted at room temperature for 1 or 5 hr. Successively, 70 µL of Termination Buffer (QuickScout Screening Assist MSA; Carna Biosciences) was added to discontinue the reaction. Then, the substrate peptide and the phosphorylated peptide in the reaction solution were separated and quantified by the LabChip system (Perkin Elmer). The kinase reaction was evaluated by the resultant product ratio (P/(P+S)) calculated from the substrate peptide peak height (S) and the phosphorylated peptide peak height (P).

As the result of this test, the IC₅₀ values of k-1:I-1, k-1:D-1, k-1:J-1, k-1:H-1, k-1:H-7, k-1:B-1, k-5:H-1, k-1:H-3, k-1:D-4 and k-1:D-10 are shown in Table 7. These compounds showed IC₅₀ of not more than 100 nM against LATS2, and have an inhibitory effect against LATS2.

**[Table 7]**

| **compound** | IC₅₀ (nM) |
|---|---|
| k-1:I-1 | 96 |
| k-1:D-1 | 1.84 |
| k-1:J-1 | 5.26 |
| k-1:H-1 | 12.9 |
| k-1:H-7 | 6.57 |
| k-1:B-1 | 5.12 |
| k-5:H-1 | 21.6 |
| k-1:H-3 | 46.9 |
| k-1:D-4 | 33.4 |
| k-1:I-10 | 15.0 |

### [Example 4] Action of specific compound on nuclear translocation of YAP protein

Compounds k-1:B-1, k-1:H-1, and k-1:J-1 were evaluated for nuclear translocation of YAP protein and TAZ protein. For the nuclear translocation, imaging analysis was performed using the human ovarian cancer cell line SKOV3 (manufactured by DS Pharma Biomedical Co., Ltd) and OperettaCLS (manufactured by PerkinElmer) described below. The compounds were added such that the final concentration of the test concentration of k-1:B-1, k-1:H-1, and k-1:J-1 was 10 µM.

### Imaging analysis:

Human ovarian cancer cell line SKOV3 cultured according to the protocol recommended by DS Pharma Biomedical Co., Ltd. was seeded on a 96-well plate (manufactured by PerkinElmer, CellCarrier-Ultra) at 15,000 cells/90 µL/well on the cycloolefin bottom surface, and cultured in a CO₂ incubator (37°C, 5% CO₂) for 1 day. After culturing, 10 µL of k-1:B-1, k-1:H-1, and k-1:J-1 solutions having a concentration of 100 µM and dissolved in DMSO was added. The cells were further cultured in a CO₂ incubator at 37°C for 4 hr. The medium was exchanged with 4% para-formaldehyde, and the cells were fixed by allowing to stand at room temperature for 10 min. The fixed cells were subjected to immunostaining with AlexaFluor(R) 647-labeled YAP antibody (manufactured by Cell Signaling Technology), anti-mouse TAZ (D-8) antibody (manufactured by SantaCruz), AlexaFluor (R) 555-labeled anti-mouse IgG antibody (manufactured by Thermo Fisher Scientific), according to the recommended protocol produced by Cell Signaling Technology, and the cell nucleus was further stained with 10 µg/mL Hoechst33342 solution.

Nine fields of view were imaged per well using a 20x objective lens of Operatta CLS. The cell nucleus region and the cytoplasm region were identified from the obtained fluorescence images by using the analysis software Harmony (manufactured by PerkinElmer), and AlexaFluor (R) 647 fluorescence intensity per unit area and the AlexaFluor (R) 555 fluorescence intensity per unit area in each region were extracted. The nuclear translocation was evaluated by calculating the ratio of the fluorescence intensity of the cell nucleus region to the fluorescence intensity of the cytoplasmic region from the extracted values. The value becomes greater than 1 as the YAP protein and TAZ protein transfer into the cell nucleus, and less than 1 when localized in the cytoplasm.

As the result of this test, Table 8 shows the nuclear translocation rate when no compound was added and when k-1:B-1, k-1:H-1, and k-1:J-1 were added. As shown in Table 8, it was clarified that the compounds k-1:B-1, k-1:H-1, and k-1:J-1 has an action of promoting the nuclear translocation of the YAP protein and the TAZ protein.

**[Table 8]**

| **compound** | **YAP nuclear translocation rate** | **TAZ nuclear translocation rate** |
|---|---|---|
| **no addition** | 6.15 | 6.98 |
| k-1 :B-1 | 6.98 | 7.93 |
| k-1 : H-1 | 7.60 | 8.84 |
| k-1: J-1 | 7.92 | 9.14 |

### [Example 5] Action of specific compound on nuclear translocation of YAP protein

Compounds k-1:B-1, k-1:H-1, and k-1:J-1 were evaluated for nuclear translocation of YAP protein and TAZ protein. For the nuclear translocation, imaging analysis was performed using the human bone marrow-derived mesenchymal stem cells (manufactured by PromoCell) and OperettaCLS (manufactured by PerkinElmer) described in Example 4. The compounds were added such that the final concentration of the test concentration of k-1:B-1, k-1:H-1, and k-1:J-1 was 10 µM.

### Sample preparation:

Human bone marrow-derived mesenchymal stem cells cultured according to the protocol recommended by PromoCell were seeded on a 96-well plate (manufactured by PerkinElmer, CellCarrier-Ultra) at 25,000 cells/90 µL/well on the cycloolefin bottom surface, and cultured in a CO₂ incubator (37°C, 5% CO₂) for 1 day. After culturing, k-1:B-1, k-1:H-1, and k-1:J-1 solutions having a concentration of 100 µM were added. The cells were cultured in a CO₂ incubator at 37°C for 4 hr. The medium was exchanged with 4% para-formaldehyde solution, and the cells were fixed by allowing to stand at room temperature for 10 min. The fixed cells were subjected to immunostaining by the method described in Example 4.

As the result of this test, Table 9 shows the nuclear translocation rate of the compound no addition group and when k-1:B-1, k-1:H-1, and k-1:J-1 were added. As shown in Table 9, it was clarified that the compounds k-1:B-1, k-1:H-1, and k-1:J-1 has an action of promoting the nuclear translocation of the YAP protein and the TAZ protein.

**[Table 9]**

| **compound** | **YAP nuclear translocation rate** | **TAZ nuclear translocation rate** |
|---|---|---|
| **no addition** | 8.96 | 10.05 |
| k-1:B-1 | 10.17 | 11.59 |
| k-1:H-1 | 11.55 | 13.42 |
| k-1:J-1 | 12.70 | 14.95 |

### [Example 6] Action of specific compound on gene expression

According to the method of patent document (WO 2014/017513), a composition of McCoy's 5a medium (manufactured by Sigma-Aldrich) containing 0.015%(w/v) deacylated gellan gum (KELCOGEL CG-LA, manufactured by Sansho Co., Ltd.), 30 ng/mL human HB-EGF (manufactured by PEPROTECH), and 15%(v/v) FBS was prepared. Human ovarian cancer cell line SKOV3 cultured according to the protocol recommended by DS Pharma Biomedical Co., Ltd. was suspended in the medium and seeded on a 6 well plate (manufactured by Corning Incorporated, #3471) at 1,000,000 cells/3.3 mL/well. Successively, k-1:B-1 dissolved in DMSO was added to the cell suspension at a final concentration of 10 µM. After further culturing in a CO₂ incubator at 37°C for 6 or 24 hr, 11 mL of phosphate buffered saline (PBS, manufactured by Sigma-Aldrich Japan) was added to the cell culture medium and the cells were precipitated by a centrifugation treatment (400G, 3 min). The supernatant was removed, the cells were suspended in PBS (10 mL), and further subjected to a centrifugation treatment (400G, 5 min) to give cell pellets. Successively, total RNA (ribonucleic acid) was isolated from the obtained cells by using RNAeasy Mini Kit (manufactured by Qiagen). DNA microarray of this RNA was performed by HumanGene 2.0ST Array (manufactured by Affymetrix), and changes in the human gene expression due to the addition of the specific compound were comprehensively analyzed (gene analysis contract service of Kurabo Industries Ltd. was used, product number; GA0312).

As the result of this analysis, Table 10 shows the YAP-regulated genes whose expression was increased due to the addition of k-1:B-1. The numerical values in Table 10 show the rate of expression level with addition of k-1:B-1 when the gene expression level without addition of the compound is 1. As shown in Table 10, it was clarified that compound k-1:B-1 has an effect of increasing the expression level of a gene whose expression is regulated by YAP protein.

**[Table 10]**

| gene name | expression rate when no addition is 1 | |
|---|---|---|
| | 6 hr later | 24 hr later |
| CTGF | 21.4 | 23.2 |
| CCND1 | 1.63 | 2.01 |
| CYR61 | 29.6 | 26.9 |
| TGFB2 | 3.63 | 9.84 |
| AMOTL2 | 6.92 | 7.89 |
| BIRC2 | 1.27 | 1.44 |
| ANKRD1 | 35.3 | 72.1 |

### [Example 7] Action of specific compound on protein amount of YAP, phosphorylated YAP, TAZ

2D culture conditions: Human ovarian cancer cell line SKOV3 cultured according to the protocol recommended by DS Pharma Biomedical Co., Ltd. was suspended in McCoy's 5a medium (manufactured by Sigma-Aldrich) containing 15%(v/v) FBS and seeded on a 100 mm plate (manufactured by Corning Incorporated, #430167) at 1,000,000 cells/10 mL and cultured in a CO₂ incubator for 24 hr. Successively, k-1:B-1 dissolved in DMSO was added to the culture medium at a final concentration of 1, 5, 10 µM. As the control, the medium without addition and the medium added with DMSO were cultured. After 1 hr, the cells were washed 3 times with ice-cooled phosphate buffered saline (PBS, manufactured by Wako Pure Chemical Industries, Ltd.), 250 µL of RIPA Buffer (manufactured by Wako Pure Chemical Industries, Ltd.) containing a protease inhibitor cocktail (manufactured by Roche) was added, and the cells were lysed with a scraper. The lysed solution was transferred to a tube, irradiated with ultrasonic waves at 4°C for 45 sec, and then centrifuged at 4°C for 10 min at 15,000 rpm. The supernatant was dispensed, the protein concentration was measured using BCA protein assay kit (manufactured by PIERCE), and then SDS (sodium dodecyl sulfate) gel electrophoresis was performed on the lysate. The protein was transferred to a membrane (manufactured by BioRad) and stored in a blocking buffer. Thereafter, the protein was probed with a primary antibody that recognizes YAP (manufactured by CST Japan), phosphorylated YAP (Ser127, manufactured by CST Japan), TAZ (manufactured by CST Japan), and GAPDH (manufactured by Santa Cruz). After incubation with a secondary antibody (manufactured by Horseradish peroxidase) linked with an anti-rabbit HRP (horseradish peroxidase) or an anti-mouse HRP, the protein was detected by a chemiluminescence detector (manufactured by BioRad) using ImmunoStar Zeta (manufactured by Wako Pure Chemical Industries, Ltd.).

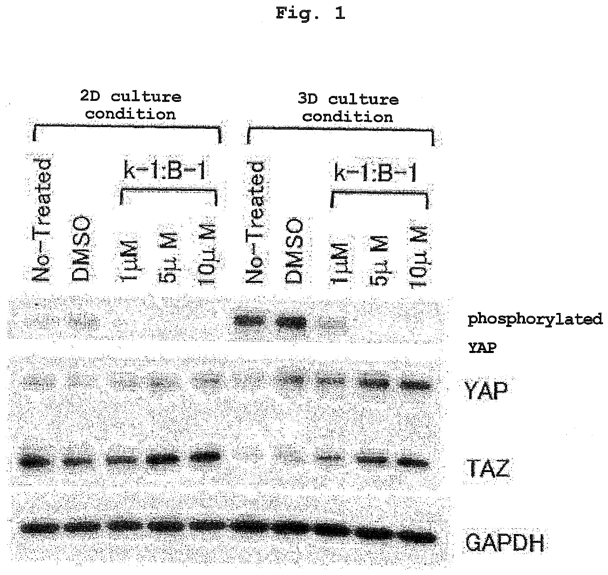

3D culture conditions: According to the method of patent document (WO 2014/017513), a composition of McCoy's 5a medium (manufactured by Sigma-Aldrich) containing 0.015%(w/v) deacylated gellan gum (KELCOGEL CG-LA, manufactured by Sansho Co., Ltd.), 30 ng/mL human HB-EGF (manufactured by PEPROTECH), and 15% (v/v) FBS was prepared. Human ovarian cancer cell line SKOV3 cultured according to the protocol recommended by DS Pharma Biomedical Co., Ltd. was suspended in the medium, seeded on a low adhesion 100 mm dish (manufactured by Corning Incorporated, #3262) at 2,000,000 cells/10 mL, and cultured in a CO₂ incubator for 24 hr. Thereafter, k-1:B-1 dissolved in DMSO was added to the culture medium to a final concentration of 1, 5, 10 µM. As the control, the medium without addition and the medium added with DMSO were cultured. After 1 hr, the culture medium was transferred to a 50 mL tube, and 40 mL of ice-cooled phosphate buffered saline (PBS, manufactured by Wako Pure Chemical Industries, Ltd.) was added. After centrifugation at 4°C for 3 min at 10,000 rpm, the supernatant was removed. Again, 50 mL of ice-cooled PBS was added, and the mixture was centrifuged at 4°C for 3 min at 10,000 rpm, and then the supernatant was removed. Ice-cooled PBS (1 mL) was added, the mixture was transferred to an Eppendorf tube, centrifuged at 4°C for 3 min at 15,000 rpm, and the supernatant was removed. Again, 1 mL of ice-cooled PBS was added and the mixture was centrifuged at 4°C for 3 min at 15,000 rpm, and the supernatant was removed. Thereafter, 250 µL of RIPA Buffer (manufactured by Wako Pure Chemical Industries, Ltd.) containing a protease inhibitor cocktail (manufactured by Roche) was added, and the cells were lysed by pipetting. After irradiation with ultrasonic waves at 4°C for 45 sec, the solution was centrifuged at 4°C for 10 min at 15,000 rpm. The supernatant was dispensed, the protein concentration was measured using BCA protein assay kit (manufactured by PIERCE), and then SDS gel electrophoresis was performed on the lysate. The protein was transferred to a membrane and stored in a blocking buffer. Thereafter, the protein was probed with a primary antibody that recognizes YAP (manufactured by CST Japan), phosphorylated YAP (Ser127, manufactured by CST Japan), TAZ (manufactured by CST Japan), and GAPDH (manufactured by Santa Cruz). After incubation with a secondary antibody (manufactured by Horseradish peroxidase) linked with an anti-rabbit HRP or an anti-mouse HRP, the protein was detected by a chemiluminescence detector (manufactured by BioRad) using ImmunoStar Zeta (manufactured by Wako Pure Chemical Industries, Ltd.). The results are shown in Fig. 1.

As the result of this analysis, it was clarified that the addition of k-1:B-1 suppressed the phosphorylation of YAP under both the conditions of 2D culture and 3D culture. In addition, it was clarified that the addition of k-1:B-1 increased the total YAP protein amount and total TAZ protein amount in the cell under both the conditions of 2D culture and 3D culture.

### [Example 8] Action of specific compound on liver weight increase in mouse partial liver resection model

A partial liver resection model in which the mouse liver was partially resected was prepared as described below, and the liver weight increasing effect of the specific compound was evaluated by comparing the liver weight immediately after the resection with the liver weight 3 days after the resection.

### Evaluation sample:

A 0.5w/v% methylcellulose aqueous solution (0.5% MC, manufactured by FUJIFILM Wako Pure Chemical Corporation) and k-1:B-1, k-1:J-1 were evaluated. k-1:B-1 and k-1:J-1 were suspended in 0.5% MC, and prepared at a final concentration of 1 mg/mL.

### Procedure:

Mice, BALB/cAnNCrlCrlj, 5-week-old, male, were purchased from Charles River Laboratories, Japan. In terms of the 0.5% MC administration control group, 0.5% MC 10 mL/kg was administered intraperitoneally once, and 24 hr later, the entire liver was isolated and the liver weight was measured (n=5). In terms of the 0.5% MC administration liver resection group, 0.5% MC 10 mL/kg was intraperitoneally administered 4 times in total every 24 hr. Immediately after the second administration, the abdomen was opened under isoflurane (manufactured by Intervet Co., Ltd.) anesthesia, and the root of liver lobe was ligated with a suture thread, and about 30% of the liver was finally removed by excising the tip of the ligated liver lobe. After that, the abdomen was closed, and 24 hr after the final administration, the entire liver was removed and the liver weight was measured (n=4). In terms of the k-1:B-1 or k-1:J-1 administration-control group, k-1:B-1 or k-1:J-1 10 mg/kg, 10 mL/kg, was intraperitoneally administered once, and 24 hr later, the entire liver was isolated and the liver weight was measured (n=5). In terms of the k-1:B-1 or k-1:J-1-liver liver resection group, k-1:B-1 or k-1:J-1 10 mg/kg, 10 mL/kg, was intraperitoneally administered continuously 4 times in total every 24 hr. Immediately after the second administration, the abdomen was opened under isoflurane anesthesia, the root of liver lobe was ligated with a suture thread, and about 30% of the liver was finally removed by excising the tip of the ligated lobe. After that, the abdomen was closed, and 24 hr after the final administration, the entire liver was removed and the liver weight was measured (n=5) .

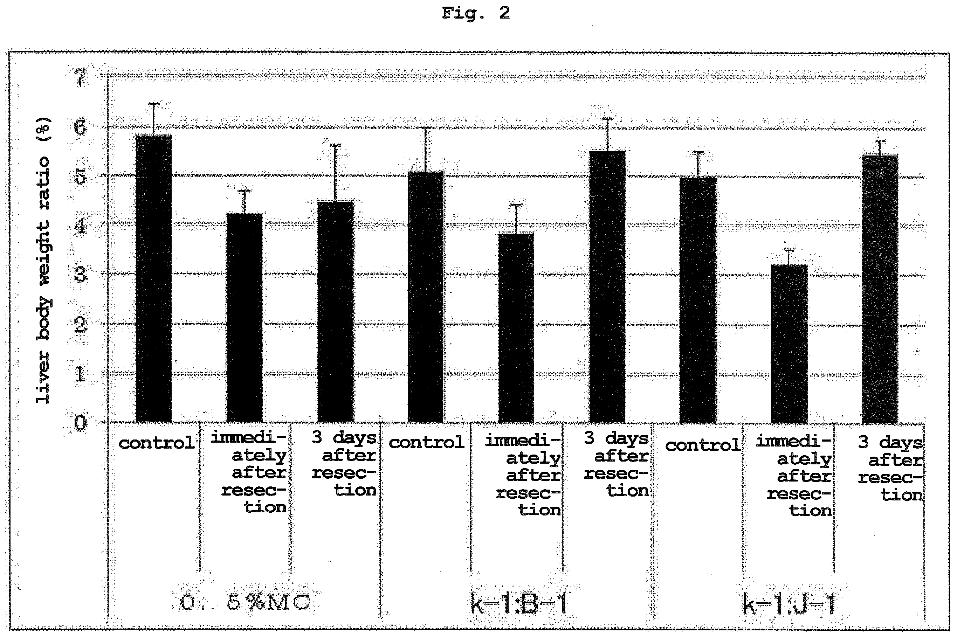

As the result of this analysis, Fig. 2 shows changes in the liver weight ratio of the partial liver resection mouse administered with 0.5% MC, k-1:B-1 or k-1:J-1. As shown in Fig. 2, it was clarified that k-1:B-1 and k-1:J-1 have an action of promoting recovery of the liver weight after excision. That is, it was clarified that k-1:B-1 and k-1:J-1 has a treatment effect on tissue damage.

### [Example 9] Evaluation of kinase inhibitory activity of optical isomer

For compound k-1:B-1 (racemate), GA-002A, GA-002B, GA-005A and GA-005B, inhibitory activity evaluation (calculation of IC₅₀) was performed on LATS1 and LATS2 (manufactured by Carna Biosciences). The activity was measured using the Kinase Assay method described below. The compounds were added such that the test concentration of k-1:B-1 (racemate), GA-002A, GA-002B, GA-005A and GA-005B was in 8 steps (10 µM, 1 µM, 100 nM, 30 nM, 10 nM, 3 nM, 1 nM, 0.3 nM) from the final concentration of 10 µM to 0.3 nM.

As the analysis method of the data, the mean signal of the control well containing reaction components other than the evaluation compound was set to 0% inhibition, the mean signal without enzyme addition was set to 100% inhibition, and the inhibition rate was calculated from the mean signal of 2 wells of each test substance test. IC₅₀ value was calculated using EXSUS (version 7.1.6, manufactured by CAC EXICARE Corporation).

### Kinase Assay method:

2 µL of a compound of the present invention solution at 2.5-fold concentration, 1 µL of substrate at 5-fold concentration (SGKtide: NH₂-CKKRNRRLSVA-COOH, manufactured by SignalChem), ATP (manufactured by Sigma-Aldrich), MgCl₂ (manufactured by FUJIFILM Wako Pure Chemical Corporation) solution (final concentration 10 µM, 400 µM or 5 mM), and 2 µL of kinase solution at 2.5-fold concentration (final concentration of LATS1 or LATS2 5 µg/mL), each prepared with assay buffer (20 mM HEPES, 0.01% Triton X-100, 2 mM DTT, pH 7.5), were mixed in the wells of a polypropylene 384-well plate (manufactured by Greiner Bio-One, #784900) and reacted at room temperature for 5 hr. Successively, 5 µL detection solution (Fluorospark (registered trade mark)Kinase/ADP Multi-Assay Kit; manufactured by FUJIFILM Wako Pure Chemical Corporation) was added, and the mixture was reacted at room temperature 30 min. Successively, 5 µL of a reaction quenching liquid (Fluorospark (registered trade mark) Kinase/ADP Multi-Assay Kit; manufactured by FUJIFILM Wako Pure Chemical Corporation) was added to discontinue the reaction. Thereafter, the ADP concentration of the reaction solution was quantified by EnSpire (manufactured by PerkinElmer) to detect the kinase reaction.

As the result of this test, the eleventh table shows the IC₅₀ values of k-1:B-1 (racemate), GA-002A (levorotatory k-1:B-1, R form), GA-002B (dextrorotatory k-1:B-1, S form), GA-005A (R form) and GA-005B (S form), and the twelfth table shows the inhibitory rates.

### [Eleventh Table]

**[Table 11]**

| **compound** | LATS1 IC₅₀ (nM) | LAT S2 IC₅₀ (nM) |
|---|---|---|
| k-1 : B-1 | 8.88 | 30.8 |
| GA-002A | 2.67 | 8.23 |
| GA-0 0 2B | 4.81 | 15 44 |
| GA-005A | 4.79 | 7.39 |
| GA-005B | 1271 | 652 |

### [Twelfth Table]

**[Table 12]**

| **compound** | **LARS1 inhibitory rate (%)** | | **LARS2 inhibitory rate (%)** | |
|---|---|---|---|---|
| | 100 nM | 1000 nM | 100 nM | 1000 nM |
| K-1:B-1 | 94 | 102 | 75 | 96 |
| GA-002A | 101 | 101 | 91 | 98 |
| GA-002B | 33 | 77 | 9 | 45 |
| GA-005A | 96 | 97 | 99 | 100 |
| GA-005B | 14 | 45 | 27 | 55 |

As shown in the eleventh table and the twelfth table, it was shown that k-1:B-1 (racemate), GA-002A and GA-005A have a high inhibitory effect against LATS1 and LATS2. Particularly, the IC₅₀ of GA-002A decreased by 2 times or more as compared to k-1:B-1 (racemate).

### [Example 10] Action of specific compound on cell proliferation using human epidermal keratinocyte

As human primary epidermal keratinocyte, #PCS-200-010 (ATCC; American Type Culture Collection) was precultured using an epidermal cell basal medium (ATCC, #PCS-200-030) supplemented with a keratinocyte additive kit (ATCC, #PCS-200-040) and by a monolayer culture method. After suspending the epidermal keratinocytes in the same medium, the specific compound used in the present invention and dissolved in DMSO was added to a final concentration of 10 µM and the mixture was seeded in a 96 well U-bottom cell low-adhesion plate (Corning Incorporated, #4520) at 1600 cells/90 µL/well. The cells were cultured at 37°C in a 5% CO₂ incubator for 4 days. As a control, DMSO was added to a final concentration of 0.1%. After culturing, the number of viable cells was measured using an ATP reagent (Promega KK, CellTiter-Glo (registered trade mark) Luminescent Cell Viability Assay). ATP reagent was added to the culture medium in the flat bottom plate at 64 µL/well and suspended therein, and the suspension (100 µL/well) was transferred to an assay white plate (Corning Incorporated, #3912). After allowing to stand at room temperature for 10 min, the amount of luminescence was measured using a plate reader (PerkinElmer Inc., EnSpire).

The relative value of the measured luminescence amount with respect to the control group (DMSO) was calculated, and it was found that the luminescence amount increased 4 times or more by adding the following compound. That is, the number of cells increased 4 times or more by the addition of the following compound. From this, it was shown that the compound of the present invention has an effect of promoting the proliferation of epidermal keratinocytes.
Compounds with 4 times or more increase in cell number: k-1:B-1, GA-002A, k-1:J-1, k-1:H-1

### [Example 11] Action of specific compound on gene expression of human primary epidermal keratinocyte and human primary cornea epithelial cell

Similar to Example 10, human primary epidermal keratinocyte (ATCC, #PCS-200-010) was precultured using an epidermal cell basal medium (ATCC, #PCS-200-030) supplemented with a keratinocyte additive kit (ATCC, #PCS-200-040) and by a single layer culture method. In addition, human primary cornea epithelial cell (ATCC, #PCS-700-010) was precultured using a cornea epithelial cell basal medium (ATCC, #PCS-700-030) supplemented with a cornea epithelial cell additive kit (ATCC, #PCS-700-040) and by a single layer culture method. The cornea epithelial cell and epidermal keratinocyte were seeded on a 24 well flat bottom plate (Corning Incorporated, #3526) at 100,000 cells/380 µL/well and cultured at 37°C in a 5% CO₂ incubator for 24 hr. Then, the medium was exchanged with a medium containing, at a final concentration of 10 µM, the specific compound used in the present invention and dissolved in DMSO, and the cells were further cultured for 24 hr. After culturing, the cell culture medium was removed, D-PBS (FUJIFILM Wako Pure Chemical Corporation, #049-29793) was added at 500 µL/well and removed. Successively, total RNA (ribonucleic acid) was isolated from the cell using RNeasy Mini Kit (manufactured by Qiagen). Gene expression analysis of the RNA was performed using TaqmanAssay (AppliedBioSystems), and changes in the human gene expression at the downstream of YAP and TAZ proteins due to the addition of the specific compound were analyzed. The analyzed gene name and the AssayID of the probe used are shown below.
gene name AssayID
GAPDH Hs02758991_g1
CYR61 Hs00155479_m1
ANKRD1 Hs00173317_m1
CCND1 Hs00765553_m1

The relative value of the obtained expression level with respect to GAPDH was calculated. As a result, the relative expression level increased as compared to the control group (DMSO) by the addition of the specific compound. That is, it was clarified that the specific compound used in the present invention has an effect of increasing the expression level of a gene whose expression is regulated by YAP protein.
Compounds with 5 times or more increase in ANKRD relative expression level (epidermal keratinocyte):
   k-1:B-1, GA-002A, k-1:J-1, k-1:H-1
Compounds with 3 times or more increase in ANKRD relative expression level (cornea epithelial cell):
   k-1:B-1, GA-002A, k-1:J-1, k-1:H-1
Compounds with 5 times or more increase in ANKRD relative expression level (cornea epithelial cell):
   k-1:B-1, GA-002A, k-1:J-1
Compounds with 1.5 times or more increase in CCND1 relative expression level (epidermal keratinocyte):
   k-1:B-1, GA-002A, k-1:J-1, k-1:H-1
Compounds with 1.5 times or more increase in CCND1 relative expression level (cornea epithelial cell):
   k-1:B-1, GA-002A, k-1:J-1, k-1:H-1
Compounds with 1.5 times or more increase in CYR61 relative expression level (epidermal keratinocyte):
   k-1:B-1, GA-002A, k-1:J-1, k-1:H-1
Compounds with 2 times or more increase in CYR61 relative expression level (cornea epithelial cell) :
   k-1:B-1, GA-002A, k-1:J-1, k-1:H-1

### [Example 12] Action of specific compound on nuclear translocation of YAP protein and TAZ protein

The specific compound used in the present invention was evaluated for the nuclear translocation of YAP protein and TAZ protein. For the nuclear translocation, imaging analysis was performed using the human ovarian cancer cell line SKOV3 (DS Pharma Biomedical Co., Ltd) and OperettaCLS (PerkinElmer) described below. The specific compound was added such that the final concentration of the test concentration of the compound used in the present invention was 10 µM.

### Imaging analysis:

Human ovarian cancer cell line SKOV3 cultured according to the protocol recommended by DS Pharma Biomedical Co., Ltd. was seeded on a 96-well plate (PerkinElmer, CellCarrier-Ultra) at 15,000 cells/90 µL/well on the cycloolefin bottom surface, and cultured in a CO₂ incubator (37°C, 5% CO₂) for 1 day. After culturing, a solution of the specific compound used in the present invention dissolved in DMSO was added. The cells were further cultured in a CO₂ incubator at 37°C for 4 hr, the medium was exchanged with 4% para-formaldehyde, and the cells were fixed by allowing to stand at room temperature for 10 min. The fixed cells were subjected to immunostaining with AlexaFluor(R) 647-labeled YAP antibody (Cell Signaling Technology), anti-mouse TAZ (D-8) antibody (SantaCruz), AlexaFluor (R) 555-labeled anti-mouse IgG antibody (Thermo Fisher Scientific), according to the recommended protocol produced by Cell Signaling Technology, and the cell nucleus was further stained with 10 µg/mL Hoechst33342 solution.

Nine fields of view were imaged per well using a 20x objective lens of Operatta CLS. The cell nucleus region and the cytoplasm region were identified from the obtained fluorescence images by using the analysis software Harmony (manufactured by PerkinElmer), and YAP protein-AlexaFluor(R)647 fluorescence intensity and TAZ protein-AlexaFluor(R)555 fluorescence intensity in each region were extracted. Furthermore, the ratio of the fluorescence intensity of the cell nucleus region to the fluorescence intensity of the cytoplasmic region was calculated from the extracted values. This time, the case where the fluorescence intensity ratio was 1.4 or more was defined as the cell population in which the YAP protein and the TAZ protein were transferred into the nucleus, and the ratio thereof was calculated.

The relative value of the calculated ratio of the cell population with respect to the control group (DMSO) was calculated, and it was found that the cell population increased 6 times or more for YAP protein and 1.5 times or more for TAZ protein by the addition of the following compound. Therefrom it was clarified that the specific compound used in the present invention has an action of promoting the nuclear translocation of the YAP protein and TAZ protein.
Compounds with 6 times or more increase in cell population with nuclear translocation of YAP protein:
   k-1:B-1, GA-002A, k-1:J-1, k-1:H-1
Compounds with 1.5 times or more increase in cell population with nuclear translocation of TAZ protein:
   k-1:B-1, GA-002A, k-1:J-1, k-1:H-1

### [Example 13] YAP phosphorylation inhibitory action of the compound of the present invention

Human ovarian cancer cell line SKOV3 (manufactured by DS Pharma Biomedical Co., Ltd) was precultured (single layer culture) in a 15% FBS-containing McCoy's 5a medium (manufactured by Sigma Ltd. Aldrich). The above-mentioned cells in the logarithmic growth phase were washed with PBS, a 0.25%(w/v) trypsin-1 mmol/L ethylenediaminetetraacetic acid (EDTA) solution (manufactured by Fujifilm Wako Pure Chemical Corporation) was added, and adherent cells were detached by incubating at 37°C for 3 min. The above-mentioned medium was added and the mixture was centrifuged and resuspended in the same medium.

According to the method of patent document (WO 2014/017513), a composition of 10% FBS-containing DMEM medium (Phenol-red free, manufactured by FUJIFILM Wako Pure Chemical Corporation) containing 0.015%(w/v) deacylated gellan gum (KELCOGEL CG-LA, manufactured by SANSHO Co., Ltd.) was prepared using FCeM-series Preparation Kit (manufactured by FUJIFILM Wako Pure Chemical Corporation). Then, various cells prepared above were suspended in the above-mentioned medium composition (3D conditions) added with deacylated gellan gum or a medium without the addition (2D conditions), and seeded on a 50 µL/well (manufactured by Corning Incorporated, 96 well flat bottom plate, #3585, 2D conditions) or 25 µL/well (manufactured by Corning Incorporated, 96 well low adhesion flat bottom plate, #3474) at 100000 cells/well. The plate was allowed to stand at 37°C in a 5% CO₂ incubator, and the compound of the present invention diluted with the medium at 2 or 6 times the final concentration, DMSO, or the medium alone was added to the plate 3 hr after the seeding at 50 µL/well (2D conditions) or 5 µL/well (3D conditions) to make the final concentration of each compound 10 µM. After culture at 37°C in a 5% CO₂ incubator for 1 hr, the supernatant was aspirated, supplemented Lysis buffer (1-fold concentration, manufactured by Cisbio, YAP-total kit or YAP phospho-S127 kit) (50 µL) was added under 2D conditions, and supplemented Lysys buffer (4-fold concentration) (10 µL) was added to the culture supernatant under 3D conditions, and the mixture was stirred by a plate shaker for 30 min. Thereafter, the plate was centrifuged, 16 µL of the supernatant was transferred to a 384-well white plate (Corning, #4512), and each antibody solution (pYAP d2 antibody or Total-YAP d2 antibody, and pYAP Cryptate antibody or Total-YAP Cryptate antibody) included in the kit was added at 4 µL/well in total, and the mixture was allowed to stand at room temperature overnight. As a blank control, pYAP Cryptate antibody or Total-YAP Cryptate antibody alone was added to cells free of stimulation with compound and the mixture was treated in the same manner (Non-treatment). The next day, the wavelengths at 665 nm and 615 nm were measured in the HTRF mode of EnVision (manufactured by PerkinElmer).

The obtained signal was calculated as follows. Ratio=signal(665 nm)/signal(615 nm)×10000, ΔR=Signal(Ratio)-Background fluorescence, and the relative value with respect to ΔR of the compound non-addition group (Non-treatment) as 100% was calculated, and the phosphorylated YAP amount or total YAP amount by each compound was evaluated.

**[Table 13]**

| **2D condition** | **phosphorylated YAP (%)** | **total YAP (%)** |
|---|---|---|
| Non-treatment | 100 | 100 |
| DMSO | 99 | 100 |
| k -1:B-1 | 14 | 89 |
| k-1:H-1 | 38 | 94 |
| k-1:J-1 | 1 3 | 86 |
| GA-002A | 1 0 | 86 |
| GA-005A | 10 | 91 |

| **3D condition** | **phosphorylated YAP (%)** | **total YAP (%)** |
|---|---|---|
| Non-treatment | 100 | 100 |
| DMS O | 106 | 97 |
| k-1:B-1 | 31 | 99 |
| k-1:H-1 | 80 | 107 |
| k-1:J-1 | 30 | 109 |
| GA-002A | 14 | 105 |
| GA-005A | 1 0 | 107 |

As shown in Table 13, it was shown that k-1:B-1, k-1:H-1, k-1:J-1, GA-002A, and GA-005A have a YAP phosphorylation inhibitory action under both 2D and 3D culture conditions.

### [Example 14] Inflammation suppressive effect on colitis model mouse

Colitis model administered with dextran sulfate sodium (DSS) (manufactured by FUJIFILM Wako Pure Chemical Corporation) was produced as described below. The length of the large intestine is used as a morphological parameter in the evaluation of colitis, and the large intestine shortens according to the degree of inflammation. Therefore, the antiinflammatory effect of the compound was evaluated by comparing the length of the large intestine.

### Evaluation sample:

Compound K-1:J-1 was prepared as a 0.5w/v% methylcellulose aqueous solution (0.5% MC, manufactured by FUJIFILM Wako Pure Chemical Corporation) at 5 mg/mL and intraperitoneally administered at 50 mg/kg per administration. 0.5% MC was administered to the negative control.

### Procedure:

As the mouse, male C57BL/6NCrl, 6-week-old, was purchased from CHARLES RIVER LABORATORIES JAPAN, INC. As drinking water, distilled water or a 2.5 w/v% DSS aqueous solution was freely given. The DSS drinking start date was set as day 1, and the water was given until day 8. Compound K-1:J-1 was intraperitoneally administered at 50 mg/kg every 24 hr from day 1. On day 8, the mouse was dissected, the large intestine was isolated and the length of the large intestine was measured. Conducted in 4 cases in each group.

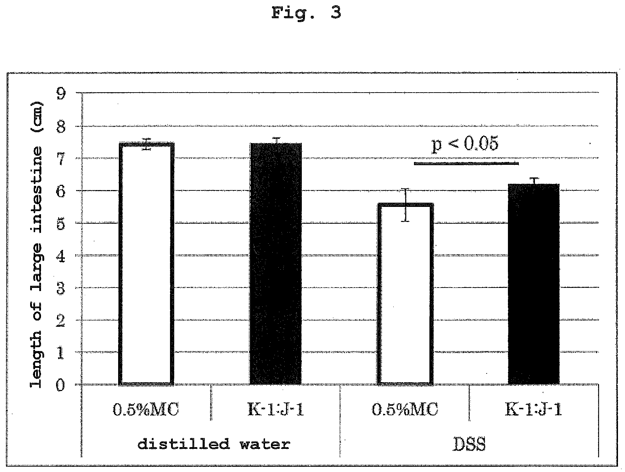

As the result of this test, Fig. 3 shows the length of the large intestine of the mouse. As shown in Fig. 3, the large intestine of the DSS drinking water group was shorter than that of the distilled water drinking group. The shortening of the large intestine was suppressed more in the DSS drinking water - compound K-1:J-1 administration group than in the DSS drinking water - 0.5% MC administration group. From the above, it was clarified that compound K-1:J-1 has an effect of suppressing the onset of DSS-derived colitis.

### [Example 15] Wound therapy promoting effect in mouse full-thickness skin wound model

A full-thickness skin wound in which the skin on the back of a mouse was partially removed was prepared as described below, and the wound therapy promoting effect of the compound was evaluated by comparing the reduction rate of the wound area. Evaluation sample:
Compound K-1:J-1 was dissolved in a solvent (10% Pluronic F-68, 2% Propylene Glycol, 20 mM Tris buffer (pH 8.5)) at a concentration of 1 mg/mL, and administered by adding dropwise to the wound site (0.04 mL per one wound site). To the negative control, the solvent alone was administered by dropwise addition.

### Procedure:

As the mouse, male BKS.Cg-Dock7m+/+Leprdb/J, 7-week-old, was purchased from CHARLES RIVER LABORATORIES JAPAN, INC. After removing the hair on the back, an 8 mm full-thickness skin wound was prepared using biopsy trepan (Kai Corporation) and protected with a coating material (Tegaderm transparent dressing, 3M). The wound preparation date was set as day 1, and 1 mg/mL compound K-1:J-1 was added dropwise at a dose of 0.04 mL per wound site every 24 hr until day 12. The area of the wound site was measured on days 1, 3, 6, 9, 13. The wound area on day 1 was set to 100%, and the total measured area was calculated as a percentage (%) with respect to the wound preparation date. Conducted in 6 cases in each group.

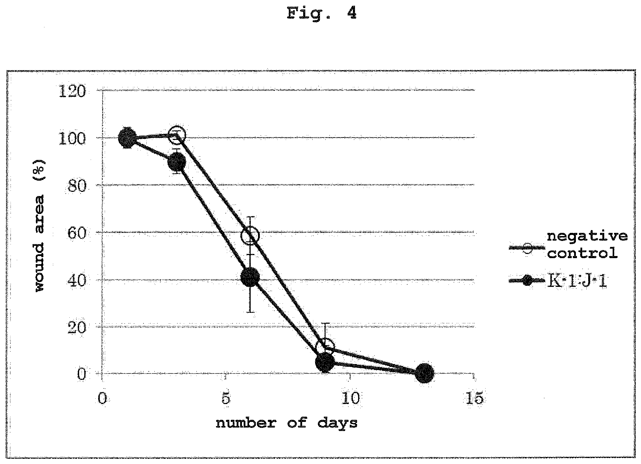

As the result of this test, Fig. 4 shows changes in the wound area of the full-thickness skin wound model mouse. In addition, the area under the wound area ratio (Area Under the Curve (AUC), % × day) calculated from the result of the change over time of the wound area ratio was 854.2±119.2 in the negative control, and 659.6±163.7 in the K-1:J-1 administration group. As shown in Fig. 4 and AUC value, it was clarified that compound K-1:J-1 has a wound therapy promoting effect.

### [Industrial Applicability]

According to the present invention, it is possible to inhibit plural kinases including LATS (particularly LATS2) which is the major kinase in the Hippo signal transduction pathway. In addition, diseases or tissue damage associated with failure of cellular proliferation can be treated. Therefore, the present invention is beneficial, for example, in the research field of cell functions and diseases, in which the Hippo signal transduction pathway is involved, and the like. Furthermore, it is beneficial in the medical field for the treatment of such diseases and the like.

This application is based on patent application No. 2018-110748 filed in Japan (filing date: June 8, 2018) and patent application No. 2019-014898 filed in Japan (filing date: January 30, 2019), the contents of which are incorporated in full herein.

## Claims

1. A kinase inhibitor comprising a compound represented by the following formula (I), or a salt thereof: 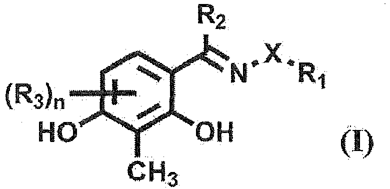 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.

2. The inhibitor according to claim 1, wherein the kinase is selected from the group consisting of CGK2, LATS2, MSK1, p70S6K, PKACα, PKACβ, SGK2, and SGK3.

3. The inhibitor according to claim 2, wherein the kinase is LATS2.

4. A Hippo signal transduction pathway inhibitor comprising a compound represented by the following formula (I) or a salt thereof: 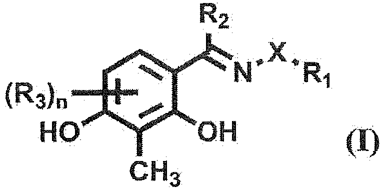 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.

5. A therapeutic agent for a disease or tissue damage associated with failure of cellular proliferation, comprising a compound represented by the following formula (I) or a salt thereof: 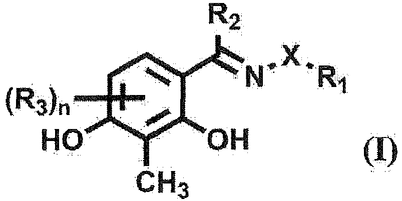 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.

6. The therapeutic agent according to claim 5, wherein the disease or tissue damage associated with failure of cellular proliferation is a disease associated with suppressed nuclear translocation of YAP and/or TAZ.

7. The therapeutic agent according to claim 5, wherein the disease or tissue damage associated with failure of cellular proliferation is selected from the group consisting of inflammatory disease, inflammatory bowel disease, neurodegenerative disease, immune-nerve disease, muscular dystrophy, myopathy, trauma, burn, chemical burn, skin ulcer, traumatic ulcer, lower leg ulcer, frostbite ulcer, immune-ulcer, postherpetic ulcer, radiation ulcer, pressure ulcer, diabetic skin ulcer, giant pigmented nevus, scar, disorder due to tattoo, vitiligo vulgaris, leukopathia, spinal cord damage, muscle damage, liver failure, drug-induced hepatopathy, alcohol-induced hepatopathy, ischemic hepatopathy, viral hepatopathy, autoimmune hepatopathy, acute hepatitis, chronic hepatitis, cirrhosis, skin damage, brain edema, myocardial infarction, cerebral hemorrhage, and cerebral infarction.

8. A proliferation promoter of a cell or tissue for a transplantation treatment, comprising a compound represented by the following formula (I) or a salt thereof: 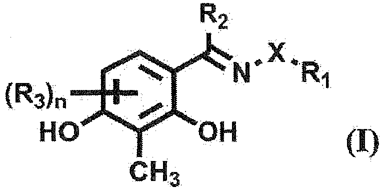 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2}.

9. The inhibitor, therapeutic agent or proliferation promoter according to any one of claims 1 to 8, wherein X is -NHCO-.

10. The inhibitor, therapeutic agent or proliferation promoter according to any one of claims 1 to 9, wherein R₂ is an alkyl group having 1 - 6 carbon atoms, and n is 0.

11. The inhibitor, therapeutic agent or proliferation promoter according to any one of claims 1 to 10, wherein R₁ is -Y-W-Z-Ar, Y is a methylene group optionally having an alkyl group having 1 - 6 carbon atoms, W is N(R₄), Z is a single bond, and Ar is an aryl group optionally having a halogen atom, a hydroxyl group, an alkyl group having 1 - 6 carbon atoms or an alkoxy group having 1 - 6 carbon atoms.

12. The inhibitor, therapeutic agent or proliferation promoter according to any one of claims 1 to 11, wherein R₂ is a methyl group, an ethyl group, or an isobutyl group,
n is 0,
Ar is a phenyl group optionally substituted by a hydroxyl group or a methyl group, and
Y is a methylene group optionally substituted by a methyl group or an ethyl group.

13. The inhibitor, therapeutic agent or proliferation promoter according to any one of claims 1 to 12, wherein the compound represented by the formula (I) is a compound selected from the group consisting of the following:

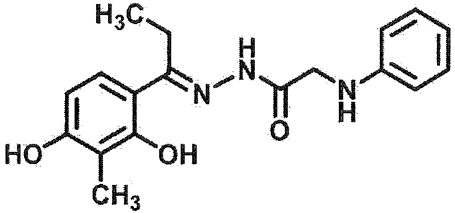

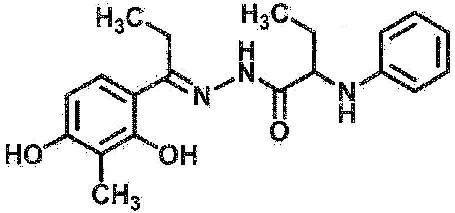

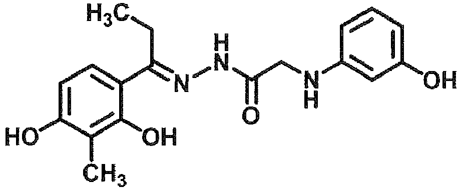

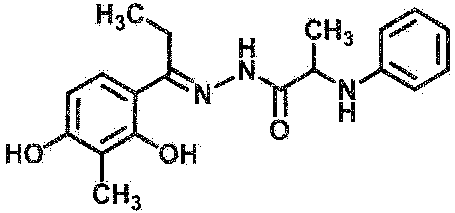

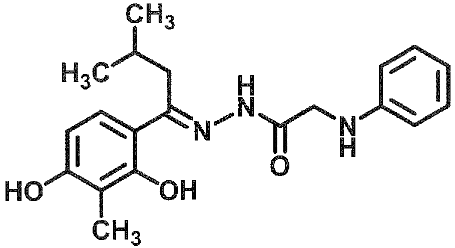

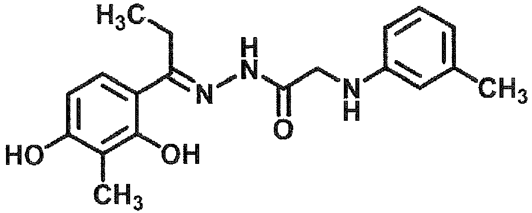

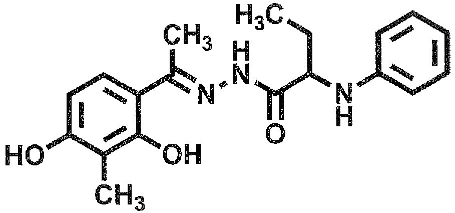

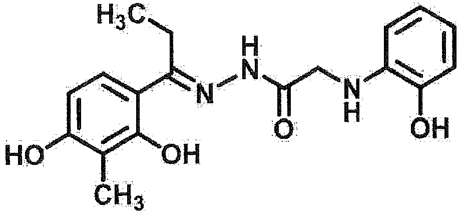

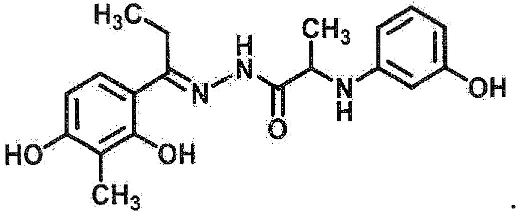

14. The inhibitor, therapeutic agent or proliferation promoter according to any one of claims 1 to 12, wherein the compound represented by the formula (I) is an optical isomer of the R form of a compound selected from the group consisting of:

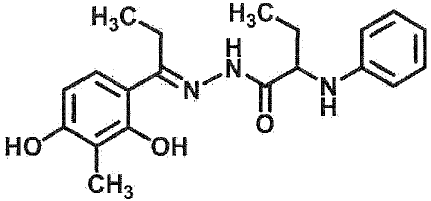

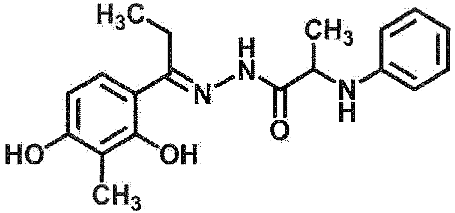

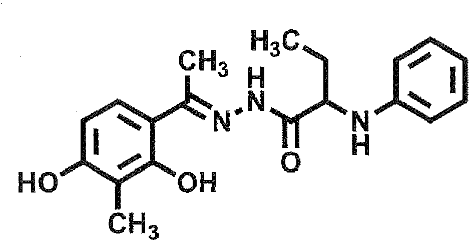

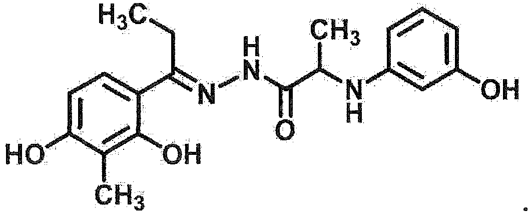

15. The inhibitor, therapeutic agent or proliferation promoter according to any one of claims 1 to 12, wherein R₁ is -Y-W-Z-Ar, Y is a single bond, W is N(R₄), R₄ is a hydrogen atom, Z is a methylene group optionally having an alkyl group having 1 - 6 carbon atoms, and Ar is an aryl group optionally having a halogen atom, a hydroxyl group, an alkyl group having 1 - 6 carbon atoms or an alkoxy group having 1 - 6 carbon atoms.

16. The inhibitor, therapeutic agent or proliferation promoter according to any one of claims 1 to 10, and 15, wherein R₂ is a methyl group, an ethyl group, or an isobutyl group,
n is 0,
Ar is a phenyl group optionally substituted by a hydroxyl group, and
Z is a methylene group optionally substituted by a methyl group or an ethyl group.

17. The inhibitor, therapeutic agent or proliferation promoter according to any one of claims 1 to 10, 15, and 16, wherein the compound represented by the formula (I) is a compound selected from the group consisting of the following:

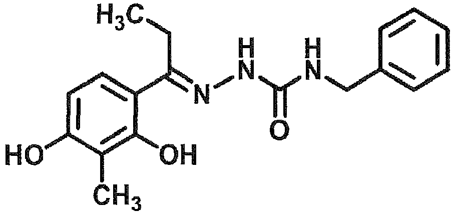

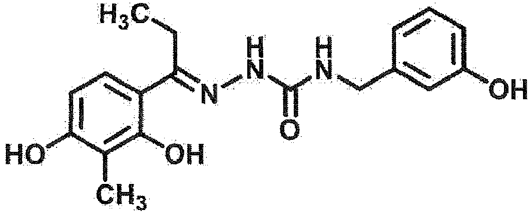

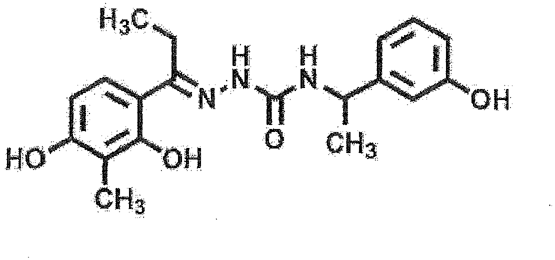

18. The inhibitor, therapeutic agent or proliferation promoter according to any one of claims 1 to 12, 15, and 16, wherein the compound represented by the formula (I) is an optical isomer of the R form of the following compound:

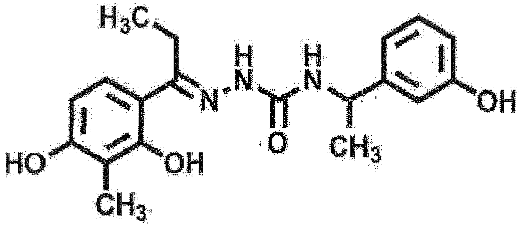

19. A method for screening for a substance for treating and/or preventing a disease associated with promoted nuclear translocation of YAP and/or TAZ, comprising the following steps:
(step 1) a step of culturing cells in a medium containing a compound represented by the following formula (I) or a salt thereof: 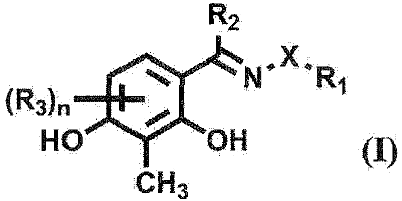 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2},
(step 2) a step of measuring, in the presence of a test substance, an abundance of YAP and/or TAZ in the nucleus of the cell obtained in (step 1);
(step 3) a step of determining the test substance as a substance for treating and/or preventing a disease associated with promoted nuclear translocation of YAP and/or TAZ when the abundance of YAP and/or TAZ in the nucleus measured in (step 2) is reduced compared to an abundance of YAP and/or TAZ in the absence of the test substance.

20. An optically active form of a compound represented by the following formula (I), or a salt thereof: 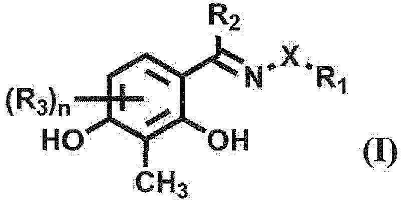 {wherein, X is a single bond, -CH₂COO-, -CONH-, or -NHCO-, R₁ is an alkyl group having 1 - 10 carbon atoms and optionally having substituent(s), an aryl group optionally having substituent(s), or -Y-W-Z-Ar wherein Y and Z are each a single bond or an alkylene group having 1 - 6 carbon atoms and optionally having substituent(s), W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms and optionally having substituent(s), R₃ is a hydroxyl group, and n is 0, 1 or 2 (provided that when X is -NHCO-, R₂ is an ethyl group, and n is 0, then R₁ is not -CH₂-NH-C₆H₅)}.

21. The optically active form or a salt thereof according to claim 20, wherein X is -NHCO-.

22. The optically active form or a salt thereof according to claim 20 or 21, wherein R₂ is an alkyl group having 1 - 6 carbon atoms, and n is 0.

23. The optically active form or a salt thereof according to any one of claims 20 to 22, wherein R₁ is -Y-W-Z-Ar, Y is a methylene group having an alkyl group having 1 - 6 carbon atoms, W is N(R₄), Z is a single bond, and Ar is an aryl group optionally having a halogen atom, a hydroxyl group, an alkyl group having 1 - 6 carbon atoms or an alkoxy group having 1 - 6 carbon atoms.

24. The optically active form or a salt thereof according to any one of claims 20 to 23, wherein the optically active form is an R form.

25. The optically active form or a salt thereof according to any one of claims 20 to 24, wherein the compound represented by the formula (I) is a compound selected from the group consisting of the following:

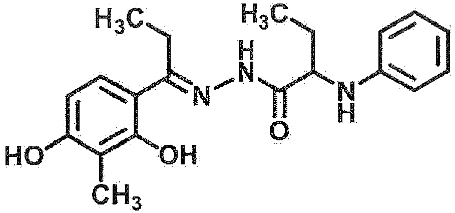

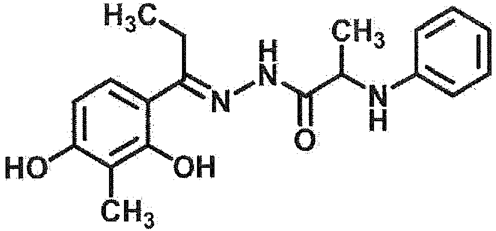

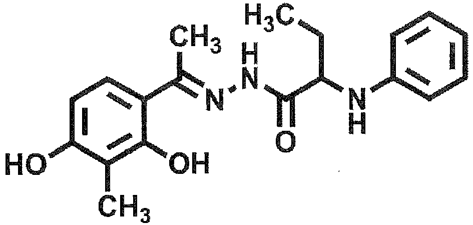

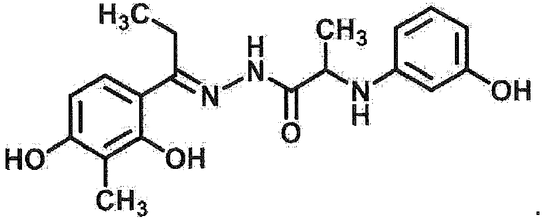

26. The optically active form or a salt thereof according to any one of claims 20 to 22, wherein R₁ is -Y-W-Z-Ar, Y is a single bond, W is N(R₄), R₄ is a hydrogen atom, Z is a methylene group having an alkyl group having 1 - 6 carbon atoms, and Ar is an aryl group optionally having a halogen atom, a hydroxyl group, an alkyl group having 1 - 6 carbon atoms or an alkoxy group having 1 - 6 carbon atoms.

27. The optically active form or a salt thereof according to any one of claims 20 to 22, and 26, wherein the optically active form is an R form.

28. The optically active form or a salt thereof according to any one of claims 20 to 22, 26, and 27, wherein the compound represented by the formula (I) is the following compound:
